# EUROPEAN PATENT SPECIFICATION

(11) **EP 3 378 495 B1**
(45) Date of publication and mention of the grant of the patent: **05.08.2020**
(21) Application number: 16866393.8
(22) Date of filing: 17.11.2016
(51) Int. Cl.: A61K 47/50, A61K 31/4745, A61K 31/704, A61K 31/7068, A61K 47/34, A61P 35/00, A61P 43/00

(54) **COMPOSITION COMPRISING NOVEL GLUTAMIC ACID DERIVATIVE AND BLOCK COPOLYMER, AND USE THEREOF**
ZUSAMMENSETZUNG MIT NEUARTIGEM GLUTAMINSÄUREDERIVAT UND BLOCKCOPOLYMER UND VERWENDUNG DAVON
COMPOSITION COMPRENANT UN NOUVEAU DÉRIVÉ DE L'ACIDE GLUTAMIQUE ET UN COPOLYMÈRE À BLOCS, ET UTILISATION DE CETTE DERNIÈRE

(30) Priority: 18.11.2015 JP 2015225820
(43) Date of publication of application: 26.09.2018
(73) Proprietor: Nippon Kayaku Kabushiki Kaisha, Tokyo 100-0005 (JP)
(72) Inventor: NIITSUMA, Setsuko, Tokyo 115-8588 (JP); SEKINE, Keiko, Tokyo 115-8588 (JP); YONETA, Yasushi, Tokyo 115-8588 (JP); TOMIYAMA, Chisato, Tokyo 115-8588 (JP)
(74) Representative: Ter Meer Steinmeister & Partner
(86) International application number: PCT/JP2016/084119
(87) International publication number: WO 2017/086392

(56) References cited:
- EP-A1- 2 011 516
- WO-A1-2006/033296
- WO-A1-2015/178265
- WO-A1-2015/178265
- WO-A2-03/043631
- CN-A- 106 008 650
- JP-A- H0 769 900
- JP-A- H06 206 815
- JP-A- 2005 514 359
- QINGZHI ZHANG ET AL: "Development and Characterization of Glutamyl-Protected N -Hydroxyguanidines as Reno-Active Nitric Oxide Donor Drugs with Therapeutic Potential in Acute Renal Failure", JOURNAL OF MEDICINAL CHEMISTRY, vol. 56, no. 13, 11 July 2013 (2013-07-11) , pages 5321-5334, XP055237991, US ISSN: 0022-2623, DOI: 10.1021/jm400146r
- PATRICK D. BRAUN ET AL: "A Bifunctional Molecule That Displays Context-Dependent Cellular Activity", JOURNAL OF THE AMERICAN CHEMICAL SOCIETY, vol. 125, no. 25, 1 June 2003 (2003-06-01) , pages 7575-7580, XP055606281, ISSN: 0002-7863, DOI: 10.1021/ja035176q
- QING LU ET AL: "ChemInform Abstract: Two New Alkaloids from Brachystemma calycinum and Their Inhibitory Effects on Lymphocyte Proliferation.", CHEMINFORM, vol. 39, no. 19, 6 May 2008 (2008-05-06), XP055606284, DE ISSN: 0931-7597, DOI: 10.1002/chin.200819197
- SEOKAN PARK ET AL: "Mitochondria-targeting ratiometric fluorescent probe for [gamma]-glutamyltranspeptidase and its application to colon cancer", CHEMICAL COMMUNICATIONS, vol. 52, no. 68, 1 January 2016 (2016-01-01), pages 10400-10402, XP055606286, ISSN: 1359-7345, DOI: 10.1039/C6CC05573G
- ZHANG,Q. et al.: "Development and Characterization of Glutamyl-Protected N-Hydroxyguanidines as Reno-Active Nitric Oxide Donor Drugs with Therapeutic Potential in Acute Renal Failure", Journal of Medicinal Chemistry, vol. 56, no. 13, 2013, pages 5321-5334, XP055237991, DOI: doi:10.1021/jm400146r
- ZHANG, Q. et al.: "A new class of NO -donor pro-drugs triggered by gamma-glutamyl transpeptidase with potential for reno-selective vasodilatation", Chemical Communications, vol. 49, no. 14, 2013, pages 1389-1391, XP055596324, Cambridge, United Kingdom ISSN: 1359-7345, DOI: 10.1039/c2cc38382a
- KEREN, R. et al.: "y-Glutamyl transpeptidase-dependent mutagenicity and cytotoxicity of y-glutamyl derivatives: A model for biochemical targeting of chemotherapeutic agents", Environmental and Molecular Mutagenesis, vol. 32, no. 4, 1998, pages 377-386, XP055237992,

## Description

### Technical Field

The present invention relates to a composition containing a novel glutamic acid derivative, which is a novel compound that is enzyme-selectively activated at a target site, and a block copolymer; and to a use of the composition. More particularly, the invention relates to a composition containing a novel glutamic acid derivative, which is a prodrug that is activated by γ-glutamyl transpeptidase (GGT, E.C. 2.3.2.2), and a block copolymer having a hydrophilic segment and a hydrophobic segment, and to a use thereof.

### Background Art

A prodrug is a substance that changes into an active type drug after being metabolized in vivo. Examples of the purpose of producing a drug into a prodrug include improvement of stability, improvement of solubility, improvement of absorbability, reduction of side effects, improvement of drug action time (sustenance of action), and exhibition of action at specific sites. Several drugs have been hitherto developed as prodrugs, and those prodrugs are clinically used as medicines for the treatment of various diseases.

A significant number of antitumor agents that are used for the chemotherapy of cancer exhibit cell proliferation inhibitory action in cancer cells as well as in normal cells, and therefore, side effects attributed to this exhibition of cell proliferation inhibitory action has been a problem. Therefore, if an antitumor agent could be caused to selectively act on cancer cells, an antitumor agent having reduced side effects would be possibly supplied. Thus, if an antitumor agent could be produced into a prodrug and be selectively activated at a target site such as a tumor tissue, it would be anticipated to reduce side effects and to simultaneously enhance the therapeutic effect significantly.

As a method of selectively converting a prodrug into an active compound at a target site, a method of using an enzyme that is expressed at a high level at the target tissue has been considered. As a technique of producing a prodrug of a drug using an enzyme-specific reaction of the target site, there is known a technique of interposing a self-cleaving type linker between an enzyme recognition site and the drug (Non Patent Literature 1). This enables the enzyme recognition site to be cleaved by an enzyme-specific reaction, and in a conjugate of a linker and a drug produced by the cleavage, the linker portion to undergo self-cleavage and to thereby release the drug.

γ-Glutamyl transpeptidase (GGT) is an enzyme that takes charge of the incipient stage of metabolic degradation of glutathione (γ-Glu-Cys-Gly) and glutathione conjugates, and it is known that γ-glutamyl transpeptidase exists universally in almost all living organisms including from higher animals and plants to microorganisms (Non Patent Literature 2, Non Patent Literature 3, and Non Patent Literature 4). GGT is an enzyme that hydrolyzes a γ-glutamyl bond of glutathione, and produces Glu and Cys-Gly, while giving a γ-glutamyl transfer product to acceptors such as various amino acids, dipeptides, and amines.

GGT is known to be expressed at a high level in multiple kinds of various cancer cells, and there also have been reports that point out GGT as a drug target for cancer chemotherapy (Non Patent Literature 5).

Patent Literature 1 discloses a compound obtained by γ-glutamylation of an antitumor agent. A prodrug that can be cut off by trypsin has been mentioned as a prodrug that uses a self-cleaving linker (Non Patent Literature 1). However, there is no description on a prodrug which is recognized by GGT and promotes drug release by using a self-cleaving linker. Furthermore, in Non Patent Literature 6, a compound produced by glutamylating an antitumor agent is mentioned, and enzyme-dependent cellular toxicity of the compound is disclosed. However, this compound is such that a pharmacologically active compound is dissociated at a very high enzyme concentration, and thus, the compound is not capable of functioning as a prodrug in an in vivo environment. Also, in Non Patent Literature 7, a prodrug type compound having a drug linked to the γ-position of glutamic acid, the compound having an appropriate linker, is mentioned.

As a means for reducing side effects of an antitumor agent or enhancing the therapeutic effect of an antitumor agent, in addition to producing a prodrug of the antitumor agent, there is also available a method of changing the pharmacokinetics of the antitumor agent by formulating a DDS preparation of the antitumor agent using a polymer carrier or the like. Representative known examples include PEGylation, liposome formulation, and micelle formulation.

Preparations obtained by incorporating medicines such as doxorubicin hydrochloride, irinotecan hydrochloride, vincristine sulfate, docetaxel, and indomethacin into polymeric micelle carriers have been reported in Patent Literatures 2 and 3. Patent Literature 4 discloses amino-acid derived anti-cancer compounds that are selectively converted to active substances in tumors.

However, there is no known example of further formulating a prodrug intended for tissue-selective activation of GGT or the like into a DDS preparation using a polymer carrier or the like.

### Citation List

### Patent Literature

Patent Literature 1: US Patent No. 7989188
Patent Literature 2: WO 2007/126110 A
Patent Literature 3: WO 2007/1436134 A
Patent Literature 4: WO 2003/043631 A2

### Non Patent Literature

Non Patent Literature 1: J. Med. Chem., 24, 479-480 (1981)
Non Patent Literature 2: Adv. Enzymol. Relat. Areas Mol. Biol., 72, 239-278 (1998)
Non Patent Literature 3: Methods Enzymol., 113, 400-419 (1985)
Non Patent Literature 4: Methods Enzymol., 113, 419-437 (1985)
Non Patent Literature 5: Biochemical Pharmacology 71, 231-238 (2006)
Non Patent Literature 6: Environmental and Molecular Mutagenesis 32, 377-386 (1998)
Non Patent Literature 7: Chem. Commun., 49, 1389-1391 (2013)

### Summary of Invention

### Technical Problem

Since a prodrug that is recognized by GGT may selectively release an active compound in tissues expressing GGT at a high level, the prodrug may be expected to become a medicine having reduced side effects and an enhanced therapeutic effect. However, a prodrug which sufficiently exhibits the stability or effect required as a medicine may not be obtained, and a GGT-recognizable prodrug preparation that may be used as a medicine is desirable. Furthermore, it is an object to provide a composition that efficiently exhibits the effectiveness of the GGT-recognizable prodrug.

### Solution to Problem

In order to solve the problems described above, the inventors of the present invention repeatedly conducted thorough investigation, and as a result, the inventors found that a glutamic acid derivative obtained by conjugating an appropriate γ-glutamyl aromatic amide with a physiologically active substance, is very stable in a physiological environment and rapidly releases the physiologically active substance when recognized by GGT. Furthermore, the inventors found that when the glutamic acid derivative as a GGT-recognizable prodrug is prepared into a composition together with a block copolymer in which a polyethylene glycol segment is linked to a polyamino acid segment with a hydrophobic functional group, the pharmacological activity of the GGT-recognizable prodrug may be increased, and side effects may be reduced. The inventors of the present invention thus completed the present invention based on these findings.

That is, the gist of the present application is based on the inventions disclosed in the following items [1] to [15].
[1] A composition including:
   (I) a glutamic acid derivative represented by General Formula (1): 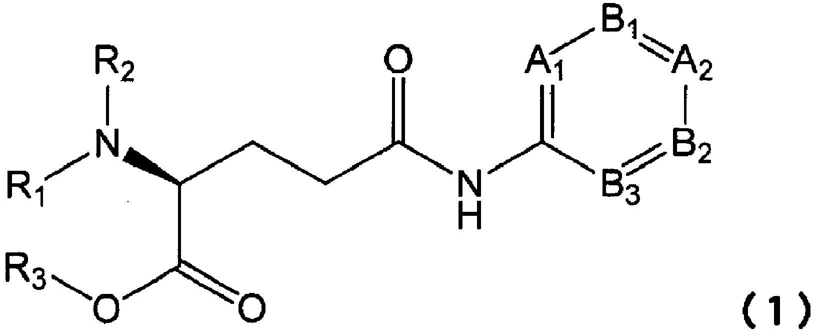 wherein **R**₁ and **R**₂ each independently represent a group selected from the group consisting of a hydrogen atom, an alkyl group which may have a substituent, and an alkoxycarbonyl group which may have a substituent; **R**₃ represents a hydrogen atom or an alkyl group which may have a substituent; **A**₁ and **A**₂ each represent a group selected from the group consisting of C-**R**₆, C-**R**₇, and a nitrogen atom; **R**₆ represents one or more groups selected from the group consisting of a hydrogen atom, a halogen atom, a nitro group, a hydroxy group, an alkyl group which may have a substituent, and an alkoxy group which may have a substituent; **R**₇ is represented by the following General Formula (2): 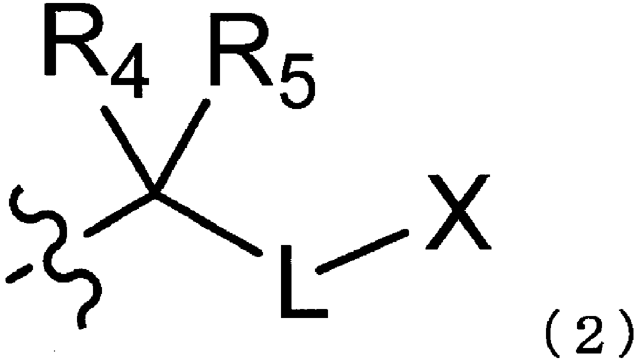 wherein **R**₄ and **R**₅ each independently represent a hydrogen atom or an alkyl group which may have a substituent; **L** represents a linking group selected from the group consisting of an oxygen atom, an oxycarbonyl group, and a bond; **X** represents a residue of a physiologically active substance having one or more functional groups selected from the group consisting of an aliphatic hydroxy group, an aromatic hydroxy group, an amino group, and a carboxy group,
      (a) when **X** is a residue of a physiologically active substance having one or more functional groups selected from the group consisting of an aliphatic hydroxy group and an amino group, **L** represents an oxycarbonyl group;
      (b) when **X** represents a residue of a physiologically active substance having a carboxy group, **L** represents an oxygen atom; and
      (c) when **X** represents a residue of a physiologically active substance having an aromatic hydroxy group, **L** represents a bond or an oxycarbonyl group,
      wherein any one of **A**₁ and **A**₂ represents C-**R**₇; the other represents C-**R**₆ or a nitrogen atom; and **B**₁, **B**₂, and **B**₃ each independently represent C-**R**₆ or a nitrogen atom;
      or a pharmacologically acceptable salt; and
   (II) a block copolymer having a polyethylene glycol segment linked to a polyamino acid segment with a hydrophobic functional group.
      The present application is an invention related to a useful composition including a glutamic acid derivative (I), which is a novel GGT-recognizable prodrug, as an active ingredient in combination with a block copolymer (II) as a pharmaceutical additive, the composition having enhanced pharmacological activity/action as well as reduced side effects.

   According to the present invention, it is preferable to use the following block copolymer (II).
[2] The composition according to the above-described item [1], wherein the polyamino acid in the block copolymer (II) is one or more selected from the group consisting of polyaspartic acid, polyglutamic acid, and a poly(aspartic acid-glutamic acid) copolymer.
[3] The composition according to [1] or [2], wherein the hydrophobic functional group in the block copolymer (II) is one or more groups selected from the group consisting of a linear, branched or cyclic (C1-C30) alkyl group which may have a substituent; a linear, branched or cyclic (C2-C30) alkenyl group which may have a substituent; a linear or branched (C7-C30) aralkyl group which may have a substituent; an aryl group which may have a substituent; a heterocyclic aryl group which may have a substituent; and a residue of a physiologically active substance.
[4] The composition according to any one of [1] to [3], wherein the weight average molecular weight of the polyethylene glycol segment in the block copolymer (II) is 1 kilodalton to 500 kilodaltons, and the polymerization number of the polyamino acid is 2 to 200.
[5] The composition according to any one of [1] to [4], wherein the block copolymer (II) is a copolymer represented by General Formula (3): 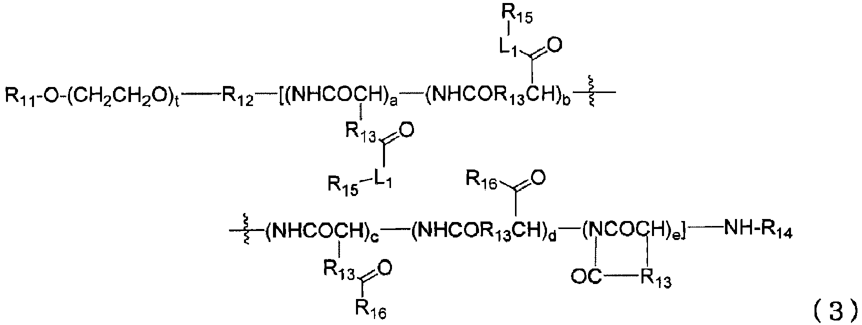
   wherein **R**₁₁ represents a hydrogen atom or a linear or branched (C1-C10) alkyl group; **R**₁₂ represents a (C1-C6) alkylene group; **R**₁₃ represents a methylene group and/or an ethylene group; **R**₁₄ is selected from the group consisting of a hydrogen atom, a (C1-C6) acyl group, and a (C1-C6) alkyloxycarbonyl group;
   **R**₁₅ represents one or more kinds of groups selected from the group consisting of a linear, branched or cyclic (C1-C30) alkyl group which may have a substituent, a linear, branched or cyclic (C2-C30) alkenyl group which may have a substituent, a linear, branched or cyclic (C7-C30) aralkyl group which may have a substituent, an aryl group which may have a substituent, a heterocyclic aryl group which may have a substituent, and a residue of a physiologically active substance;
   **R**₁₆ represents a hydroxy group and/or -N(**R**₁₇)CONH(**R**₁₈), wherein **R**₁₇ and **R**₁₈, which may be identical or different, each represent a linear, branched or cyclic (C3-C8) alkyl group, or a (C1-C6) alkyl group which may be substituted with a tertiary amino group;
   **L**₁ represents a linking group or a bond; t represents an integer from 20 to 11,500; a, b, c, d, and e each independently represent an integer from 0 to 100; (a + b + c + d + e), which is the total polymerization number of the polyamino acid segment, represents an integer from 10 to 100; (a + b) represents an integer from 3 to 100; and the various constituent units to which **R**₁₅ and **R**₁₆ are bonded, and the intramolecularly cyclized type constituent units of side-chain carboxy groups each independently have a randomly arranged segment structure.
[6] The composition according to any one of [1] to [5], wherein the hydrophobic functional group in the block copolymer (II) is one or more groups selected from the group consisting of a residue of an amino acid derivative modified with a hydrophobic functional group, a residue of a sterol derivative, a (C7-C20) aralkyl group which may have a substituent, an anthracycline-based antibiotic substance, a camptothecin derivative, and a nucleic acid antimetabolite.
   According to the present invention, it is preferable to use the following glutamic acid derivative as an active ingredient.
[7] The composition according to any one of the above-described items [1] to [6], wherein in the glutamic acid derivative represented by General Formula (1) or a pharmacologically acceptable salt thereof (I), **R**₇ is represented by the following General Formula (4): 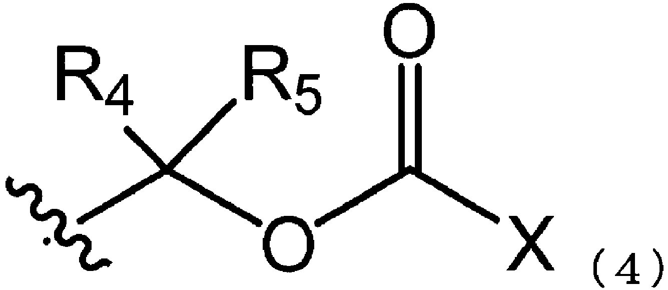 wherein **R**₄ and **R**₅ are as defined above; and **X** represents a residue of a physiologically active substance having one or more functional groups selected from the group consisting of an aliphatic hydroxy group, an aromatic hydroxy group, and an amino group.
   That is, in a case in which a physiologically active substance having an aliphatic hydroxy group, an aromatic hydroxy group, or an amino group is used, it is preferable that the linking group represented by **L** in General Formula (2) uses an oxycarbonyl group, and an embodiment thereof is shown here.
[8] The composition according to [7], wherein the physiologically active substance in the residue of a physiologically active substance represented by **X** is camptothecin or a derivative thereof.
[9] The composition according to [7], wherein the physiologically active substance in the residue of a physiologically active substance represented by **X** is a physiologically active substance selected from the group consisting of doxorubicin, daunorubicin, epirubicin, pirarubicin, and amrubicin.
[10] The composition according to [7], wherein the physiologically active substance in the residue of a physiologically active substance represented by **X** is a physiologically active substance selected from the group consisting of gemcitabine, ethynyl cytidine, cytarabine, and CNDAC (2'-cyano-2'-deoxy-1-β-D-arabinofuranosylcytosine).
   Furthermore, in the case of using a physiologically active substance having an aromatic hydroxy group, the linking group represented by **L** of General Formula (2) is a bond, that is, an embodiment in which the physiologically active substance is directly linked without mediating a linking group is preferred, and an embodiment thereof may be shown as follows.
[11] The composition according to any one of the above-described items [1] to [6], wherein **R**₇ is represented by the following General Formula (5): 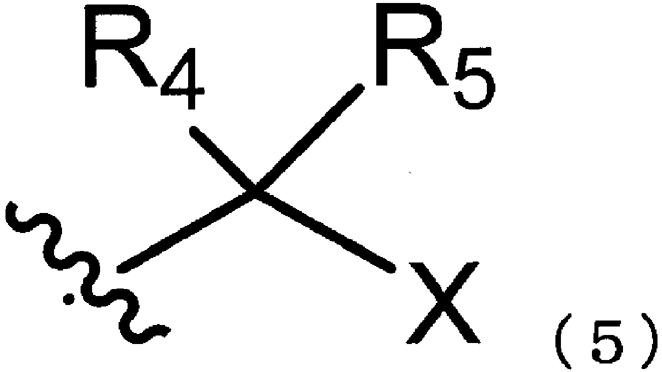 wherein **R**₄ and **R**₅ are as defined above; and **X** represents a residue of a physiologically active substance having an aromatic hydroxy group.
[12] The composition according to [11], wherein the physiologically active substance in the residue of a physiologically active substance represented by **X** is selected from the group consisting of 7-ethyl-10-hydroxycamptothecin, nogitecan, and derivatives thereof.
   More preferred embodiments of the composition of the present invention are shown below.
[13] The composition according to any one of [1] to [12], wherein the mass ratio of the glutamic acid derivative or a pharmacologically acceptable salt thereof (I) to the block copolymer (II) is such that (I) : (II) = 1 : 0.5 to 50.
[14] The composition according to any one of [1] to [13], wherein the glutamic acid derivative or a pharmacologically acceptable salt thereof (I) is associated with the block copolymer (II).
[15] A medicine including the composition according to any one of [1] to [14].

### Advantageous Effects of Invention

The glutamic acid derivative of the present invention or a pharmacologically acceptable salt thereof has a property of rapidly releasing a physiologically active substance by being recognized by GGT. GGT is known to be expressed at a high level in many malignant tumors. Therefore, when the glutamic acid derivative of the present invention is applied to a physiologically active substance having an antitumor effect, the glutamic acid derivative may release a compound exhibiting antitumor activity in a target tissue-selective manner, and thus, an antitumor drug having reduced side effects and having an enhanced therapeutic effect may be provided.

Furthermore, when a composition is prepared by mixing the glutamic acid derivative with a block copolymer in which a polyethylene glycol segment is linked to a polyamino acid segment with a hydrophobic group, a suitable pharmaceutical preparation capable of further exhibiting the effectiveness of the glutamic acid derivative as a GGT-recognizable prodrug may be provided. Particularly, the composition based on a glutamic acid derivative that uses an antitumor compound as the physiologically active substance may effectively accumulate the glutamic acid derivative at the tumor affected area, and the composition shows superior effects against tumors, while release of a physiologically active substance may be suppressed in the bone marrow tissue where the expression ratio of GGT is low. Therefore, side effects such as myelosuppression, which becomes a problem in the use of antitumor drugs, may be avoided.

### Brief Description of Drawings

Fig. 1 shows the results of a cell proliferation suppression test of Synthesis Example 1 for OS-RC-2 cells;
Fig. 2 shows the results of a cell proliferation suppression test of Synthesis Example 1 for SK-OV-3 cells;
Fig. 3 shows the results of a cell proliferation suppression test of Synthesis Example 1 for OS-RC-2 cells in the presence of a GGT inhibitor;
Fig. 4 shows the results of a cell proliferation suppression test of the compound of Synthesis Example 6 for OS-RC-2 cells;
Fig. 5 shows the results of a cell proliferation suppression test of the compound of Synthesis Example 6 for SK-OV-3 cells;
Fig. 6 shows the results of a cell proliferation suppression test of the compound of Synthesis Example 6 for OS-RC-2 cells in the presence of a GGT inhibitor;
Fig. 7 shows the results of the drug concentration profiles of Synthesis Example 1 in tissues;
Fig. 8 shows the results of an antitumor effect test of Synthesis Example 1 against OS-RC-2 tumors;
Fig. 9 shows the results of an antitumor effect test of Synthesis Example 1 against SHIN-3 tumors;
Fig. 10 shows the results of an antitumor effect test of Synthesis Example 6 against OS-RC-2 tumors;
Fig. 11 shows the results of the drug concentration profile of Example 1 in blood plasma; and
Fig. 12 shows the results of an antitumor effect test of Example 6 against OS-RC-2 tumors.

### Description of Embodiments

The present invention relates to a composition including a glutamic acid derivative represented by General Formula (1) or a pharmacologically acceptable salt thereof (I), and a block copolymer (II) having a polyethylene glycol segment and a polyamino acid segment with a hydrophobic functional group linked together. Furthermore, the present invention relates to a use of the composition as a medicine. The details of the present invention will be described below.

First, the glutamic acid derivative or a pharmacologically acceptable salt thereof (I) will be explained. The glutamic acid derivative of the present invention or a pharmacologically acceptable salt thereof (I) is a glutamic acid derivative represented by the following General Formula (1) or a pharmacologically acceptable salt thereof: 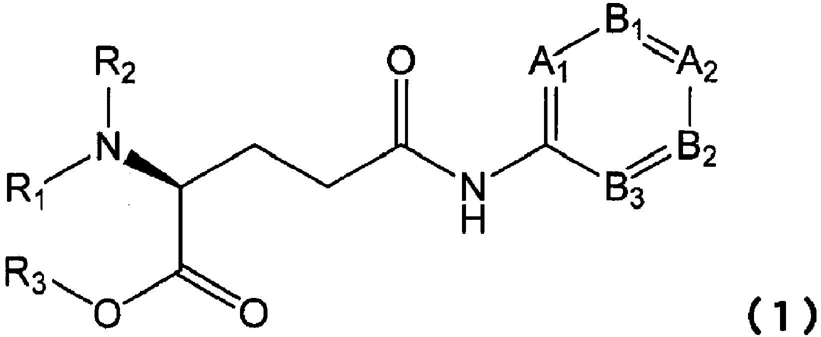 wherein **R**₁ and **R**₂ each independently represent a group selected from the group consisting of a hydrogen atom, an alkyl group which may have a substituent, and an alkoxycarbonyl group which may have a substituent; **R**₃ represents a hydrogen atom or an alkyl group which may have a substituent; **A**₁ and **A**₂ each represent a group selected from the group consisting of C-**R**₆, C-**R**₇, and a nitrogen atom; **R**₆ represents one or more groups selected from the group consisting of a hydrogen atom, a halogen atom, a nitro group, a hydroxy group, an alkyl group which may have a substituent, and an alkoxy group which may have a substituent; **R**₇ is represented by the following General Formula (2): 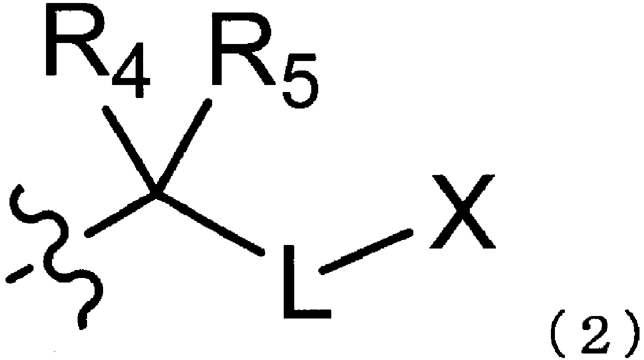 wherein **R**₄ and **R**₅ each independently represent a hydrogen atom or an alkyl group which may have a substituent; **L** represents a linking group selected from the group consisting of an oxygen atom, an oxycarbonyl group, and a bond; **X** represents a residue of a physiologically active substance having one or more functional groups selected from the group consisting of an aliphatic hydroxy group, an aromatic hydroxy group, an amino group, and a carboxy group;
(a) when **X** represents a residue of a physiologically active substance having one or more functional groups selected from the group consisting of an aliphatic hydroxy group and an amino group, **L** represents an oxycarbonyl group;
(b) when **X** represents a residue of a physiologically active substance having a carboxy group, **L** represents an oxygen atom; and
(c) when **X** represents a residue of a physiologically active substance having an aromatic hydroxy group, **L** represents a bond or an oxycarbonyl group;
here, any one of **A**₁ and **A**₂ is C-**R**₇, while the other is C-**R**₆ or a nitrogen atom; and **B**₁, **B**₂, and **B**₃ each independently represent C-**R**₆ or a nitrogen atom.

**R**₁ and **R**₂ in General Formula (1) each independently represent a hydrogen atom, an alkyl group which may have a substituent, or an alkoxycarbonyl group which may have a substituent.

The alkyl group for the alkyl group which may have a substituent represents a linear, branched or cyclic alkyl group having 1 to 30 carbon atoms. Examples of the linear alkyl group include a methyl group, an ethyl group, a n-propyl group, a n-butyl group, a n-hexyl group, a n-dodecyl group, a n-tetradecyl group, and a n-hexadecyl group. Examples of the branched alkyl group include an isopropyl group, a t-butyl group, a 1-methylpropyl group, a 2-methylpropyl group, and a 2,2-dimethylpropyl group. Examples of the cyclic alkyl group include a cyclopropyl group, a cyclobutyl group, a cyclopentyl group, a cyclohexyl group, and an adamantyl group.

The alkoxycarbonyl group for the alkoxycarbonyl group which may have a substituent represents an alkoxycarbonyl group having 1 to 10 carbon atoms. Examples thereof include a methoxycarbonyl group, an ethoxycarbonyl group, as well as a primary alkoxycarbonyl group such as a benzyloxycarbonyl group or a 9-fluorenylmethoxycarbonyl group; a secondary alkoxycarbonyl group such as an isopropoxycarbonyl group or a sec-butoxycarbonyl group; and a tertiary alkoxycarbonyl group such as a t-butoxycarbonyl group, all of which have appropriate aromatic substituents.

Examples of the substituent that may be carried by the alkyl group and the alkoxycarbonyl group include a mercapto group, a hydroxy group, a halogen atom, a nitro group, a cyano group, an alkenyl group having 2 to 10 carbon atoms, an alkynyl group having 2 to 10 carbon atoms, a carbocyclic or heterocyclic aryl group, an alkylthio group having 1 to 8 carbon atoms, an arylthio group, an alkylsulfinyl group having 1 to 8 carbon atoms, an arylsulfinyl group, an alkylsulfonyl group having 1 to 8 carbon atoms, an arylsulfonyl group, an alkoxy group having 1 to 8 carbon atoms, an aryloxy group, an aliphatic or aromatic amino group, an aliphatic amino group-substituted alkyl group having 1 to 8 carbon atoms, a formyl group, an acyl group, a carboxy group, and a silyl group. The position of substitution on the aromatic ring may be the ortho-position, the meta-position, or the para-position.

It is preferable that the alkyl group which may have a substituent or the alkoxycarbonyl group which may have a substituent for **R**₁ and **R**₂ in General Formula (1) described above is a protective group for an amino group. That is, any protective group for an amino group in an organic synthesis reaction may be used without particular limitations. Particularly preferred is the alkoxycarbonyl group, and examples include a benzyloxycarbonyl group (Cbz group), a t-butoxycarbonyl group (Boc group), a 9-fluorenylmethoxycarbonyl group (Fmoc group), and an allyloxycarbonyl group (Aloc group).

It is preferable that **R**₁ and **R**₂ are a hydrogen atom and/or an alkoxycarbonyl group which may have a substituent. The case in which **R**₁ and **R**₂ are both hydrogen atoms, or the case in which a mixture of a hydrogen atom and an alkoxycarbonyl group which may have a substituent is used, is preferred.

**R**₃ in General Formula (1) may be a hydrogen atom or an alkyl group which may have a substituent.

The alkyl group for the alkyl group which may have a substituent represents a linear, branched or cyclic alkyl group having 1 to 30 carbon atoms. Examples of the linear alkyl group include a methyl group, an ethyl group, a n-propyl group, a n-butyl group, a n-hexyl group, a n-dodecyl group, a n-tetradecyl group, and a n-hexadecyl group. Examples of the branched alkyl group include an isopropyl group, a t-butyl group, a 1-methylpropyl group, a 2-methylpropyl group, and a 2,2-dimethylpropyl group. Examples of the cyclic alkyl group include a cyclopropyl group, a cyclobutyl group, a cyclopentyl group, a cyclohexyl group, and an adamantyl group. Furthermore, examples also include a benzyl group and a 9-fluorenylmethyl group, all of which have an appropriate aromatic substituent. The substituent for the alkyl group of **R**₃ has the same meaning as described above.

Regarding the alkyl group which may have a substituent of **R**₃, it is preferable that a protective group for a carboxylic acid is used. Any protective group for a carboxylic acid in an organic synthesis reaction may be used without particular limitations. Particularly preferred examples include a methyl group, an ethyl group, a t-butyl group, an allyl group, a benzyl group, and a 9-fluorenylmethyl group.

In General Formula (1), **A**₁ and **A**₂ are each a group selected from the group consisting of C-**R**₆, which is a carbon atom substituted with **R**₆; C-**R**₇, which is a carbon atom substituted with **R**₇; and a nitrogen atom. Furthermore, **B**₁, **B**₂, and **B**₃ each independently represent C-**R**₆, which is a carbon atom substituted with **R**₆, or a nitrogen atom.

**R**₆ described above represents one or more substituents selected from the group consisting of a hydrogen atom, a halogen atom, a hydroxy group, a nitro group, an alkyl group which may have a substituent, and an alkoxy group which may have a substituent.

The halogen atom represents a fluorine atom, a chlorine atom, a bromine atom, or an iodine atom.

The alkyl group for the alkyl group which may have a substituent represents a linear, branched or cyclic alkyl group having 1 to 30 carbon atoms. Examples of the linear alkyl group include a methyl group, an ethyl group, a n-propyl group, a n-butyl group, a n-hexyl group, a n-dodecyl group, a n-tetradecyl group, and a n-hexadecyl group. Examples of the branched alkyl group include an isopropyl group, a t-butyl group, a 1-methylpropyl group, a 2-methylpropyl group, and a 2,2-dimethylpropyl group. Examples of the cyclic alkyl group include a cyclopropyl group, a cyclobutyl group, a cyclopentyl group, a cyclohexyl group, and an adamantyl group.

The alkoxy group for the alkoxy group which may have a substituent represents an alkoxy group having 1 to 10 carbon atoms. Examples thereof include a primary alkoxy group such as a methoxy group, an ethoxy group, or a benzyloxy group; a secondary alkoxy group such as an isopropoxy group or a sec-butoxy group; and a tertiary alkoxy group such as a t-butoxy group.

Examples of the substituent that may be carried by the alkyl group and the alkoxy group include a mercapto group, a hydroxy group, a halogen atom, a nitro group, a cyano group, an alkenyl group having 2 to 10 carbon atoms, an alkynyl group having 2 to 10 carbon atoms, a carbocyclic or heterocyclic aryl group, an alkylthio group having 1 to 8 carbon atoms, an arylthio group, an alkylsulfinyl group having 1 to 8 carbon atoms, an arylsulfinyl group, an alkylsulfonyl group having 1 to 8 carbon atoms, an arylsulfonyl group, an alkoxy group having 1 to 8 carbon atoms, an aryloxy group, an aliphatic or aromatic amino group, an aliphatic amino group-substituted alkyl group having 1 to 8 carbon atoms, a formyl group, an acyl group, a carboxy group, and a silyl group. The position of substitution on the aromatic ring may be the ortho-position, the meta-position, or the para-position.

**A**₁, **A**₂, **B**₁, **B**₂, and **B**₃ described above may be each a nitrogen atom. That is, the 6-membered ring aromatic group including **A**₁ to **B**₃ may be a nitrogen-containing heterocyclic ring. The nitrogen-containing heterocyclic ring includes a heterocyclic group containing one to three nitrogen atoms for these **A**₁ to **B**₃. Examples include a pyridine ring, a pyridazine ring, a pyrimidine ring, a pyrazine ring, and a triazine ring. The nitrogen-containing heterocyclic ring may have a substituent. Examples of the substituent include the substituents defined for **R**₆ as described above.

**R**₄ and **R**₅ each independently represent a hydrogen atom or an alkyl group which may have a substituent.

The alkyl group for the alkyl group which may have a substituent represents a linear, branched or cyclic alkyl group having 1 to 30 carbon atoms. Examples of the linear alkyl group include a methyl group, an ethyl group, a n-propyl group, a n-butyl group, a n-hexyl group, a n-dodecyl group, a n-tetradecyl group, and a n-hexadecyl group. Examples of the branched alkyl group include an isopropyl group, a t-butyl group, a 1-methylpropyl group, a 2-methylpropyl group, and a 2,2-dimethylpropyl group. Examples of the cyclic alkyl group include a cyclopropyl group, a cyclobutyl group, a cyclopentyl group, a cyclohexyl group, and an adamantyl group. The substituent that may be carried has the same meaning as the substituent for **R**₁ and **R**₂ described above.

**R**₇ is a group represented by General Formula (2) described above. In this General Formula (2), **L** represents a linking group selected from the group consisting of an oxygen atom, an oxycarbonyl group, and a bond; **X** represents a residue of a physiologically active substance having one or more functional groups selected from the group consisting of an aliphatic hydroxy group, an aromatic hydroxy group, an amino group, and a carboxy group;
(a) when **X** represents a residue of a physiologically active substance having one or more functional groups selected from the group consisting of an aliphatic hydroxy group and an amino group, **L** represents an oxycarbonyl group;
(b) when **X** represents a residue of a physiologically active substance having a carboxy group, **L** represents an oxygen atom; and
(c) when **X** represents a residue of a physiologically active substance having an aromatic hydroxy group, **L** represents a bond or an oxycarbonyl group. **L** is preferably a bond.

Meanwhile, a residue of a physiologically active substance means a residue of the physiologically active substance to which the linking group represented by **L** is bonded. That is, the residue is a residue obtained by eliminating a hydrogen atom from an aliphatic hydroxy group, an amino group, or an aromatic hydroxy group, and is a residue obtained by eliminating a hydroxy group from a carboxy group. Specific embodiments in which the residue and a linking group are bonded will be described below.

In a case in which the **X** group is a residue of a physiologically active substance having an aliphatic hydroxy group or an aromatic hydroxy group, the residue is a residue which is linked to an oxycarbonyl group through a carbonate bond formed by the hydroxy group and the oxycarbonyl group. Meanwhile, in a case in which the **X** group is a residue of a physiologically active substance having an amino group, the residue is a residue which is linked to an oxycarbonyl group through a urethane bond formed by the amino group and an oxycarbonyl group.

Furthermore, in a case in which the **X** group is a residue of a physiologically active substance having a carboxy group, the residue is a residue which is linked to an oxygen atom as a linking group **L** through an ester bond formed by the oxygen atom and the carbonyl group derived from the physiologically active substance.

Furthermore, in a case in which the **X** group is a residue of a physiologically active substance having an aromatic hydroxy group, the **X** group is a residue which is linked by an ether bond derived from an oxygen atom of the aromatic hydroxy group of the physiologically active substance.

Furthermore, according to one embodiment of the glutamic acid derivative (I), **R**₇ is represented by the following General Formula (4): 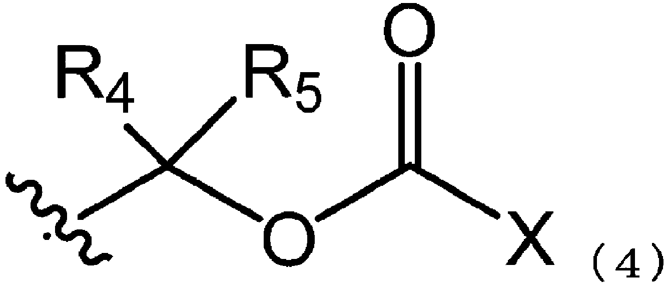 wherein **R**₄ and **R**₅ are as defined above; and **X** represents a residue of a physiologically active substance having one or more functional groups selected from the group consisting of an aliphatic hydroxy group, an aromatic hydroxy group, and an amino group.

According to another embodiment of the present invention, **R**₇ is represented by the following General Formula (5): 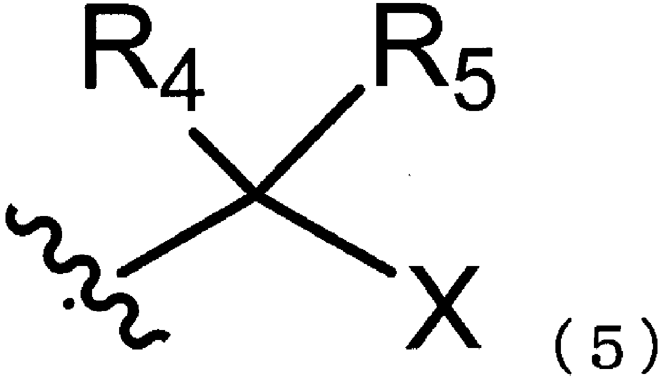 wherein **R**₄ and **R**₅ are as defined above; and **X** represents a residue of a physiologically active substance having an aromatic hydroxy group.

The physiologically active substance of **X** is a chemical substance exhibiting a pharmacological function when administered in vivo, and any compound having one or more functional groups selected from the group consisting of an aliphatic hydroxy group, an aromatic hydroxy group, an amino group, and a carboxy group may be used without particular limitations.

In regard to General Formula (2), in a case in which **X** is a residue of a physiologically active substance having an aliphatic hydroxy group and/or an amino group, this **L** uses an oxycarbonyl group as a linking group, and the compound of General Formula (2) becomes a compound having a carbonate bond and/or a urethane bond. In a case in which **X** is a residue of a physiologically active substance having a carboxy group, this **L** becomes an oxygen atom, and the compound of General Formula (2) becomes a compound that is ester-bonded to a carbonyl group derived from the physiologically active substance. In a case in which **X** is a residue of a physiologically active substance having an aromatic hydroxy group, a bond or an oxycarbonyl group is used as this **L,** and the compound of General Formula (2) becomes a compound having an ether bond or a carbonate bond.

The aliphatic hydroxy group may be any substituent selected from a primary hydroxy group, a secondary hydroxy group, and a tertiary hydroxy group. The amino group may be any substituent selected from a primary amino group, a secondary amino group, and a tertiary amino group.

The physiologically active substance of **X** may also be a physiologically active substance in which an aliphatic hydroxy group and/or an aromatic group and an amino group co-exist. In a case in which the physiologically active substance is a compound in which an aliphatic hydroxy group and/or an aromatic hydroxy group and an amino group co-exist, it may be considered that this **X** group is generally a residue linked through the amino group; however, in consideration of the reaction conditions or steric elements, the residue may be a residue linked through any active functional group selected from the aliphatic hydroxy group and/or aromatic hydroxy group and the amino group, or may be a mixture of a residue linked through the aliphatic hydroxy group and/or aromatic hydroxy group, and a residue linked through the amino group.

Furthermore, the physiologically active substance of **X** may also be a physiologically active substance in which an aliphatic hydroxy group and/or an aromatic hydroxy group and/or an amino group and a carboxy group co-exist. In the case of using a physiologically active substance in which an aliphatic hydroxy group and/or an aromatic hydroxy group and/or an amino group and a carboxy group co-exist, the bonding mode may be selected as appropriate according to the reaction conditions.

The physiological activity of the physiologically active substance is not particularly limited; however, it is preferable that the physiological activity is pharmacological activity related to disease treatment, and it is preferable to use a pharmacologically active compound for disease treatment. Since GGT is expressed at a high level in malignant tumors, the physiologically active substance is preferably an antitumor active substance, and it is preferable to apply an antitumor agent. That is, it is preferable that the physiologically active substance represented by **X** of General Formula (2) is an antitumor agent having one or more functional groups selected from the group consisting of an aliphatic hydroxy group, an aromatic hydroxy group, an amino group, and a carboxy group.

Examples of an antitumor agent suitable as a physiologically active substance represented by **X** of General Formula (2) include a sirolimus-based antitumor agent, an anthracycline-based antitumor agent, a cytidine-based antitumor agent, a tyrosine kinase inhibitor, a DNA topoisomerase inhibitor, a hormone therapy drug, a photodynamic therapy drug, a microtubule inhibitor, a Hsp90 inhibitor, and other antitumor agents exhibiting cell division inhibitory action.

Examples of the sirolimus-based antitumor agent include everolimus, temsirolimus, tacrolimus, and rapamycin.

Examples of the cytidine-based antitumor agent include ethynyl cytidine, CNDAC (2'-cyano-2'-deoxy-1-β-D-arabinofuranosylcytosine), gemcitabine, and cytarabine.

The tyrosine kinase inhibitor is a tyrosine kinase inhibitor having a hydroxy group and/or an amino group, and examples include crizotinib and dasatinib.

Examples of the DNA topoisomerase inhibitor include camptothecin-based antitumor agents, which are DNA topoisomerase I inhibitors such as camptothecin, 7-ethyl-10-hydroxycamptothecin, irinotecan, nogitecan, 9-aminocamptothecin, and 9-nitrocamptothecin. Furthermore, DNA topoisomerase II inhibitors include etoposide and teniposide. Also, anthracycline-based antitumor agents such as doxorubicin, daunorubicin, epirubicin, pirarubicin, idarubicin, mitoxantrone, and amrubicin may be mentioned.

Examples of the hormone therapy drug include raloxifene, goserelin, and leuprorelin.

Examples of the photodynamic therapy drug include 2-butylamino-2-demethoxyhypocrellin.

Examples of the microtubule inhibitor include taxane-based antitumor agents such as paclitaxel and docetaxel; combretastatin and derivatives thereof, podophyllotoxin, eribulin, and olistatin.

Examples of the Hsp90 inhibitor include ganetespib, macbecin, and radicicol.

Furthermore, in regard to **X** of General Formula (2), examples of an antitumor agent suitable as a physiologically active substance having a carboxy group include methotrexate, pemetrexed, DMXAA (5,6-dimethylxanthenone-4-acetic acid), bexarotene, and tamibarotene.

In regard to **X** of General Formula (2), a physiologically active peptide may also be used as the physiologically active substance having an amino group or a carboxy group. The physiologically active peptide having an amino group or a carboxy group may be produced into a form of being bonded using a terminal amino group or carboxy group.

Examples of the physiologically active peptide include bestatin and ester derivatives such as bestatin methyl ester; glufanide, ghrelin, tertomotide, PR1, octreotide, lanreotide, and pasireotide.

It is preferable that **X** of General Formula (2) is a residue of a physiologically active substance having one or more functional groups selected from the group consisting of an aliphatic hydroxy group, an aromatic hydroxy group, and an amino group. In this case, an oxycarbonyl group is used as the linking group **L** in General Formula (2). That is, in this case, **R**₇ is a substituent represented by General Formula (4) described above.

The physiologically active substance having one or more functional groups selected from the group consisting of an aliphatic hydroxy group, an aromatic hydroxy group, and an amino group, is preferably a camptothecin derivative such as camptothecin, 7-ethyl-10-hydroxycamptothecin, irinotecan, nogitecan, 9-aminocamptothecin, or 9-nitrocamptothecin. These compounds include a tertiary hydroxy group of a lactone ring, an aromatic hydroxy group, or an amino group, and these substituents form a carbonate bond and/or a urethane bond with an oxycarbonyl group, which is the linking group described above.

Furthermore, the physiologically active substance having one or more functional groups selected from the group consisting of an aliphatic hydroxy group, an aromatic hydroxy group, and an amino group, is also preferably an anthracycline-based antitumor agent such as doxorubicin, daunorubicin, epirubicin, pirarubicin, idarubicin, mitoxantrone, or amrubicin. More preferred examples include doxorubicin, daunorubicin, epirubicin, pirarubicin, and amrubicin. These compounds include a hydroxy group and/or an amino group, and these substituents form a carbonate bond and/or a urethane bond with an oxycarbonyl group, which is the linking group described above.

Furthermore, the physiologically active substance having one or more functional groups selected from the group consisting of an aliphatic hydroxy group, an aromatic hydroxy group, and an amino group, is preferably a cytidine-based antitumor agent such as gemcitabine, ethynyl cytidine, cytarabine, or CNDAC (2'-cyano-2'-doexy-1-β-D-arabinofuranosylcytosine). These compounds have a hydroxy group and/or an amino group of cytidine base, and these substituents form a carbonate bond and/or a urethane bond with an oxycarbonyl group, which is the linking group described above.

Furthermore, according to another embodiment, **X** of General Formula (2) is preferably a residue of a physiologically active substance having an aromatic hydroxy group. In this case, a bond or an oxycarbonyl group is used as the linking group **L** in General Formula (2). The linking group **L** is preferably a bond. That is, preferably, **R**₇ is a substituent represented by Genera Formula (5) described above.

It is preferable to use 7-ethyl-10-hydroxycamptothecin or nogitecan as the physiologically active substance having an aromatic hydroxy group, and it is preferable that **X** is a residue which is linked by an ether bond derived from the hydroxy group at the 10-position of 7-ethyl-10-hydroxycamptothecin or nogitecan.

The glutamic acid derivative (I) according to the present invention is such that any one of **A**₁ and **A**₂ represents C-**R**₇. That is, an embodiment of the present invention is a glutamic acid derivative represented by the following General Formula (6), in which **A**₁ is C-**R**₇, or a pharmacologically acceptable salt thereof (I).

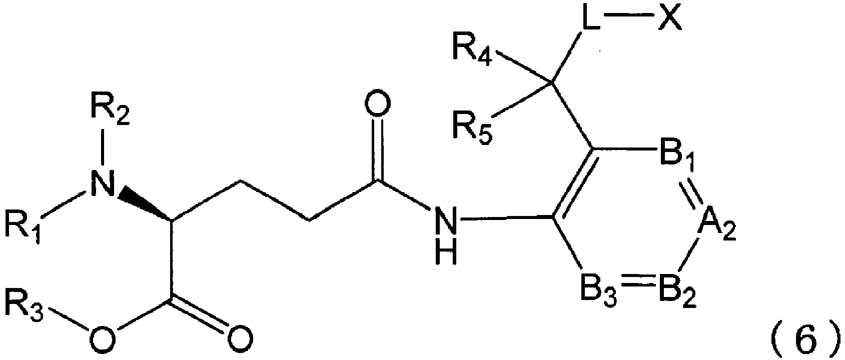

In General Formula (6), **R**₁, **R**₂, **R**₃, **R**₄, **R**₅, **A**₂, **B**₁, **B**₂, **B**₃, **L** and **X** are as defined above.

Furthermore, another embodiment of the glutamic acid derivative (I) according to the present invention is a glutamic acid derivative represented by the following General Formula (7), in which **A**₂ is C-**R**₇, or a pharmacologically acceptable salt thereof (I).

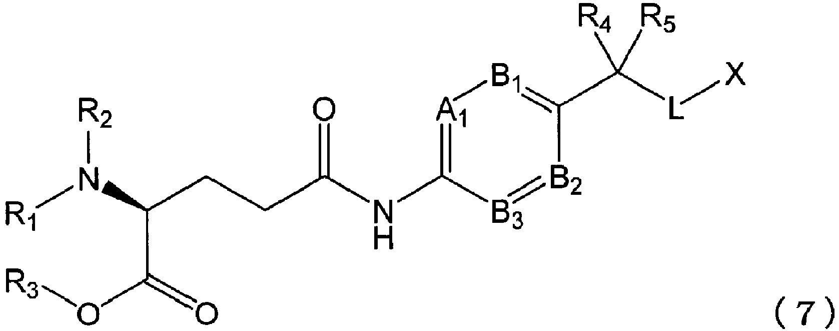

In General Formula (7), **R**₁, **R**₂, **R**₃, **R**₄, **R₅, A**₁, **B**₁, **B**₂, **B**₃, **L,** and **X** are as defined above.

In regard to the glutamic acid derivative represented by General Formula (1) of the present invention or a pharmacologically acceptable salt thereof (I), in order for the derivative or salt to be recognized by GGT, it is necessary that the structure of the γ-glutamic acid-bonded part is a free amino acid structure. That is, in order for the glutamic acid derivative (I) to function as a prodrug that is activated by being recognized by GGT, it is necessary that **R**₁, **R**₂, and **R**₃ are hydrogen atoms. Therefore, in a case in which this glutamic acid derivative is used as a medicinal drug for exhibiting pharmacological activity, it is preferable that **R**₁, **R**₂, and **R**₃ are all hydrogen atoms.

However, the case in which any one of **R**₁, **R**₂, and **R**₃ is a protective group for an amino group or a carboxy group, and after the glutamic acid derivative is administered in vivo, the protective group is dissociated to give a structure in which **R**₁, **R**₂, and **R**₃ are all converted to hydrogen atoms, is also included as an embodiment of the glutamic acid derivative (I) for pharmaceutical use.

In addition, a compound in which **R**₁, **R**₂, and **R**₃ each represent an alkyl group which may have a substituent, or an alkoxycarbonyl group, is a compound useful as an intermediate for the production of the compound for pharmaceutical use, and is included in the content of the present invention.

The glutamic acid derivative (I) represented by General Formula (1) may also exist as a pharmacologically acceptable salt. Examples of the salt include a base addition salt, an acid addition salt, and an amino acid salt. Examples of the base addition salt include metal salts such as sodium salt, potassium salt, calcium salt, and magnesium salt; and organic amine salts such as ammonium salt, triethylamine salt, piperidine salt, and morpholine salt. Examples of the acid addition salt include mineral acid salts such as hydrochloride, sulfate, and nitrate; and organic acid salts such as methanesulfonate, para-toluenesulfonate, citrate, and oxalate. Examples of the amino acid salt include glycine salt. However, the salt of the compound of the present invention is not limited to these.

The glutamic acid derivative represented by General Formula (1) and a salt thereof (I) may have one or two or more asymmetric carbon atoms depending on the type of the substituent, and optical isomers or stereoisomers such as diastereoisomers may exist. Stereoisomers of pure forms, arbitrary mixtures of stereoisomers, racemates, and the like are all included in the scope of the present invention.

The glutamic acid derivative represented by General Formula (1) and a salt thereof (I) may also exist as a hydrate or a solvate, and these substances are all included in the scope of the present invention. The type of the solvent that forms a solvate is not particularly limited; however, examples include solvents such as ethanol, acetone, and isopropanol. The number of bonds of the hydrate or solvate is not particularly limited, and an isolable stable type hydrate or solvate is desirable.

Since a method for producing a representative compound that is included in the compound of the present invention represented by General Formula (1) is specifically disclosed in the Examples of the present specification, a person ordinarily skilled in the art may easily produce any compound included in General Formula (1) by referring to the disclosure of the present specification and by appropriately selecting the starting raw materials, reagents, reaction conditions, and the like as necessary.

The glutamic acid derivative (I) represented by General Formula (1) of the present invention is such that in a case in which **A**₂ is C-**R**₇; **X** is a residue of a physiologically active substance having an aliphatic hydroxy group, and/or an aromatic hydroxy group, and/or an amino group; and **L** is an oxycarbonyl group, the glutamic acid derivative (I) may be produced, for example, as follows.

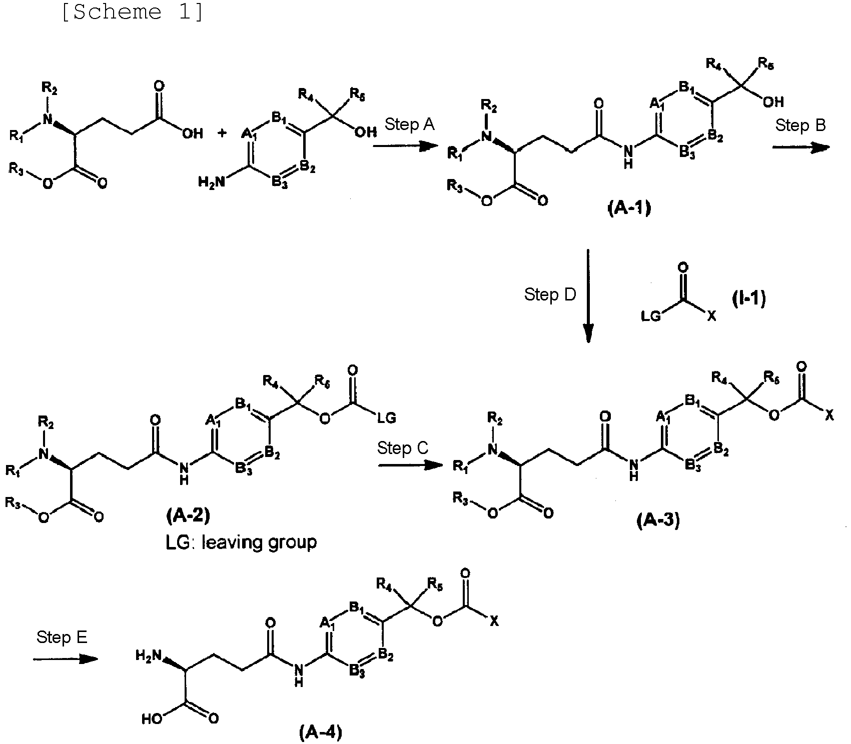

In Scheme 1), **R**₁ to **R**₃, **X, A**₁, and **B**₁ to **B**₃ are as defined above. Here, **R**₁ and/or **R**₂ is a protective group for an amino group, and **R**₃ is a protective group for a carboxylic acid. LG represents a leaving group such as a halogen atom, a p-nitrophenoxy group, or a hydroxysuccinimide group. The various steps will be explained below.

[Step A]: This is a step of synthesizing a γ-glutamic acid amide derivative (A-1) by amidating a glutamic acid derivative in which an amino group and an α-carboxy group have been protected, with an aromatic amine. The present step is an amidation condensation reaction, and the reaction may be carried out using a general condensing agent. The present step may be carried out by performing the reaction using, for example, 1-ethoxycarbonyl-2-ethoxy-1,2-dihydroquinoline (EEDQ) as a condensing agent in a solvent such as dichloromethane, at a temperature of 0°C to 150°C, and preferably 0°C to 30°C. Regarding the condensing agent, for example, a carbodiimide-based condensing agent, an imidazole-based dehydration condensing agent, a triazine-based condensing agent, a phosphonium-based dehydration condensing agent, a uronium-based condensing agent, diphenylphosphoric acid azide (DPPA), a BOP reagent, and 4-(4,6-dimethoxy-1,3,5-triazin-2-yl)-4-methylmorpholinium chloride (DMT-MM) may be used. According to necessity, the condensing agent may be allowed to co-exist with an activating agent such as 1-hydroxybenzotriazole.
[Step B]: This is a step of synthesizing a carbonyl derivative represented by General Formula (A-2) from the γ-glutamic acid amide derivative represented by General Formula (A-1). The present step may be carried out by, for example, allowing p-nitrophenyl chloroformate to react with the γ-glutamic acid amide derivative in the presence of pyridine in a solvent such as tetrahydrofuran, at a temperature of -30°C to 150°C, and preferably -10°C to 30°C.
[Step C]: This is a step of synthesizing a physiologically active substance-bonded derivative represented by General Formula (A-3) from the carbonyl derivative represented by General Formula (A-2). The present step may be carried out by, for example, allowing a physiologically active substance having an aliphatic hydroxy group, and/or an aromatic hydroxy group, and/or an amino group to react with the carbonyl derivative in the presence of diisopropylethylamine in a solvent such as N,N-dimethylformamide, at a temperature of 0°C to 150°C, and preferably 0°C to 30°C.
[Step D]: This is a process route for synthesizing the physiologically active substance-bonded derivative represented by General Formula (A-3) from the γ-glutamic acid amide derivative represented by General Formula (A-1) in a single stage. The present step may be carried out by, for example, allowing a carbonylated physiologically active substance derivative represented by General Formula (I-1) to react with the γ-glutamic acid amide derivative in the presence of N,N-dimethylaminopyridine in a solvent such as dichloromethane, at a temperature of 0°C to 150°C, and preferably 0° to 30°C.
   The carbonylated physiologically active substance derivative represented by General Formula (I-1) may be produced by allowing a physiologically active substance **X** having an aliphatic hydroxy group, and/or an aromatic hydroxy group, and/or an amino group to react with triphosgene, for example, in the presence of N,N-dimethylaminopyridine in a solvent such as dichloromethane, at a temperature of 0°C to 150°C, and preferably 0°C to 30°C. Alternatively, the carbonylated physiologically active substance derivative may be produced by, for example, allowing p-nitrophenyl chloroformate to react with the physiologically active substance **X** in the presence of pyridine in a solvent such as dichloromethane, at a temperature of 0°C to 150°C, and preferably 0°C to 30°C.
[Step E]: This is a step of performing deprotection reactions of the amino group and the carboxy group of the γ-glutamic acid-bonded part in the physiologically active substance-bonded derivative represented by General Formula (A-3).

In a case in which any one of **R**₁ and **R**₂ is a t-butoxycarbonyl group (Boc group), the other is a hydrogen atom, and **R**₃ is a t-butyl group, the deprotection of Step E may be carried out under acidic conditions. Regarding the acid, inorganic acids such as hydrochloric acid and sulfuric acid; carboxylic acids such as acetic acid and trifluoroacetic acid; and the like may be used. In addition to those, any catalyst that is known to be able to deprotect a t-butoxycarbonyl group or a t-butyl ester and does not affect any part other than a protective group, may be used without particular limitations.

In a case in which any one of **R**₁ and **R**₂ is a 9-fluorenylmethoxycarbonyl group (Fmoc group), the other is a hydrogen atom, and **R**₃ is a fluorenylmethyl group, this Step E may be carried out under alkaline conditions. Regarding the base, ammonia, or organic bases such as piperidine and morpholine may be used. In addition to those, any catalyst that is known to be able to deprotect a fluorenylmethoxycarbonyl group or a fluorenylmethyl ester and does not affect any part other than a protective group, may be used under any deprotection reaction conditions.

Furthermore, in a case in which any one of **R**₁ and **R**₂ is an allyloxycarbonyl group (Aloc group), the other is a hydrogen atom, and **R**₃ is an allyl group, this Step E may be carried out in the presence of a palladium catalyst. Regarding the palladium catalyst, tetrakis(triphenylphosphine)palladium(0) or the like may be used. In addition to those, any catalyst that is known to be able to deprotect an allyloxycarbonyl group or an allyl ester and does not affect any part other than a protective group, may be used under any deprotection reaction conditions.

In a case in which **X** is a residue of a physiologically active substance having an amino group, a derivative represented by General Formula (A-3) may be produced, for example, as follows.

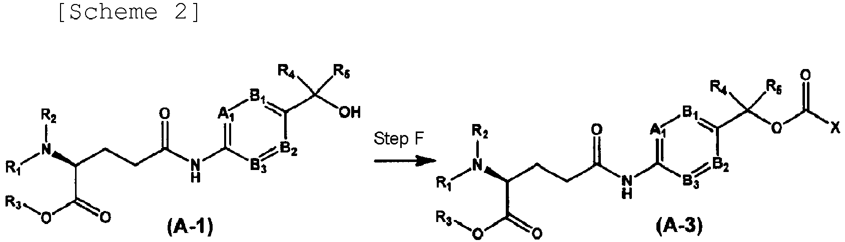

In Scheme 2, **R**₁ to **R**₃, **X, A**₁, and **B**₁ to **B**₃ are as defined above; **R**₁ and/or **R**₂ is a protective group for an amino group; and **R**₃ is a protective group for a carboxylic acid. The various steps will be explained below.

[Step F]: This is a step of synthesizing a physiologically active substance-bonded derivative represented by General Formula (A-3) from the γ-glutamic acid amide derivative represented by General Formula (A-1). The present step may be carried out by, for example, allowing an isocyanate derivative of a physiologically active substance **X** having an amino group to react with the γ-glutamic acid amide derivative in the presence of N,N-dimethylaminopyridine in a solvent such as dichloromethane, at a temperature of 0°C to 150°C, and preferably 0°C to 30°C.

The isocyanate derivative of a physiologically active substance **X** having an amino group may be produced by, for example, allowing a physiologically active substance **X** having an amino group to react with triphosgene in a mixed solvent of an aqueous solution of potassium hydrogen carbonate and dichloromethane or the like, at a temperature of 0°C to 150°C, and preferably 0°C to 30°C.

In a case in which **X** is a physiologically active substance having a carboxy group, and **L** is an oxygen atom, the physiologically active substance-bonded derivative represented by General Formula (A-3) may be produced, for example, as follows.

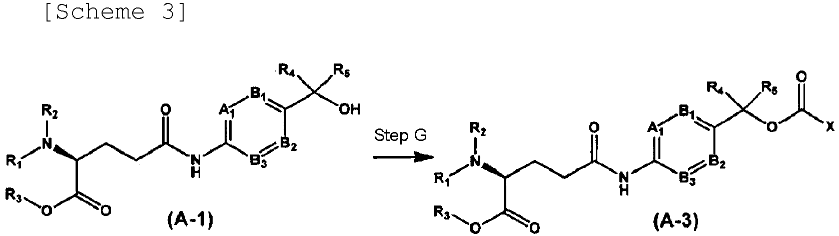

In Scheme 3, **R**₁ to **R**₃, **X, A**₁, and **B**₁ to **B**₃ are as defined above; **R**₁ and/or **R**₂ is a protective group for an amino group; and **R**₃ is a protective group for a carboxylic acid. The various steps will be explained below.

[Step G]: This is a step of esterifying the γ-glutamic acid amide derivative represented by General Formula (A-1) and a physiologically active substance having a carboxy group through a condensation reaction. For the present step, a general condensing agent may be used, and for example, the present step may be carried out by, for example, performing the reaction by using 1-(3-dimethylaminopropyl)-3-ethylcarbodiimide hydrochloride as a condensing agent in a solvent such as N,N-dimethylformamide, at a temperature of 0°C to 150°C, and preferably 0°C to 30°C. Regarding the condensing agent, besides, a carbodiimide-based condensing agent, an imidazole-based dehydration condensing agent, a triazine-based condensing agent, a phosphonium-based dehydration condensing agent, a uronium-based condensing agent, a diphenylphosphoric acid azide (DPPA), a BOP reagent, 4-(4,6-dimethoxy-1,3,5-triazin-2-yl)-4-methylmorpholinium chloride (DMT-MM), and the like may be used. If necessary, an activating agent such as 1-hydroxybenzotriazole or N,N-dimethyl-4-aminopyridine may be allowed to co-exist.

Furthermore, a glutamic acid derivative represented by General Formula (1) of the present invention, in which **A**₂ is C-**R**₇; **X** is a physiologically active substance having an aromatic hydroxy group; and **L** is a bond, may be produced, for example, as follows.

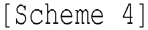

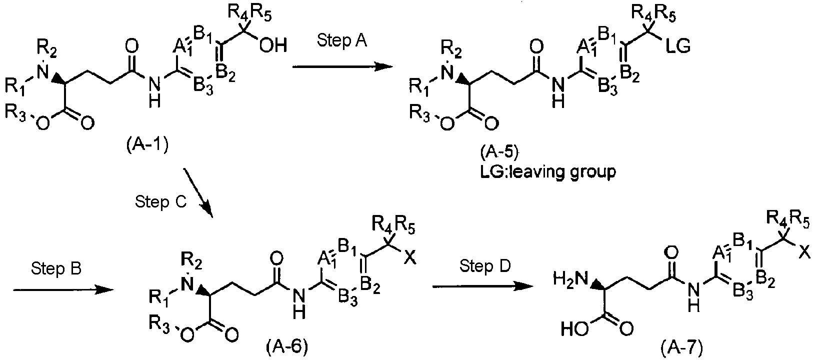

In Scheme 4, **R**₁ to **R**₃, **X**, **A**₁, and **B**₁ to **B**₃ are as defined above. Here, **R**₁ and/or **R**₂ is a protective group for an amino group, and **R**₃ is a protective group for a carboxylic acid.

LG represents a leaving group such as a halogen atom or a methanesulfonyloxy group. The various steps will be explained below.

[Step A]: This is a step of synthesizing a derivative represented by General Formula (A-5) from the γ-glutamic acid amide derivative represented by General Formula (A-1). LG in General Formula (A-5) is a leaving group, and examples include a methanesulfonyloxy group and a p-toluenesulfonyloxy group. The present step may be carried out, for example, in a case in which the leaving group is a methanesulfonyloxy group, by allowing methanesulfonyl chloride to react with the γ-glutamic acid amide derivative in the presence of N,N-diisopropylethylamine in a solvent such as dichloromethane, at a temperature of -30°C to 150°C, and preferably -10°C to 30°C.
[Step B]: This is a step of synthesizing a physiologically active substance-bonded derivative represented by General Formula (A-6) from the derivative represented by General Formula (A-5). The present step may be carried out by, for example, a physiologically active substance having an aromatic hydroxy group to react with the derivative in the presence of cesium carbonate in a solvent such as N,N-dimethylformamide, at a temperature of 0°C to 150°C, and preferably 0°C to 30°C.
[Step C]: This is a process route for synthesizing the physiologically active substance-bonded derivative represented by General Formula (A-6) from the γ-glutamic acid amide derivative represented by General Formula (A-1) in a single stage. The present step may be carried out by, for example, allowing a compound having an aromatic hydroxy group to react with the γ-glutamic acid amide derivative in the presence of triphenylphosphine and diisopropyl azodicarboxylate in a solvent such as N,N-dimethylformamide, at a temperature of 0°C to 150°C, and preferably 0°C to 30°C.
[Step D]: This is a step of performing deprotection reactions for the amino group and carboxy group of the γ-glutamic acid-bonded part in the physiologically active substance-bonded derivative represented by General Formula (A-6) .

In a case in which any one of **R**₁ and **R**₂ is a t-butoxycarbonyl group (Boc group); the other is a hydrogen atom; and **R**₃ is a t-butyl group, the deprotection of Step E may be carried out under acidic conditions. Regarding the acid, inorganic acids such as hydrochloric acid and sulfuric acid; and carboxylic acids such as acetic acid and trifluoroacetic acid may be used. In addition to those, any catalyst that is known to be able to deprotect a t-butoxycarbonyl group or a t-butyl ester and does not affect any part other than a protective group, may be used without particular limitations.

Furthermore, in a case in which any one of **R**₁ and **R**₂ is a 9-fluorenylmethoxycarbonyl group (Fmoc group), the other is a hydrogen atom, and **R**₃ is a fluorenylmethyl group, this Step E may be carried out under alkaline conditions. Regarding the base, ammonia or organic bases such as piperidine and morpholine may be used. In addition to those, any catalyst that is known to be able to deprotect a fluorenylmethoxycarbonyl group or a fluorenylmethyl ester and does not affect any parts other than a protective group, may be used under any deprotection reaction conditions.

Furthermore, in a case in which any one of **R**₁ and **R**₂ is an allyloxycarbonyl group (Aloc group), the other is a hydrogen atom, and **R**₃ is an allyl group, this Step E may be carried out in the presence of a palladium catalyst. Regarding the palladium catalyst, tetrakis(triphenylphosphine)palladium(0) or the like may be used. In addition to those, any catalyst that is known to be able to deprotect an allyloxycarbonyl group or an allyl ester and does not affect any part other than a protective group, may be used under any deprotection reaction conditions.

Next, a block copolymer (II) in which a polyethylene glycol segment is linked to a polyamino acid segment with a hydrophobic functional group will be explained.

In the present invention, a block copolymer (II) in which a polyethylene glycol segment is linked to a polyamino acid segment with a hydrophobic functional group is used. This block copolymer (II) is an AB block copolymer in which a polyethylene glycol segment is bonded to a polyamino acid segment via an appropriate linking group, and since the polyamino acid segment has a hydrophobic functional group, and a hydrophilic polyethylene glycol segment co-exists with the polyamino acid segment, the block copolymer (II) is an amphiphilic block copolymer having both hydrophilicity and hydrophobicity.

The polyethylene glycol segment represents a polymer part including a polyethylene glycol chain in which a degree of polymerization of a (CH₂CH₂O) unit structure is 5 to 20,000. Preferably, the degree of polymerization is 20 to 11,500. Particularly preferably, the polyethylene glycol segment is a polymer part including a polyethylene glycol chain in which the degree of polymerization is 40 to 2,500. The polyethylene glycol-equivalent average molecular weight of the polyethylene glycol segment is 0.2 kilodaltons to 900 kilodaltons, preferably 1 kilodalton to 500 kilodaltons, and particularly preferably 2 kilodaltons to 100 kilodaltons. Meanwhile, the molecular weight of the polyethylene glycol segment is the peak top molecular weight measured by a GPC method (Gel Permeation Chromatography) based on polyethylene glycol standard products.

Regarding the polyamino acid segment with a hydrophobic functional group, any polymer segment showing relative hydrophobicity compared to the polyethylene glycol segment may be used without particular limitations. An example of the polyamino acid segment with a hydrophobic functional group may be a polyamino acid segment including one or more units of an amino acid having a hydrophobic side chain and/or an amino acid derivative modified with a hydrophobic functional group.

Examples of the amino acid having a hydrophobic side chain include alanine, valine, leucine, isoleucine, phenylalanine, tyrosine, methionine, tryptophan, proline, asparagines, and glutamine. It is preferable to use alanine, valine, leucine, isoleucine, phenylalanine, methionine, tryptophan, or proline. The amino acid is not limited to natural amino acids, and a synthetic amino acid may also be used.

Examples of the amino acid derivative modified with a hydrophobic functional group include amino acid derivatives in which a side-chain carboxy group of aspartic acid or glutamic acid, or a side-chain amino group of lysine is modified with a hydrophobic functional group.

Regarding the polyamino acid segment, for the reason that the degree of hydrophobicity may be easily adjusted, it is preferable to use a polycarboxylic acid ester and/or amide, in which a poly(carboxy group)-containing polymer segment, which is a polymer of aspartic acid and/or glutamic acid, is used and a plurality of hydrophobic substituents have been introduced into these carboxy groups by ester bonds and/or amide bonds. Preferably, the polyamino acid segment is a polyaspartic acid ester and/or amide, in which hydrophobic functional groups are bonded to side chains of a polyaspartic acid; a polyglutamic acid ester and/or amide, in which hydrophobic functional groups are bonded to side chains of a polyglutamic acid; or a poly(aspartic acid-glutamic acid) ester and/or amide, in which hydrophobic functional groups are bonded to side chains of a poly(aspartic acid-glutamic acid).

The main chain of the polyamino acid segment is preferably a polymer segment having a single composition structure, and it is preferable to use a polyaspartic acid ester and/or amide, in which hydrophobic functional groups are bonded to side chains of a polyaspartic acid; or a polyglutamic acid ester and/or amide, in which hydrophobic functional groups are bonded to side chains of a polyglutamic acid.

Here, the hydrophobic functional groups included in the polyamino acid segment may be functional groups of a single kind, or may be functional groups of a plurality of kinds.

Regarding the molecular weight of the polyamino acid segment, any molecular weight based on a repeated polymerization number of the extent that may have a number of hydrophobic functional groups to the extent that the polyamino acid segment exhibits hydrophobicity with respect to the polyethylene glycol segment, and the block copolymer (II) may exhibit hydrophilic-hydrophobic amphiphilicity, may be used without particular limitations. Therefore, it is definitely reasonable that the polyamino acid segment is set as appropriate according to the molecular weight of the polyethylene glycol segment.

In regard to the block copolymer (II), when the average molecular weight of the polyethylene glycol segment is 1 kilodalton to 100 kilodaltons, the equivalent molecular weight of the polyamino acid segment is preferably a structural part having a molecular weight of 1 kilodalton to 100 kilodaltons as a segment equivalent average molecular weight, and particularly preferably 3 kilodaltons to 60 kilodaltons.

In the case of using a polyaspartic acid, a polyglutamic acid, or a poly(aspartic acid-glutamic acid) as the polyamino acid segment, it is preferable to determine the polymerization number of the polyamino acid, by which the molecular weight of the polyamino acid segment is defined based on the carboxy group equivalent for introducing hydrophobic groups. The carboxy group equivalent is preferably such that the amount of carboxy groups per polyamino acid segment is preferably 10 molar equivalents to 300 molar equivalents, and more preferably 10 molar equivalents to 100 molar equivalents. That is, in the case of using a polyaspartic acid or a polyglutamic acid, the polymerization number of the polymer is preferably 10 to 300, and the polymerization number is more preferably 10 to 100.

In the case of using the polymer of an amino acid derivative modified with a hydrophobic functional group as the polyamino acid segment, the hydrophobic functional group may be one or more selected from the group consisting of an alkyl group, an alkenyl group, an aralkyl group, an aryl group, a heterocyclic aryl group, and a residue of a physiologically active substance. Preferably, one or more kinds of hydrophobic functional groups selected from the group consisting of a linear, branched or cyclic (C1-C30) alkyl group which may have a substituent; a linear, branched or cyclic (C2-C30) alkenyl group which may have a substituent; a linear or branched (C7-C30) aralkyl group which may have a substituent; an aryl group which may have a substituent; a heterocyclic aryl group which may have a substituent; and a residue of a physiologically active substance, is used. In a case in which a plurality of units of the hydrophobic functional group exists in the polyamino acid segment, the hydrophobic functional groups may be functional groups of a single kind, or a plurality of kinds of functional groups may exist in mixture.

These hydrophobic functional groups are introduced into the side-chain carboxy group of aspartic acid or glutamic acid, or into the side-chain amino group of lysine, via an appropriate linking group.

Examples of the linear, branched or cyclic (C1-C30) alkyl group include a methyl group, an ethyl group, a n-propyl group, an isopropyl group, a n-butyl group, a t-butyl group, a n-pentyl group, a cyclopentyl group, a n-hexyl group, a cyclohexyl group, a cyclohexylmethyl group, a cyclohexylethyl group, a n-octyl group, a n-decyl group, a n-dodecyl group, a n-tetradecyl group, a n-hexadecyl group, a n-octadecyl group, an isooctyl group, an isodecyl group, an isododecyl group, an isotetradecyl group, an isohexadecyl group, an isooctadecyl group, a t-octyl group, a t-decyl group, a t-dodecyl group, a t-tetradecyl group, a t-hexadecyl group, and a t-octadecyl group. The alkyl group is more preferably a linear, branched or cyclic (C8-C20) alkyl group.

The linear, branched or cyclic (C2-C30) alkenyl group is an alkenyl group having a carbon-carbon double bond at any one site. Examples include an ethenyl group, a 1-propenyl group, a 1-butenyl group, a 2-methyl-2-butenyl group, a 1-pentenyl group, a 2-methyl-1,3-butadienyl group, a 1-octenyl group, a 1-decenyl group, a 1-tetradecenyl group, a 9-hexadecenyl group, a cis-9-octadecenyl group, and a cis,cis-9,12-octadecadienyl group. The alkenyl group is more preferably a linear, branched or cyclic (C8-C20) alkenyl group.

The linear, branched or cyclic (C7-C30) aralkyl group is a linear or branched alkyl group having a hydrogen atom at any one site substituted with an aryl group. Examples include a benzyl group, a 2-phenylethyl group, a 4-phenylbutyl group, a 3-phenylbutyl group, a 5-phenylpentyl group, a 6-phenylhexyl group, and an 8-phenyloctyl group. Preferred examples include a 4-phenylbutyl group, a 5-phenylpentyl group, a 6-phenylhexyl group, and an 8-phenyloctyl group.

Examples of the aryl group which may have a substituent include a phenyl group, a naphthyl group, an anthracenyl group, and a phenanthrenyl group.

Examples of the heterocyclic aryl group which may have a substituent include a pyridyl group, a quinolyl group, a pyrimidyl group, a pyrazyl group, a benzopyrrolyl group, a benzofuranyl group, a benzothiophenyl group, and a quinoxalyl group.

Examples of the residue of a physiologically active substance include residues of anthracycline derivatives such as doxorubicin, daunorubicin, epirubicin, pirarubicin, and amrubicin; camptothecin derivatives such as camptothecin, 7-ethyl-10-hydroxycamptothecin, and nogitecan (9-(N,N-dimethylaminomethyl)-10-hydroxycamptothecin); nucleic acid antimetabolites such as gemcitabine, cytarabine, ethynyl cytidine, and CNDAC (2'-cyano-2'-deoxy-1-β-D-arabinofuranosylcytosine); steroid derivatives such as dexamethasone and prednisolone; taxane derivatives such as paclitaxel and docetaxel; and the like; which are linked by a hydroxy group or an amino group included in the respective molecules.

The alkyl group, alkenyl group, aralkyl group, aryl group, heterocyclic aryl group, and residue of a physiologically active substance, all of which are used as hydrophobic functional groups, may respectively have an appropriate substituent.

Examples of the substituent include a mercapto group, a hydroxy group, a halogen atom, a nitro group, a cyano group, a carbocyclic aryl group, a heterocyclic aryl group, an alkylthio group, an arylthio group, an alkylsulfinyl group, an arylsulfinyl group, an alkylsulfonyl group, an arylsulfonyl group, a sulfamoyl group, an alkoxy group, an aryloxy group, an acyloxy group, an alkoxycarbonyloxy group, a carbamoyloxy group, a substituted or unsubstituted amino group, an acylamino group, an alkoxycarbonylamino group, a ureido group, a sulfonylamino group, a sulfamoylamino group, a formyl group, an acyl group, a carboxy group, an alkoxycarbonyl group, a carbamoyl group, and a silyl group. The position of substitution on the aromatic ring may be the ortho-position, the meta-position, or the para-position.

Examples of the carbocyclic aryl group include a phenyl group and a naphthyl group.

Examples of the heterocyclic aryl group include a pyridyl group, a pyrimidinyl group, a quinolyl group, a quinazolinyl group, a naphthyridinyl group, a furyl group, a pyrrolyl group, an indolyl group, an imidazolyl group, a pyrazolyl group, an oxazolyl group, an isoxazolyl group, and a triazolyl group.

The alkylthio group represents a (C1-C8) alkylthio group, and examples include a methylthio group, an isopropylthio group, a n-hexylthio group, and a benzylthio group.

Examples of the arylthio group include a phenylthio group, a naphthylthio group, and a pyridylthio group.

The alkylsulfinyl group represents a (C1-C8) alkylsulfinyl group, and examples include a methylsulfinyl group, an isopropylsulfinyl group, a cyclohexylsulfinyl group, and a benzylsulfinyl group.

Examples of the arylsulfinyl group include a phenylsulfinyl group, a naphthylsulfinyl group, and a pyridylsulfinyl group.

The alkylsulfonyl group represents a (C1-C8) alkylsulfonyl group, and examples include a methylsulfonyl group, an isopropylsulfonyl group, and a benzylsulfonyl group.

Examples of the arylsulfonyl group include a phenylsulfonyl group, a naphthylsulfonyl group, and a pyridylsulfonyl group.

Examples of the sulfamoyl group include a dimethylsulfamoyl group and a phenylsulfamoyl group.

The alkoxy group represents a (C1-C8) alkoxy group, and examples include primary alkoxy groups such as a methoxy group, a n-propyloxy group, a n-hexyloxy group, a n-octyloxy group, and a benzyloxy group; secondary alkoxy groups such as an isopropoxy group and a sec-butoxy group; and tertiary alkoxy groups such as a t-butoxy group.

Examples of the aryloxy group include a phenoxy group, a naphthyloxy group, and a pyridyloxy group.

The acyloxy group represents a (C1-C8) acyloxy group, and examples include an acetoxy group and a benzoyloxy group.

The alkoxycarbonyloxy group represents a (C1-C8) alkoxycarbonyloxy group, and examples include a methoxycarbonyloxy group and a trifluoromethoxycarbonyloxy group.

Examples of the carbamoyloxy group include a dimethylcarbamoyloxy group and a phenylcarbamoyloxy group.

Examples of the substituted or unsubstituted amino group include an unsubstituted amino group, a non-cyclic aliphatic primary amino group, a non-cyclic aliphatic secondary amino group, and a cyclic aliphatic secondary amino group.

The non-cyclic aliphatic primary amino group is an amino group in which a linear, branched or cyclic (C1-C10) alkyl group is N-monosubstituted. Examples include a methylamino group, an isopropylamino group, a neopentylamino group, a n-hexylamino group, a cyclohexylamino group, and a n-octylamino group.

The non-cyclic aliphatic secondary amino group is an amino group that is N,N-disubstituted with linear, branched or cyclic (C1-C10) alkyl groups, which may be identical or different. Examples include a dimethylamino group, a diisopropylamino group, and a N-methyl-N-cyclohexylamino group.

Examples of the cyclic aliphatic secondary amino group include a morpholino group, a piperazin-1-yl group, a 4-methylpiperazin-1-yl group, a piperidin-1-yl group, and a pyrrolidin-1-yl group.

Examples of the acylamino group include an acetylamino group and a benzoylamino group.

Examples of the alkoxycarbonylamino group include a methoxycarbonylamino group, an ethoxycarbonylamino group, and a benzyloxycarbonylamino group.

Examples of the ureido group include a trimethylureido group and a 1-methyl-3-phenylureido group.

Examples of the sulfonylamino group include a methanesulfonylamino group and a benzenesulfonylamino group.

Examples of the sulfamoylamino group include a dimethylsulfamoylamino group.

Examples of the acyl group include an acetyl group, a pivaloyl group, a benzoyl group, and a pyridinecarbonyl group.

Examples of the alkoxycarbonyl group include a methoxycarbonyl group and a benzyloxycarbonyl group.

Examples of the carbamoyl group include a dimethylcarbamoyl group and a phenylcarbamoyl group.

Examples of the silyl group include a trimethylsilyl group, a triisopropylsilyl group, a t-butyldimethylsilyl group, and a tert-butyldiphenylsilyl group.

In a case in which the hydrophobic functional group is introduced into a side-chain carboxy group of aspartic acid or glutamic acid, or into a side-chain amino group of lysine, via an appropriate linking group.

The linking group that mediates the bond between a hydrophobic functional group and a polyamino acid side chain of a carboxy group, an amino group or the like, is a linking group in which one terminal is a functional group bondable to a carboxy group or an amino group, and the other terminal has a functional group bondable to the hydrocarbon group.

Examples include an oxygen atom (-O-), -NH-, a sulfur atom (-S-), -NH-(CH₂)_{x'}- (wherein x' represents an integer from 1 to 10), -NH-(CH₂)_{x'}-O- (wherein x' represents an integer from 1 to 10), -NH-(CH₂)_{x'}-NH- (wherein x' represents an integer from 1 to 10), -NH-(CH₂)_{x'}-S- (wherein x' represents an integer from 1 to 10), -O-(CH₂)_{y'}- (wherein y' represents an integer from 1 to 10), -O-(CH₂)_{y'}-O- (wherein y' represents an integer from 1 to 10), -O-(CH₂)_{y'}-NH- (wherein y' represents an integer from 1 to 10), -O-(CH₂)_{y'}-S- (wherein y' represents an integer from 1 to 10), -CO-O-, -CO-NH-, -CO-(CH₂)_{x'}-O- (wherein x' represents an integer from 1 to 10), - CO-(CH₂)_{x'}-NH- (wherein x' represents an integer from 1 to 10), -CO-(CH₂)_{x'}-S- (wherein x' represents an integer from 1 to 10), -CO-O-(CH₂)_{x'}-O- (wherein x' represents an integer from 1 to 10), -CO-O-(CH₂)_{x'}-NH- (wherein x' represents an integer from 1 to 10), -CO-O-(CH₂)_{x'}-S- (wherein x' represents an integer from 1 to 10).

Furthermore, an amino acid may also be used as the linking group. The amino acid may be any of a natural amino acid and a non-natural amino acid. Regarding the amino acid as a linking group, an embodiment in which the N-terminal amino group forms an amide bond with a carbonyl group of a polymer main chain, and the C-terminal carbonyl group at the other end is bonded to the hydrophobic group via an ester bond or an amide bond, may be mentioned.

In the case of using a poly(carboxy group)-containing polymer segment, which is a polymer of aspartic acid and/or glutamic acid, as the polyamino acid segment, it is preferable that the hydrophobic functional group described above is bonded to a carboxy group of a polycarboxylic acid-containing polymer via an ester type bond and/or an amide type bond. Therefore, regarding the hydrophobic functional group, an embodiment in which an alcohol derivative or an amine derivative, which has a corresponding hydrophobic functional group, is introduced into a polycarboxylic acid-containing polymer, is preferred.

Regarding the alcohol derivative or amine derivative having a hydrophobic functional group, it is preferable to use a biocompatible hydrophobic derivative. Examples of such an alcohol derivative include sterol derivatives such as cholesterol, pregnenolone, and β-sitosterol. Furthermore, regarding such an amine derivative, an amino acid derivative in which the C-terminal carboxy group and/or a side-chain functional group is modified by a hydrophobic functional group may be used.

These sterol derivatives and amino acid derivatives are to be included in a linear, branched or cyclic (C1-30) alkyl group having an appropriate substituent, concerning the hydrophobic functional group.

Furthermore, a physiologically active substance having a hydroxy group and/or an amino group may also be used as the alcohol derivative or amine derivative having a hydrophobic functional group. Examples of the residue of a physiologically active substance include residues of anthracycline derivatives such as doxorubicin, daunorubicin, epirubicin, pirarubicin, and amrubicin; camptothecin derivatives such as camptothecin, 7-ethyl-10-hydroxycamptothecin, and nogitecan (9-(N,N-dimethylaminomethyl)-10-hydroxycamptothecin); nucleic acid antimetabolites such as gemcitabine, cytarabine, ethynyl cytidine, and CNDAC (2'-cyano-2'-deoxy-1-β-D-arabinofuranosylcytosine); steroid derivatives such as dexamethasone and prednisolone; and taxane derivatives such as paclitaxel and docetaxel. These may be linked to a poly(carboxy group)-containing polymer segment via ester bonds and/or amide bonds derived from a hydroxy group and/or an amino group included in the molecule.

In regard to these, it is preferable to use a physiologically active substance having a structure that is identical or similar to that of the physiologically active substance represented by **X** in General Formula (1) of the glutamic acid derivative (I) described above. That is, in a case in which a compound that uses doxorubicin as the glutamic acid derivative (I) is used for **X** related to General Formula (1), regarding the hydrophobic functional group in the block copolymer (II), it is preferable to use the same doxorubicin, or to use an anthracycline derivative having a structure similar thereto.

The hydrophobic functional group constitutes the hydrophobic properties of the polyamino acid segment of the block copolymer (II), and the hydrophobic properties of the polyamino acid segment are determined by the amount of introduction and the introduction ratio of the hydrophobic functional group. That is, as the amount of introduction of the hydrophobic functional group is larger, and the introduction ratio of the hydrophobic functional group is higher, stronger hydrophobic properties are obtained. The polyamino acid segment may be used without particular limitations as long as the polyamino acid segment includes a number of hydrophobic functional groups to the extent that the polyamino acid segment exhibits hydrophobicity relative to the polyethylene glycol segment, and the block copolymer (II) according to the present invention may exhibit hydrophilic-hydrophobic amphiphilicity. Therefore, it is definitely reasonable that the polyamino acid segment is set as appropriate according to the molecular weight of the polyethylene glycol segment.

The hydrophobic functional group may be included in all of the amino acid units of the polyamino acid segment, or an embodiment in which a portion of the amino acid units include the hydrophobic functional group is also acceptable.

In a case in which a hydrophobic functional group is introduced into a side-chain carboxy group via an ester type bond and/or an amide type bond using a poly(carboxy group)-containing polymer segment, which is a polymer of aspartic acid and/or glutamic acid, as the polyamino acid segment, the hydrophobic functional group may be introduced into all of the carboxy groups of the poly(carboxy group)-containing polymer, or may be introduced into a portion of the carboxy groups. Preferably, the hydrophobic functional groups are introduced at a proportion of 10% to 100% of the total carboxy group equivalent of the poly(carboxy group)-containing polymer segment, and more preferably, the polyamino acid segment is modified with the hydrophobic functional groups at a proportion of 20% to 90% of the carboxy group equivalent.

The carboxy group into which the hydrophobic functional group has not been introduced may be in the form of free acid, or in the form of a carboxylic acid salt such as an alkali metal salt, such as sodium salt or potassium salt, or an ammonium salt, and a structure into which other substituents have been introduced is also acceptable.

A preferred embodiment of the block type copolymer block copolymer (II) of the present invention, in which a polyethylene glycol segment is linked to a polyamino acid segment, may be a block copolymer represented by the following General Formula (3): 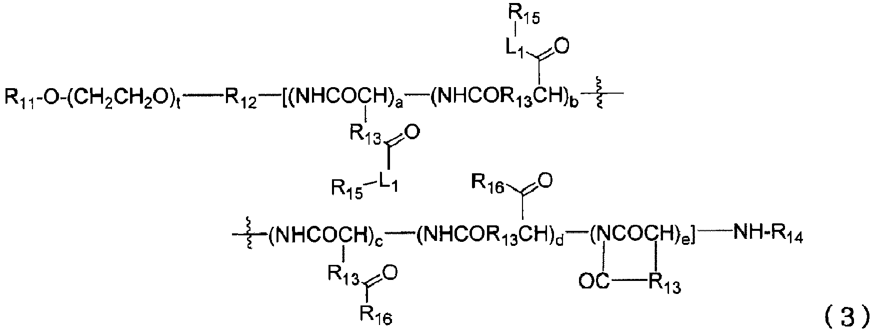 wherein **R**₁₁ represents a hydrogen atom or a linear or branched (C1-C10) alkyl group; **R**₁₂ represents a (C1-C6) alkylene group; **R**₁₃ represents a methylene group and/or an ethylene group; **R**₁₄ is selected from the group consisting of a hydrogen atom, a (C1-C6) acyl group, and a (C1-C6) alkyloxycarbonyl group; **R**₁₅ represents one or more kinds of groups selected from the group consisting of a linear, branched or cyclic (C1-C30) alkyl group which may have a substituent, a linear, branched or cyclic (C2-C30) alkenyl group which may have a substituent, a linear, branched or cyclic (C7-C30) linear or branched aralkyl group which may have a substituent, an aryl group which may have a substituent, a heterocyclic aryl group which may have a substituent, and a residue of a physiologically active substance; **R**₁₆ represents a hydroxy group and/or - N(**R**₁₇)CONH(**R**₁₈); here, **R**₁₇ and **R**₁₈, which may be identical or different, each represent a linear, branched or cyclic (C3-C8) alkyl group, or a (C1-C6) alkyl group which may be substituted with a tertiary amino group; **L**₁ represents a linking group or a bond; t represents an integer from 20 to 11,500; a, b, c, d, and e each independently represent an integer from 0 to 200; (a + b + c + d + e), which is the total polymerization number of the unit constitution of a polyaspartic acid derivative and/or a polyglutamic acid derivative, represents an integer from 10 to 100; (a + b) represents an integer from 3 to 100; and the constituent units to which **R**₁₅ is bonded, the constituent units to which **R**₁₆ is bonded, and the constituent units obtained by intramolecular cyclization of a side-chain carboxy group are each independently arranged in a random fashion.

The (C1-C10) alkyl group for **R**₁₁ represents a linear or branched (C1-C10) alkyl group which may have a substituent. Examples of the linear alkyl group include a methyl group, an ethyl group, a n-propyl group, a n-butyl group, a n-hexyl group, and a n-decyl group. Examples of the branched alkyl group include an isopropyl group, a tert-butyl group, a 1-methylpropyl group, a 2-methylpropyl group, and a 2,2-dimethylpropyl group. Examples of the cyclic alkyl group include a cyclopropyl group, a cyclobutyl group, a cyclopentyl group, a cyclohexyl group, and an adamantyl group.

t in General Formula (3) represents the degree of polymerization of the (CH₂CH₂O) unit structure, and is from 20 to 11,500. Preferably, t is 40 to 2,500.

**R**₁₂ is a linking group for linking the polyethylene glycol segment to a polyaspartic acid segment or a polyglutamic acid segment, and is a (C1-C6) alkylene group. Examples include a methylene group, an ethylene group, a trimethylene group, and a tetramethylene group.

**R**₁₃ represents a methylene group and/or an ethylene group. In a case in which **R**₁₃ is a methylene group, the structure becomes an aspartic acid unit. Meanwhile, in a case in which **R**₁₃ is an ethylene group, the structure becomes a glutamic acid unit. It is acceptable that **R**₁₃ is any one of a methylene group and an ethylene group, and the polyamino acid segment is a polyaspartic acid segment or a polyglutamic acid segment, or it is also acceptable that methylene groups and ethylene groups exist in mixture, and the polyamino acid segment is a poly(aspartic acid-glutamic acid) segment. It is preferable that the polyamino acid segment has a segment structure having a single composition, and the polyamino acid segment is preferably a polyaspartic acid segment or a polyglutamic acid segment.

In General Formula (3), in a case in which **R**₁₃ is a methylene group, the aspartic acid unit becomes a constituent unit of any one polymerization mode selected from an α-amide type constituent unit, a β-amide type constituent unit, and a constituent unit of a type in which the side-chain carboxy group has been intramolecularly cyclized. On the other hand, in a case in which **R**₁₃ is an ethylene group, similarly to the polyglutamic acid unit, the constituent unit of the main chain becomes any one constituent selected from an α-amide type constituent unit, a γ-amide type constituent unit, and a constituent unit of a type in which the side-chain carboxy group has been intramolecularly cyclized. Therefore, in General Formula (3), the polyamino acid segment structure may be a polymer structure of a single constituent unit selected from the α-amide type constituent unit, the β (γ)-amide type constituent unit, and the constituent unit in which the side-chain carboxy group has been intramolecularly cyclized, or the polyamino acid segment structure may be a polymer structure of a mixture of these constituent units.

Examples of the (C1-C6) acyl group for **R**₁₄ include a formyl group, an acetyl group, a propionyl group, a butyroyl group, a cyclopropylcarbonyl group, and a cyclopentanecarbonyl group.

Examples of the (C1-C6) alkoxycarbonyl group for **R**₁₄ include a methoxycarbonyl group, an ethoxycarbonyl group, a t-butoxycarbonyl group, and a cyclohexyloxycarbonyl group.

Examples of the linear, branched or cyclic (C1-C30) alkyl group for **R**₁₅ include a methyl group, an ethyl group, a n-propyl group, an isopropyl group, a n-butyl group, a t-butyl group, a n-pentyl group, a cyclopentyl group, a n-hexyl group, a cyclohexyl group, a cyclohexylmethyl group, a cyclohexylethyl group, a n-octyl group, a n-decyl group, a n-dodecyl group, a n-tetradecyl group, a n-hexadecyl group, a n-octadecyl group, an isooctyl group, an isodecyl group, an isododecyl group, an isotetradecyl group, an isohexadecyl group, an isooctadecyl group, a t-octyl group, a t-decyl group, a t-dodecyl group, a t-tetradecyl group, a t-hexadecyl group, and a t-octadecyl group. The alkyl group is more preferably a linear, branched or cyclic (C8-C20) alkyl group.

The linear, branched or cyclic (C2-C30) alkenyl group for **R**₁₅ is an alkenyl group having a carbon-carbon double bond at any one site. Examples include an ethenyl group, a 1-propenyl group, a 1-butenyl group, a 2-methyl-2-butenyl group, a 1-pentenyl group, a 2-methyl-1,3-butadienyl group, a 1-octenyl group, a 1-decenyl group, a 1-tetradecenyl group, a 9-hexadecenyl group, a cis-9-octadecenyl group, and a cis,cis-9,12-octadecadienyl group. The alkenyl group is more preferably a linear, branched or cyclic (C8-C20) alkenyl group.

The linear, branched or cyclic (C7-C30) aralkyl group for **R**₁₅ is a linear or branched alkyl group in which a hydrogen atom at any one site has been substituted with an aryl group. Examples include a benzyl group, a 2-phenylethyl group, a 4-phenylbutyl group, a 3-phenylbutyl group, a 5-phenylpentyl group, a 6-phenylhexyl group, and an 8-phenyloctyl group. Preferred examples include a 4-phenylbutyl group, a 5-phenylpentyl group, a 6-phenylhexyl group, and an 8-phenyloctyl group.

Examples of the aryl group which may have a substituent for **R**₁₅ include a phenyl group, a naphthyl group, an anthracenyl group, and a phenanthrenyl group.

Examples of the heterocyclic aryl group which may have a substituent for **R**₁₅ include a pyridyl group, a quinolyl group, a pyrimidyl group, a pyrazyl group, a benzopyrrolyl group, a benzofuranyl group, a benzothiophenyl group, and a quinoxalyl group.

Examples of the residue of a physiologically active substance for **R**₁₅ include residues of pharmacologically active substances having a hydroxy group and/or an amino group, such as anthracycline derivatives such as doxorubicin, daunorubicin, epirubicin, pirarubicin, and amrubicin; camptothecin derivatives such as camptothecin, 7-ethyl-10-hydroxycamptothecin, and nogitecan (9-(N,N-dimethylaminomethyl)-10-hydroxycamptothecin); nucleic acid antimetabolites such as gemcitabine, cytarabine, ethynyl cytidine, and CNDAC (2'-cyano-2'-deoxy-1-β-D-arabinofuranosylcytosine); steroid derivatives such as dexamethasone and prednisolone; and taxane derivatives such as paclitaxel and docetaxel, which are linked via ester bonds and/or amide bonds. Meanwhile, the residue represents a residue of the physiologically active substance which is linked to L₁ that will be described below, the residue being obtained by eliminating a hydrogen atom from a hydroxy group and/or an amino group.

The alkyl group, alkenyl group, aralkyl group, aryl group, heterocyclic aryl group and residue of a physiologically active substance, which are used as hydrophobic functional groups, may respectively have an appropriate substituent. Examples of the substituent include a mercapto group, a hydroxy group, a halogen atom, a nitro group, a cyano group, a carbocyclic aryl group, a heterocyclic aryl group, an alkylthio group, an arylthio group, an alkylsulfinyl group, an arylsulfinyl group, an alkylsulfonyl group, an arylsulfonyl group, a sulfamoyl group, an alkoxy group, an aryloxy group, an acyloxy group, an alkoxycarbonyloxy group, a carbamoyloxy group, a substituted or unsubstituted amino group, an acylamino group, an alkoxycarbonylamino group, a ureido group, a sulfonylamino group, a sulfamoylamino group, a formyl group, an acyl group, a carboxy group, an alkoxycarbonyl group, a carbamoyl group, and a silyl group. The position of substitution on the aromatic ring may be the ortho-position, the meta-position, or the para-position. The details of the various substituents have the same meanings as described above.

**L**₁ in General Formula (3) is a linking group for linking a carbonyl group of the polyamino acid segment and **R**₁₅. The linking group for **L**₁ is a linking group having a functional group that has one terminal exhibiting bondability to a carbon group. Examples include an oxygen atom (-O-), -NH-, a sulfur atom (-S-), -NH-(CH₂)ᵣ-O- (wherein r represents an integer from 0 to 6), -O-(CH₂)ᵣ-O- (wherein r represents an integer from 0 to 6), -S-(CH₂)ᵣ-O- (wherein r represents an integer from 0 to 6), -NH-(CH₂)ᵣ-NH- (wherein r represents an integer from 0 to 6), -O-(CH₂)ᵣ-NH- (wherein r represents an integer from 0 to 6), -S-(CH₂)ᵣ-NH- (wherein r represents an integer from 0 to 6), -NH-(CH₂)ᵣ-NHCO- (wherein r represents an integer from 0 to 6), -O-(CH₂)ᵣ-NHCO- (wherein r represents an integer from 0 to 6), -S-(CH₂)ᵣ-NHCO- (wherein r represents an integer from 0 to 6), -NH-(CH₂)ᵣ-CONH-(wherein r represents an integer from 0 to 6), -O-(CH₂)ᵣ-CONH- (wherein r represents an integer from 0 to 6), -S-(CH₂)ᵣ-CONH- (wherein r represents an integer from 0 to 6), - NH-(CH₂)ᵣ-CO-O- (wherein r represents an integer from 0 to 6), -O-(CH₂)ᵣ-CO-O- (wherein r represents an integer from 0 to 6), and -S-(CH₂)ᵣ-CO-O- (wherein r represents an integer from 0 to 6).

Furthermore, in a case in which **L**₁ may be directly bonded to the carbonyl group and **R**₁₅, and there is no corresponding bondable functional group, this **L**₁ represents a bond.

Regarding **L**₁, an amino acid residue may also be used as a linking group. Meanwhile, the amino acid residue is a residue obtained by eliminating a hydrogen atom from the N-terminal amino group or eliminating a hydroxy group from the C-terminal carboxy group. The amino acid that is used as a linking group may be any of a natural amino acid and a non-natural amino acid. In a case in which **L**₁ is an amino acid residue, and an amino acid is used as the linking group, an embodiment in which a carbonyl group of the polyamino acid segment is bonded to the N-terminal amino group of the amino acid, and the **R**₁₅ group is bonded to the C-terminal carboxy group via an ester bond or an amide bond, may be mentioned. Therefore, in the case of using an amino acid residue as **L**₁, **L**₁ is a linking group represented by -NH-C(**R**₂₀)-COO- (wherein **R**₂₀ represents a side chain of the amino acid used).

It is preferable that the hydrophobic functional group related to **R**₁₅ uses a residue of a biocompatible compound. The residue represents a residue obtained by eliminating a hydrogen atom from a hydroxy group and/or an amino group, which is capable of bonding, or eliminating a hydroxy group from a carboxy group. Examples of the biocompatible compound include sterol derivatives such as cholesterol, pregnenolone, and β-sitosterol. Furthermore, an amino acid derivative in which the C-terminal carboxy group and/or a side-chain functional group have been modified by a hydrophobic functional group, may be used.

These sterol derivatives and amino acid derivatives are included in the linear, branched or cyclic (C1-30) alkyl group which may have a substituent, concerning the hydrophobic functional group according to the present invention, and the sterol derivatives and amino acid derivatives are functional groups corresponding to **R**₁₅ and **L**₁ in General Formula (3).

In the case of using a sterol derivative as the hydrophobic functional group, a hydroxy group at the 3-position of ring A of the steroid skeleton functions as a bondable functional group and forms an ester type bond. That is, an embodiment in which in General Formula (3), **R**₁₅ represents the steroid skeleton moiety of a sterol derivative, and **L**₁ represents the oxygen atom at the 3-position of the sterol derivative, is obtained.

Meanwhile, in the case of using the amino acid derivative described above as the hydrophobic functional group, the N-terminal amino group functions as a bondable functional group, and an embodiment of amide type bond is obtained. That is, an embodiment in which in General Formula (3), **R**₁₅ represents the α-carbon skeleton moiety of the amino acid derivative, and **L**₁ represents a group including the N-terminal amino group of the amino acid derivative, is obtained.

The amino acid derivative in which the C-terminal carboxy group and/or a side-chain functional group, which is used as the hydrophobic functional group, has been modified by a hydrophobic functional group, may be any of a natural amino acid and a non-natural amino acid. The amino acid derivative represents a substituent in which at least the C-terminal carboxy group of the amino acid has been converted as an ester derivative or an amide derivative.

The ester derivative is preferably a linear, branched or cyclic (C1-C10) alkyl ester which may have a substituent. Examples of the linear alkyl ester include a methyl ester, an ethyl ester, a n-propyl ester, a n-butyl ester, a n-hexyl ester, a n-decyl ester, a benzyl ester, a 2-phenylethyl ester, and a 4-phenylbutyl ester. Examples of the branched alkyl ester include an isopropyl ester, a tert-butyl ester, a 1-methylpropyl ester, a 2-methylpropyl ester, and a 2,2-dimethylpropyl ester. Examples of the cyclic alkyl ester include a cyclopropyl ester, a cyclobutyl ester, a cyclopentyl ester, a cyclohexyl ester, and an adamantyl ester.

The amide derivative is preferably a linear, branched or cyclic (C1-C10) alkyl amide. Examples of the linear alkyl amide include a methyl amide, an ethyl amide, a n-propyl amide, a n-butyl amide, a n-hexyl amide, a n-decyl amide, a benzyl amide, a 2-phenylethyl amide, and a 4-phenylbutyl amide. Examples of the branched alkyl amide include an isopropyl amide, a tert-butyl amide, a 1-methylpropyl amide, a 2-methylpropyl amide, and a 2,2-dimethylpropyl amide. Examples of the cyclic alkyl amide include a cyclopropyl amide, a cyclobutyl amide, a cyclopentyl amide, a cyclohexyl amide, and an adamantyl amide.

A physiologically active substance may also be used as the hydrophobic functional group. Examples of the residue of a physiologically active substance include residues of anthracycline derivatives such as doxorubicin, daunorubicin, epirubicin, pirarubicin, and amrubicin; camptothecin derivatives such as camptothecin, 7-ethyl-10-hydroxycamptothecin, and nogitecan (9-(N,N-dimethylaminomethyl)-10-hydroxycamptothecin); nucleic acid antimetabolites such as gemcitabine, cytarabine, ethynyl cytidine, and CNDAC (2'-cyano-2'-deoxy-1-β-D-arabinofuranosylcytosine); steroid derivatives such as dexamethasone and prednisolone; and taxane derivatives such as paclitaxel and docetaxel. Meanwhile, a residue of a physiologically active substance is a residue of a physiologically active substance which is linked to L₁.

Since the anthracycline derivatives have amino groups and hydroxy groups, the derivatives may be bonded in an amide-bonded form and/or an ester-bonded form by using those groups as the bondable functional groups. Since the camptothecin derivatives have hydroxy groups, the derivatives may be bonded in an ester-bonded form by using these groups as the bondable functional groups. Since the nucleic acid antimetabolites have amino groups and hydroxy groups, the antimetabolites may be bonded in an amide-bonded form and/or an ester-bonded form by using these groups as the bondable functional groups. Since steroid derivatives such as dexamethasone and prednisolone have hydroxy groups, the derivatives may be bonded in an ester-bonded form by using these groups as the bondable functional groups. Since taxane derivatives such as paclitaxel and docetaxel have hydroxy groups, the derivatives may be bonded in an ester-bonded form by using these groups as the bondable functional groups.

That is, in the case of using a physiologically active substance as a hydrophobic functional group, an embodiment in which in General Formula (3), **L**₁ represents an amino group, and/or an amino group of a bondable functional group for a hydroxy group, and/or a group containing an oxygen atom; and **R**₁₅ represents a residual moiety obtained by eliminating the bondable functional group of the physiologically active substance, is obtained.

In the case of using a physiologically active substance as a hydrophobic functional group, it is preferable to use a physiologically active substance having a structure that is identical or similar to that of the physiologically active substance represented by **X** in General Formula (1) of the glutamic acid derivative (I) described above. That is, in a case in which a compound that uses doxorubicin as **X** related to General Formula (1) is used as the glutamic acid derivative (I), regarding the hydrophobic functional group in the block copolymer (II), it is preferable to use the same doxorubicin, or to use an anthracycline derivative having a structure similar thereto. Similarly, in the case of using 7-ethyl-10-hydroxycamptothecin as **X** related to General Formula (1) as the glutamic acid derivative (I), regarding the hydrophobic functional group for the block copolymer (II), it is preferable to use the same 7-ethyl-10-hydroxycamptothecin, or to use a camptothecin derivative having a structure similar thereto.

**R**₁₆ represents a hydroxy group and/or -N(**R**₁₇)CONH(**R**₁₈). **R**₁₇ and **R**₁₈ in this -N(**R**₁₇)CONH(**R**₁₈), which may be identical or different, each represent a linear, branched or cyclic (C3-C8) alkyl group, or a (C1-C6) alkyl group which may be substituted with a tertiary amino group.

Examples of the linear, branched or cyclic (C3-C8) alkyl group include a 1-propyl group, a 2-propyl group, a cyclopentyl group, and a cyclohexyl group. Examples of the (C1-C6) alkyl group which may be substituted with a tertiary amino group include a 3-dimethylaminopropyl group and a 5-dimethylaminopentyl group.

In a case in which **R**₁₆ is a hydroxy group, an aspartic acid unit and/or a glutamic acid unit come to existence. In this case, the side-chain carboxylic acid may be in the form of free acid, or may be an alkali metal salt such as sodium salt or potassium salt; or a carboxylic acid salt such as ammonium salt. They are also included in the case in which **R**₁₆ is a hydroxy group.

a, b, c, d, and e in General Formula (3) represent the respective contents for the constituent unit in which **R**₁₅ is bonded to the polymer main chain of the polyamino acid segment, the constituent unit in which **R**₁₆ is bonded to the polymer main chain, and the constituent unit in which a side-chain carboxy group is intramolecularly cyclized. These a, b, c, d, and e each independently represent an integer from 0 to 200, and (a + b + c + d + e), which is the total polymerization number of the unit configuration of a polyaspartic acid derivative or a polyglutamic acid derivative, and is the polymerization number of the polymer main chain of the polyaspartic acid derivative or polyglutamic acid derivative as the polyamino acid segment, is an integer from 10 to 100. Among them, the constituent unit in which **R**₁₅ is bonded is an essential configuration, and the total content number (a + b) is an integer from 3 to 100. Meanwhile, the constituent unit in which **R**₁₆ is bonded and the constituent unit in which a side-chain carboxy group is intramolecularly cyclized are optional configurations.

Meanwhile, in General Formula (3), in the polyaspartic acid derivative segment and/or polyglutamic acid derivative segment, which are polyamino acid segments, the constituent unit in which **R**₁₅ is bonded, the constituent unit in which **R**₁₆ is bonded, and the constituent unit in which a side-chain carboxy group is intramolecularly cyclized, are each independently arranged in a random fashion. That is, an embodiment in which the constituent unit in which **R**₁₅ is bonded to a side-chain carboxy group of the polyaspartic acid derivative segment and/or the polyglutamic acid derivative segment, the constituent unit in which **R**₁₆ is bonded to such a side-chain carboxy group, and the constituent unit having a structure in which a side-chain carboxy group is intramolecularly cyclized, are respectively arbitrarily arranged in sequence is acceptable, an embodiment in which the respective constituent units are localized and unevenly distributed is also acceptable, and a polymer structure in which the respective constituent units are configured in a random arrangement without regularity is also acceptable.

In a case in which a hydrophobic functional group is introduced into a side-chain carboxy group in an ester form and/or amide form using a poly(carboxy group)-containing polymer segment, which is a polymer of aspartic acid and/or glutamic acid, as the polyamino acid segment, the hydrophobic functional group may be introduced into all of the carboxy groups of the poly(carboxy group)-containing polymer, or may be introduced into a portion of the carboxy groups.

The hydrophobic functional group constitutes the hydrophobic properties of the polyamino acid segment of the block copolymer (II), and the hydrophobic properties of the polyamino acid segment are determined by the amount of introduction and the introduction ratio of the hydrophobic functional group. That is, as the introduction ratio of the hydrophobic functional groups is higher, stronger hydrophobic properties are obtained. The hydrophobic group introduction ratio should be determined in consideration of the amphiphilicity of the block copolymer (II).

It is preferable that (a + b + c + d + e), which is the polymerization number of the polymer main chain of the polyaspartic acid derivative or polyglutamic acid derivative which is the polyamino acid segment, is an integer from 10 to 80, and the total content number (a + b) of the constituent unit in which the **R**₁₅ group is bonded is an integer from 6 to 80.

The hydrophobic functional group in the block copolymer (II) of the present invention is preferably one or more groups selected from the group consisting of a residue of an amino acid derivative modified with a hydrophobic functional group, a residue of a sterol derivative, a (C7-C20) aralkyl group which may have a substituent, an anthracycline-based antibiotic substance, a camptothecin derivative, and a nucleic antimetabolite.

The residue of the modified amino acid derivative in the hydrophobic functional group is preferably a linear, branched or cyclic (C1-C10) alkyl ester which may have a substituent of valine, leucine, isoleucine, phenylalanine, or tryptophan; or a linear, branched or cyclic (C1-C10) alkyl amide which may have a substituent. Specific examples of the alkyl ester and the alkyl amide have the same meanings as the substituents described for the amino acid derivatives in which the C-terminal carboxy group and/or a side-chain functional group, which is used as the hydrophobic functional group, has been modified by a hydrophobic functional group.

Furthermore, examples of the residue of a sterol derivative for the hydrophobic functional group include sterol derivatives such as cholesterol, pregnenolone, and β-sitosterol.

Examples of the (C7-C20) aralkyl group which may have a substituent for the hydrophobic functional group include a benzyl group, a 2-phenylethyl group, a 4-phenylbutyl group, a 3-phenylbutyl group, a 5-phenylpentyl group, a 6-phenylhexyl group, and an 8-phenyloctyl group.

Examples of the anthracycline derivative for the hydrophobic functional group include doxorubicin, daunorubicin, epirubicin, pirarubicin, and amrubicin.

Examples of the camptothecin derivative for the hydrophobic functional group include camptothecin, 7-ethyl-10-hydroxycamptothecin, and nogitecan (9-(N,N-dimethylaminomethyl)-10-hydroxycamptothecin).

Examples of the nucleic acid antimetabolite for the hydrophobic functional group include gemcitabine, cytarabine, ethynyl cytidine, and CNDAC (2'-cyano-2'-deoxy-1-β-D-arabinofuranosylcytosine).

Next, a method for producing the block copolymer (II) of the present invention, which is a structure obtained by linking a polyethylene glycol segment and a polyamino acid segment, will be explained.

The method for producing the block copolymer (II) of the present invention is not particularly limited; however, the method may be a method of linking a polyethylene glycol segment and a polyamino acid segment, which have been respectively produced in advance; or a method of subjecting a polyethylene glycol segment to a polymerization reaction with the polymerizable monomer of a polyamino acid segment in sequence, and thereby establishing a block type copolymer. Alternatively, a method of producing in advance an AB block type copolymer in which polyethylene glycol and a precursor of a polyamino acid segment are bonded, and introducing an appropriate hydrophobic functional group into this copolymer, is also acceptable.

Preferably, the block copolymer (II) may be produced by producing in advance an AB block type copolymer in which polyethylene glycol is bonded to a polycarboxylic acid polymer segment using a polycarboxylic acid polymer segment such as polyaspartic acid as a precursor of the polyamino acid segment, and reacting this with an appropriate hydrophobic functional group in an amide-bonded form and/or an ester-bonded form under appropriate condensation conditions. Regarding the condensation conditions, a method that may be usually used in an organic synthesis reaction may be used as appropriate.

The block copolymer (II) also include, for example, those block copolymers described in WO 2006/033296 A (Patent Literature 4), JP H07-69900 A (Patent Literature 5), and JP H06-206815 A (Patent Literature 6), and the block copolymers may be produced according to the descriptions of the patent literatures. In the following description, an embodiment of the method for producing a block copolymer (II) by using an AB block type copolymer in which a polyethylene glycol segment that is preferably used as the main chain polymer of the block copolymer (II), is linked to a polyaspartic acid segment, and introducing a hydrophobic functional group into the copolymer, will be explained.

A polyethylene glycol derivative in which one terminal is an amino group (for example, methoxy polyethylene glycol-1-propylamine) is sequentially reacted with N-carbonylaspartic acid anhydride protected with an appropriate side-chain carboxy group such as a β-benzyl ester, and an AB block type copolymer skeleton in which a polyethylene glycol segment is linked to a polyaspartic acid segment is constructed by sequential polymerization. Subsequently, the AB block type copolymer skeleton is subjected to an appropriate deprotection reaction, and an AB block type copolymer including a plurality of carboxylic acids is synthesized. In a case in which the polyaspartic acid side chain is a β-benzyl ester, a protective group removal reaction may be carried out by hydrolysis under alkali conditions or a hydrogenolysis reaction.

It is desirable that a compound containing a hydrophobic group such as a hydrocarbon group, the compound having an amino group and/or a hydroxy group, is reacted with this AB block type copolymer including a plurality of carboxylic acids, under condensation reaction conditions such as a carbodiimide dehydration condensing agent.

When a hydrophobic compound is bonded via an amide bond using a hydrophobic group-containing compound having an amino group, the reaction may be carried out according to a conventional method that is known as a peptide bond production method. For example, an acid halide method, an acid anhydride method, or a coupling method may be used; however, a coupling method of using a condensing agent is preferred. Regarding the condensing agent, 1-ethyl-(3-dimethylaminopropyl)carbodiimide (EDC), 1-ethyl-(3-dimethylaminopropyl)carbodiimide hydrochloride (EDC·HCl), dicyclohexylcarbodiimide (DCC), carbonylimidazole (CDI), 1-ethoxycarbonyl-2-ethoxy-1,2-dihydroxyquinoline (EEDQ), diphenylphosphoryl azide (DPPA), and the like may be used.

Regarding the condensing agent, it is preferable to use the condensing agent in an amount of 0.5 to 20 times the molar amount of the hydrophobic compound, and it is particularly preferable to use the condensing agent in an amount of 1 to 10 times the molar amount of the hydrophobic compound. Furthermore, at this time, N-hydroxysuccinimide (HONSu), 1-hydroxybenzotriazole (HOBt), N-hydroxy-5-norbornene-2,3-dicarboxylic acid imide (HONB), or the like may also be incorporated.

When a hydrophobic compound is bonded via an ester bond using a hydrophobic group-containing compound having a hydroxy group, the reaction may be carried out according to a conventional method that is known as an ester bond production method. For example, an acid halide method, an acid anhydride method, a coupling method, or the like may be used; however, a coupling method of using a condensing agent is preferred. Regarding the condensing agent, 1-ethyl-(3-dimethylaminopropyl)carbodiimide (EDC), 1-ethyl-(3-dimethylaminopropyl)carbodiimide hydrochloride (EDC·HCl), dicyclohexylcarbodiimide (DCC), carbonylimidazole (CDI), O-(7-azabenzotriazol-1-yl)-N,N,N',N'-tetramethyluronium hexafluorophosphate (HATU), O-(7-azabenzotriazol-1-yl)-N,N,N',N'-tetramethyluronium hexafluorophosphate (HBTU), (1-cyano-2-ethoxy-2-oxoethylideneaminoxy)dimethylaminomorphoinocarbenium hexafluorophosphate (COMU), 1-ethoxycarbonyl-2-ethoxy-1,2-dihydroxyquinoline (EEDQ), diphenylphosphoryl azide (DPPA), and the like may be used.

Regarding the condensing agent, it is preferable to use the condensing agent in an amount of 0.5 to 20 times the molar amount of the hydrophobic compound, and it is particularly preferable to use the condensing agent in an amount of 1 to 10 times the molar amount of the hydrophobic compound. Furthermore, at this time, a reaction aid such as N-hydroxysuccinimide, 1-hydroxybenzotriazole (HOBt), N-hydroxy-5-norbornene-2,3-dicarboxylic acid imide (HOBN), 4-dimethylaminopyridine (DMAP), dimesitylammonium pentafluorobenzenesulfonate, N,N-diisopropylethylamine, or triethylamine may be incorporated, and above all, DMAP is preferred.

When a reaction of bonding a hydrophobic compound to a raw material block copolymer is performed, the amount of use of the hydrophobic compound is not particularly limited; however, the hydrophobic compound is usually used in an amount of 0.1 to 2 mol with respect to 1 equivalent of carboxy groups of the raw material block type copolymer.

It is preferable that the condensation reaction is performed in a solvent, and regarding the solvent, for example, various solvents such as N,N-dimethylformamide (DMF), dimethyl sulfoxide (DMSO), dioxane, tetrahydrofuran (THF), water, and mixed solvents thereof may be used without particular limitations. The amount of use of the solvent is not particularly limited; however, the solvent is usually used in an amount of 1 to 500 times the weight of the raw material copolymer.

It is preferable that the condensation reaction is performed at -10°C to 50°C, and particularly preferably -5°C to 40°C. It is sufficient to perform the reaction for 2 to 48 hours.

The amount of introduction of a hydrophobic functional group such as a hydrocarbon group into the block copolymer (II) may be adjusted by appropriately increasing or decreasing the feed amounts of the various hydrophobic group-containing compounds in the dehydration condensation reaction.

Meanwhile, in a case in which the block copolymer (II) is produced using a carbodiimide condensing agent, a - N(**R**₁₇)CONH(**R**₁₈) group corresponding to **R**₁₆ related to General Formula (3) may be introduced simultaneously with the bonding reaction of a hydrophobic functional group related to **R**₁₅. That is, in the case of using dicyclohexylcarbodiimide (DCC) as a carbodiimide condensing agent, **R**₁₇ and **R**₁₈ of - N(**R**₁₇)CONH(**R**₁₈) both become a cyclohexyl group. In the case of performing a condensation reaction using diisopropylcarbodiimide (DIPCI), **R**₁₇ and **R**₁₈ both become an isopropyl group. In the case of using 1-ethyl-3-(3-dimethylaminopropyl)carbodiimide hydrochloride (WSC), **R**₁₇ and **R**₁₈ of -N(**R**₁₇)CONH(**R**₁₈) become a mixed substituted form of an ethyl group and 3-dimethylaminopropyl.

After completion of the reaction, the block copolymer (II) of the present invention may be produced through an optional purification process. The purification process is not particularly limited, and processes that are usually used may be employed.

Regarding the method for synthesizing polyglutamic acid that is preferably used as the polyamino acid segment of the block copolymer (II), when glutamic acid is obtained using N-carbonylglutamic acid anhydride instead of N-carbonylaspartic acid anhydride in the Synthesis Example described above, and then the hydrocarbon group-containing compound is introduced, a block type copolymer (II) in which the polyamino acid segment is polyglutamic acid may be synthesized.

The present invention relates to a composition including the glutamic acid derivative or a pharmacologically acceptable salt thereof (I) and the block copolymer (II).

The composition of the present invention is a mixture of the glutamic acid derivative (I) and the block copolymer (II). The mixing ratio may be arbitrarily adjusted. The block copolymer (II) is a compound corresponding to an additive for a pharmaceutical preparation, and particularly, as long as the block copolymer does not have any pharmacological activity, the upper limit of the amount of use thereof may be set in consideration of the use convenience as a medicine. Preferably, it is preferable to combine the components at a ratio (I) : (II) of 1 : 0.5 to 100 as a mass ratio of the respective components. The mass ratio is more preferably 1 : 0.5 to 50, and even more preferably 1 : 1 to 50, and it is particularly preferable to combine the glutamic acid derivative and the block copolymer at 1 : 2 to 20.

It is preferable that the composition of the present invention is used in a form in which the glutamic acid derivative (I) and the block copolymer (II) form a complex as a result of an interaction.

There is known a method of obtaining a pharmaceutical composition that achieves an enhancement of efficacy and/or reduction of side effects, by using an amphiphilic polymer such as the block copolymer (II) as a carrier for the medicine, and improving the pharmacokinetics. It is speculated that such a pharmaceutical composition forms a blend as a pharmaceutical compound, which is an active ingredient, and an amphiphilic polymer forms a complex based on an interaction, and exhibits particular pharmacokinetics. Therefore, it is preferable that the composition of the present invention is also a composition obtainable by a production method intended for the formation of such a complex.

Known examples of the method for producing a pharmaceutical composition using an amphiphilic polymer as a pharmaceutical preparation carrier include the following methods a) to c) described in Japanese Patent No. 2777530, and the following method d) described in JP 2001-226294 A.

### a) Method for encapsulating a drug by stirring

A method of dissolving a hydrophobic drug in a water-miscible organic solvent as necessary, and mixing the solution with a block copolymer-dispersed aqueous solution by stirring. Meanwhile, it is also possible to heat the system at the time of mixing by stirring.

### b) Solvent volatilization method

A method of mixing a non-water-miscible organic solvent solution of a hydrophobic drug with a block copolymer-dispersed aqueous solution, and volatilizing the organic solvent while stirring the mixture.

### c) Dialysis method

A method of dispersing and dissolving a hydrophobic drug and a block copolymer in a water-miscible organic solvent, and then dialyzing the resulting solution against a buffer solution and/or water by means of a dialysis membrane.

### d) Emulsion volatilization method

A method of dispersing and dissolving a hydrophobic drug and a block copolymer in a non-water-miscible organic solvent, mixing the resulting solution with water, subsequently stirring the mixture to form an (O/W)-in-water type emulsion, and then volatilizing the organic solvent.

Furthermore, the following method e) is described in JP 2003-342168 A or non-patent literatures (for example, Park, et al., Biomaterials and Drug Delivery Toward New Mellennium, 2000, 321-332; and Lavasanifar, et al., Journal of Controlled Release, 77(2001), 155-160).

### e) Solid solution method

A method of dissolving a hydrophobic drug and a block copolymer in an organic solvent, uniformly mixing the two, subsequently distilling off the solvent so as to produce a solid solution, and then dissolving the solid solution in water at 40°C to 60°C.

In a case in which an organic solvent is used in the method for preparing a composition as described above, any organic solvent capable of being removed by evaporation or dialysis may be used without limitations. Examples of the solvent that is preferable for use include dimethyl sulfoxide (DMSO), N,N-dimethylformamide (DMF), N,N-dimethylacetamide (DMA), dichloromethane, 1,2-dichloroethane, acetic acid, trifluoroacetic acid (TFA), N-methyl-2-pyrrolidone (NMP), 1,3-dimethyl-2-imidazolidinone (DMI), acetone, methanol, ethanol, 2-propanol, 1,1,1,3,3,3-hexafluoro-2-propanol (HFIP), 2,2,2-trifluoroethanol (TFE), methyl acetate, ethyl acetate, cyclohexane, diethyl ether, acetonitrile, tetrahydrofuran, and mixed solvents thereof.

The amount of use of these organic solvent is not particularly limited as long as the amount is an amount capable of dissolving a drug or a block copolymer, and the amount of use may be set as appropriate according to the preparation form.

In the case of producing the composition of the present invention, there are several methods as described above, and there are no particular limitations in any of the methods. However, a production method according to the e) solid solution method will be described below as an example.

A predetermined amount of a glutamic acid derivative (I) and a predetermined amount of a block copolymer (II) are dispersed and dissolved in a volatile organic solvent such as 1,1,1,3,3,3-hexafluoro-2-propanol (HFIP). The solvent may be a single solvent, or a solvent may also be used as a mixture with another volatile solvent for accelerating dissolution. If necessary, a small amount of water may be mixed into the solvent. Dispersion and dissolution may be carried out by optionally heating the system to the boiling point of the solvent in consideration of stability of the glutamic acid derivative. Preferably, the solutes are dissolved by homogeneously stirring the solutes at a temperature lower than or equal to normal temperature and higher than the freezing point of the solvent.

After the solutes are sufficiently dispersed and dissolved, the volatile organic solvent is removed by evaporation under reduced pressure, if necessary. In the present invention, it is not necessarily essential to remove the solvent completely; however, it is desirable that the residue remaining after the solvent removal maintains a paste form or a solid form.

A solid solution resulting from integration of the glutamic acid derivative (I) and the block copolymer (II) is mixed with an aqueous medium (an aqueous medium may be added to the solid solution, or the solid solution may be added to an aqueous medium). Regarding the aqueous medium, it is preferable to use water, or an aqueous solution including formulation additives, which may be used as a pharmaceutical preparation, is also desirable. Examples include an aqueous buffer solution including a phosphoric acid or citric acid buffering agent; an aqueous solution of saccharides such as glucose, maltose, and lactose; inorganic salt solutions such as physiological saline; and aqueous solutions having these additives incorporated therein. The amount ratio between the solid solution and the aqueous medium is not particularly limited as long as the amounts are adequate amounts capable of dispersing; however, it is desirable that the mixture is prepared at a mass/volume (w/v) ratio in the range of 1 : 10 to 1,000.

This is stirred at an appropriate temperature, for example, a temperature of 40°C or lower, and preferably 30°C or lower. If necessary, the mixture may be subjected to an ultrasonic treatment. This stirring is carried out for a time period sufficient for the solid solution containing the glutamic acid derivative (I) and the block copolymer (II) to be almost completely uniformly dispersed. Since the time taken for uniform dispersing varies with the type or the content of the glutamic acid derivative and the block copolymer, the time period is not limited. Subsequently, the uniform dispersion solution thus obtained may be used directly, or may be treated by filtering insoluble matters or precipitates. There are no limitations on the filter membrane to be used; however, the filter membrane is preferably a membrane having a pore size of about 0.1 to 1 µm. The composition according to a preferred embodiment of the present invention that may affect the interaction between the glutamic acid derivative (I) and the block copolymer (II) may be produced in a solution state by the above-described method. Meanwhile, this composition in the solution state may be subjected to cerebral leakage to a predetermined concentration by an optional concentration treatment or ultrafiltration treatment. Furthermore, a composition in a solid state may be produced by a solvent distillation treatment or freeze-drying.

The composition according to a preferred embodiment of the present invention produced by the above-described method may form an associate in an aqueous solution. An aqueous solution of the composition produced by the above-described method is detected as particles having an average particle size of preferably 1 to 1,000 nm by an analysis performed using a light scattering particle size analyzer. In the case of a preferred embodiment, it is detected that particles having a particle size of 1 to 500 nm, and particularly preferably 1 to 100 nm, as an average particle size have been formed.

Since the composition of the present invention uses an amphiphilic block copolymer (II), it is speculated that the composition forms core-shell type micellar associates having a hydrophobic polyamino acid segment as an inner core (core) and a hydrophilic polyethylene glycol segment as an outer shell (shell) in an aqueous medium. Here, since the glutamic acid derivative (I) having hydrophobic properties has an interaction with the hydrophobic polyamino acid segment, a complex is formed such that the glutamic acid derivative (I) is incorporated into the inner cores of the micellar associates. In regard to the composition of the present invention, an embodiment in which the glutamic acid derivative (I) and the block copolymer (II) affect the interaction and form a complex, is preferred.

Since the composition of the present invention that includes a glutamic acid derivative or a pharmaceutically acceptable salt thereof (I) and a block copolymer (II) includes a glutamic acid derivative (I) having physiological activity, the composition may be used as a pharmaceutical composition that employs the glutamic acid derivative as an active ingredient.

It is preferable that the composition of the present invention is usually produced into a pharmaceutical preparation together with additives that are pharmaceutically acceptable.

Examples of the additives include pharmaceutically acceptable additives such as an excipient, a disintegrant, a binder, a lubricating agent, a fluidizing agent, a coating agent, a suspending agent, an emulsifier, a stabilizer, a preservative, a flavoring agent, a fragrance, a diluent, and a dissolution aid, and a pharmaceutical preparation is produced by mixing the composition with these additives.

The preparation is safely administered orally or parenterally (systemic administration, topical administration, and the like) in a dosage form such as a powder preparation, a granular preparation, a pill, a tablet, a capsule, an injectable preparation, a suppository, or an ointment.

The preparation of the present invention is preferably used as an injectable preparation, and usually, water, physiological saline, a 5% glucose or mannitol solution, a water-soluble organic solvent (for example, glycerol, ethanol, dimethyl sulfoxide, N-methylpyrrolidone, polyethylene glycol, Cremophor, and mixed liquids thereof), a mixed liquid of water and the water-soluble organic solvent, and the like are used.

The dosage of the composition varies depending on the route of administration, the age of the patient, and actual symptoms to be prevented or treated, and there are no particular limitations. Since the composition of the present invention includes a glutamic acid derivative (I) that is recognized by GGT, which is expressed at a high level in malignant tumors, and releases a physiologically active substance, it is preferable to use the composition as an antitumor agent.

In the case of using the composition as an antitumor agent, for example, when the composition is orally administered to an adult, the composition may be administered at a dose of 0.01 mg to 2,000 mg, and preferably 0.1 mg to 1,000 mg, per day as an active ingredient, once a day or in several divided portions per day. Furthermore, in the case of administering the composition parenterally by intravenous administration, the composition may be administered at a dose of 0.01 mg/m² to 2,000 mg/m², and preferably 0.1 mg/m² to 1,000 mg/m², as an active ingredient per body surface area, and it is preferable to administer the composition once a day or in several divided portions per day. Administration by injection is performed intravenously, intra-arterially, subcutaneously, or at the affected area (tumor area).

### EXAMPLES

Next, the present invention will be more specifically explained by way of Examples; however, the present invention is not intended to be limited by these Examples.

The LC/MS analysis conditions for the detection, classification, and purity measurement of the compounds of Synthesis Examples 1 to 3 and Comparative Examples 1 and 2 were as follows.
Machine model: Shimadzu LCMS-2010A
Column: INERTSIL ODS-3, 2.1 mm × 100 mm
Mobile phase A: Acetonitrile/formic acid (99.9/0.1)
Mobile phase B: Water/formic acid (99.9/0.1)
Gradient: Time (minutes) 0.0 5.5 6.5 6.51 10.0
   Concentration of A (%) 5 90 90 5 5
   Flow rate: 0.3 mL/min

The LC/MS analysis conditions for the detection, classification, and purity measurement of the compounds of Synthesis Examples 4 to 6 were as follows.
Machine model: Shimadzu LCMS-2020
Column: INERTSIL ODS-3, 2.1 mm × 100 mm
Mobile phase A: Acetonitrile/formic acid (99.9/0.1)
Mobile phase B: Water/formic acid (99.9/0.1)
Gradient: Time (minutes) 0.0 5.5 6.5 6.51 10.0
Concentration of A (%) 20 90 90 20 20 Flow rate: 0.3 mL/min

### Synthesis Example 1

### Synthesis of (((4-((S)-4-amino-4-carboxybutanamide)benzyl)oxy)carbonyl)doxorubicin

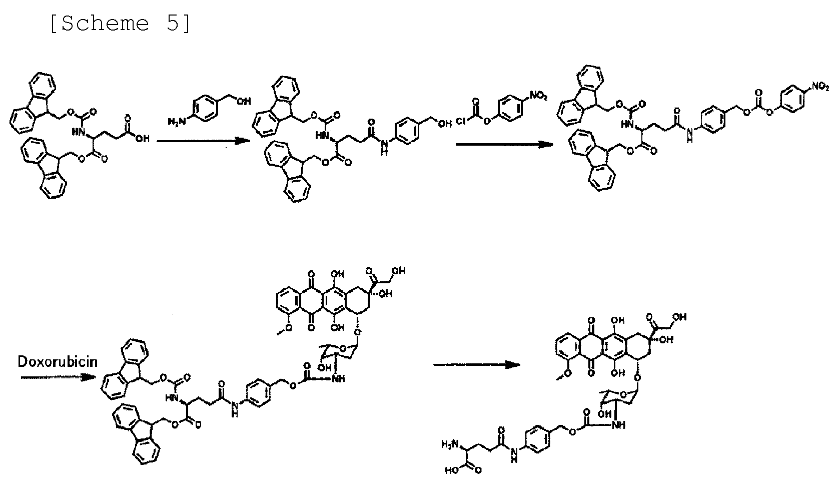

### Synthesis Example 1-1

### Synthesis of (S)-(9H-fluoren-9-yl)methyl 2-((((9H-fluoren-9-yl)methoxy)carbonyl)amino)-5-((4-(hydroxymethyl)phenyl)amino)-5-oxopentanoate

To a dry dichloromethane (5 mL) solution of (S)-5-((9H-fluoren-9-yl)methoxy)-4-((((9H-fluoren-9-yl)methoxy)carbonyl)amino)-5-oxopentanoic acid (0.182 g) and 4-aminobenzyl alcohol (0.049 g), N-ethoxycarbonyl-2-ethoxy-1,2-dihydroquinoline (EEDQ) (0.103 g) was added, and the mixture was stirred for 18 hours at room temperature. Crystals produced by adding 1 N hydrochloric acid thereto were filtered, and (S)-(9H-fluoren-9-yl)methyl 2-((((9H-fluoren-9-yl)methoxy)carbonyl)amino)-5-((4-(hydroxymethyl)phenyl)amino)-5-oxopentanoate (0.173 g) was obtained as a crude product. NMR [400MHz,DMSO-d6,TMS]ppm:1.81-1. 90 (1H,m) ,2.01-2.12(1H,m),2.40-2.46(2H,m),4.17-4.43(9H,m),7.22-7.33(6H,m),7.37-7.44(4H,m),7.55(2H,d),7.68-7.76(4H,m),7.86-7.96(5H,m),9.89(1H,brs).
LC/MS retention time: 7.3 minutes; m/z (ESI, POS): 653 [M+H]⁺

### Synthesis Example 1-2

### Synthesis of (S)-(9H-fluoren-9-yl)methyl 2-((((9H-fluoren-9-yl)methoxy)carbonyl)amino)-5-((4-((((4-nitrophenoxy)carbonyl)oxy)methyl)phenyl)amino)-5-oxopentanoate

To a dry tetrahydrofuran (100 mL) solution of (S)-(9H-fluoren-9-yl)methyl 2-((((9H-fluoren-9-yl)methoxy)carbonyl)amino)-5-((4-(hydroxymethyl)phenyl)amino)-5-oxopentanoate (0.145 g) and pyridine (0.0448 mL), a dry tetrahydrofuran (10 mL) solution of 4-nitrophenyl chloroformate (0.089 g) was added dropwise at 0°C, and the mixture was stirred for 18 hours at room temperature. Water was added thereto, and then the mixture was extracted using ethyl acetate. The organic layer was dried over anhydrous magnesium sulfate, the solvent was dried off under reduced pressure, and (S)-(9H-fluoren-9-yl)methyl 2-((((9H-fluoren-9-yl)methoxy)carbonyl)amino)-5-((4-((((4-nitrophenoxy)carbonyl)oxy)methyl)phenyl)amino)-5-oxopentanoate (0.130 g) was obtained as a crude product.
LC/MS retention time: 8.1 minutes; m/z (ESI, POS): 840 [M+Na]⁺

### Synthesis Example 1-3

### Synthesis of ((((4-((S)-5-((9H-fluoren-9-yl)methoxy)-4-(((9H-fluoren-9-yl)methoxy)carbonyl)amino)-5-oxopentanamido)benzyl)oxy)carbonyl)doxorubicin

Crude (S)-(9H-fluoren-9-yl)methyl 2-((((9H-fluoren-9-yl)methoxy)carbonyl)amino)-5-((4-((((4-nitrophenoxy)carbonyl)oxy)methyl)phenyl)amino)-5-oxopentanoate (0.05 g) was dissolved in N,N-dimethylformamide (3 mL), doxorubicin hydrochloride (0.03 g) was added thereto, and then diisopropylethylamine (0.13 mL) was added to the mixture. After the mixture was stirred for 15 hours at room temperature, water was added to the mixture, and the mixture was extracted with ethyl acetate. The organic layer was dried over anhydrous magnesium sulfate, subsequently the solvent was distilled off under reduced pressure, and ((((4-((S)-5-((9H-fluoren-9-yl)methoxy)-4-(((9H-fluoren-9-yl)methoxy)carbonyl)amino)-5-oxopentanamido)benzyl)oxy)carbonyl)doxorubicin (0.09 g)was obtained as a crude product.
LC/MS retention time: 7.8 minutes; m/z (ESI, POS): 1245 [M+Na]⁺

### Synthesis Example 1-4

### Synthesis of (((4-((S)-4-amino-4-carboxybutanamido)benzyl)oxy) carbonyl)doxorubicin

Crude ((((4-((S)-5-((9H-fluoren-9-yl)methoxy)-4-(((9H-fluoren-9-yl)methoxy)carbonyl)amino)-5-oxopentanamido)benzyl)oxy)carbonyl)doxorubicin (0.085 g) was dissolved in N,N-dimethylformamide (1.1725 mL), and a solution of piperidine in N,N-dimethylformamide solution (10% 0.275 mL) was added dropwise to the solution under ice cooling. The mixture was stirred for 30 minutes. Water (4 mL) was added thereto, and the mixed solution was purified by preparative HPLC. Thus, (((4-(4-amino-4-carboxybutanamido)benzyl)oxy)carbonyl)doxorubicin (Synthesis Example 1, 0.025 g) was obtained. NMR[400MHz,DMSO-d₆,TMS]ppm:1.11(3H,d),1.37-1.51(2H,m),1.76-1.92(4H,m),2.07-2.22(2H,m),2.62-2.69(1H,m),2.98-3.12(3H,m),3.19-3.28(1H,m),3.67-3.76(1H,m),3.98(3H,s),4.10-4.17(1H,m),4.56(2H,s),4.69-4.73(1H,m),4.87-4.98(5H,m),5.22(1H,s),5.46(1H,s),6.84(1H,d),7.23(2H,d),7.53(2 H,d),7.63(1H,d),7.88-7.95(2H,m),10.42(1H,brs).
LC/MS retention time: 4.0 minutes; m/z (ESI, POS): 822 [M+H]⁺

### Synthesis Example 2

### Synthesis of (((4-((S)-4-amino-4-carboxybutanamido)benzyl)oxy)carbonyl)-10-oxy-7-ethylcamptothecin

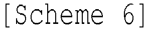

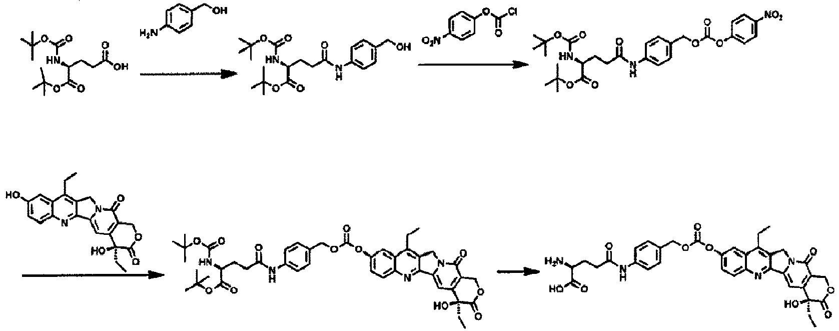

### Synthesis Example 2-1

### Synthesis of t-butyl (S)-2-((t-butoxycarbonyl)amino)-5-((4-(hydroxymethyl)phenyl)amino)-5-oxopentanoate

To a dry dichloromethane (15 mL) solution of (S)-5-(t-butoxy)-4-((t-butoxy)carbonyl)amino)-5-oxopentanoic acid (1.00 g) and 4-aminobenzyl alcohol (0.487 g), N-ethoxycarbonyl-2-ethoxy-1,2-dihydroquinoline (EEDQ) (1.019 g) was added, and the mixture was stirred for 18 hours at room temperature. Then, 1 N hydrochloric acid was added to the mixture, and the mixture was extracted with dichloromethane. The organic layer was washed with water and then was dried over anhydrous sodium sulfate. The solvent was distilled off under reduced pressure, the residue was washed with diethyl ether, and t-butyl (S)-2-((t-butoxycarbonyl)amino)-5-((4-(hydroxymethyl)phenyl)amino)-5-oxopentanoate (0.890 g) was obtained. NMR[400MHz,CDCl₃,TMS]ppm:1.46(9H,s),1.47(9H,s),1.82-1.89(1H,m),2.27-2.30(2H,m),2.44(2H,t),4.21-4.24(1H,m),4.66(2H,s),5.35-5.37(1H,m),7.33(2H,d),7.62(2H,d),8.87(1H,brs).
LC/MS retention time: 5.5 minutes; m/z (ESI, POS): 431 [M+Na]⁺

### Synthesis Example 2-2

### Synthesis of t-butyl (S)-2-((t-butoxycarbonyl)amino)-5-((4-((((4-nitrophenoxy)carbonyl)oxy)methyl)phenyl)amino)-5-oxopentanoate

To a dry tetrahydrofuran (10 mL) solution of t-butyl (S)-2-((t-butoxycarbonyl)amino)-5-((4-(hydroxymethyl)phenyl)amino)-5-oxopentanoate (0.10 g) and pyridine (0.0494 mL), a dry tetrahydrofuran (10 mL) solution of 4-nitrophenyl chloroformate (0.0987 g) was added dropwise at 0°C, and the mixture was stirred for 2 hours at room temperature. An aqueous solution of citric acid was added thereto, and then the mixture was extracted with ethyl acetate. The organic layer was washed with water and then was dried over anhydrous sodium sulfate. The solvent was distilled off under reduced pressure, the residue was purified by silica gel column chromatography, and t-butyl (S)-2-((t-butoxycarbonyl)amino)-5-((4-((((4-nitrophenoxy)carbonyl)oxy)methyl)phenyl)amino)-5-oxopentanoate (0.038 g) was obtained. NMR[400MHz,CDCl₃,TMS]ppm:1.46(9H,s)1.48(9H,s)1.79-1.90(1H,m),2.26-2.28(1H,m),2.45(2H,t),4.20-4.28(1H,m),5.26(2H,s),5.38-5.40(1H,m),7.37(2H,d),7.41(2H,d),7.69(2H,d),8.27(2H,d),9.15(1 H,brs).
LC/MS retention time: 7.1 minutes; m/z (ESI, POS): 596 [M+Na]⁺

### Synthesis Example 2-3

### Synthesis of (((4-((S)-5-(t-butoxy)-4-((t-butoxycarbonyl)amino)-5-oxopentanamido)benzyl)oxy)carbonyl)-10-oxy-7-ethylcamptothecin

t-Butyl (S)-2-((t-butoxycarbonyl)amino)-5-((4-((((4-nitrophenoxy)carbonyl)oxy)methyl)phenyl)amino)-5-oxopentanoate (0.125 g) was dissolved in N,N-dimethylformamide (8 mL), 7-ethyl-10-hydroxycamptothecin (0.0855 g) was added thereto, and then diisopropylethylamine (0.37 mL) was added thereto. After the mixture was stirred for 18 hours at room temperature, water was added to the mixture, and the mixture was extracted with ethyl acetate. The organic layer was dried over anhydrous sodium sulfate, subsequently the solvent was distilled off under reduced pressure, and (((4-((S)-5-(t-butoxy)-4-((t-butoxycarbonyl)amino)-5-oxopentanamido)benzyl)oxy)carbonyl)-10-oxy-7-ethylcamptothecin (0.227 g) was obtained as a crude product.
LC/MS retention time: 6.6 minutes; m/z (ESI, POS): 827 [M+H]⁺

### Synthesis Example 2-4

### Synthesis of (((4-((S)-4-amino-4-carboxybutanamido)benzyl)oxy)carbonyl)-10-oxy-7-ethylcamptothecin

Crude ((((4-((S)-5-(t-butoxy)-4-((t-butoxycarbonyl)amino)-5-oxopentanamido) benzyl) oxy) carbonyl)-10-oxy-7-ethylcamptothecin (0.02 g) was dissolved in a 4 N hydrochloric acid-dioxane solution (2.0 mL), and the solution was stirred for 30 minutes. The solvent was distilled off, water (4 mL) was added to the residue, and the mixed solution was purified by preparative HPLC. Thus, (((4-((S)-4-amino-4-carboxybutanamido)benzyl)oxy)carbonyl)-10-oxy-7-ethylcamptothecin (Synthesis Example 2, 0.0015 g) was obtained.
NMR[400MHz,DMSO-d₆,TMS]ppm:0.88(3H,t),1.29(3H,t),1.84-2.11(6H,m),3.16-3.29(3H,m),5.28(2H,s),5.36(2H,s),5.45(2H,s),6.55(1H,s),7.34(1 H,s),7.44(2H,d),7.65(2H,d),7.78-7.80(1H,m),8.18-8.25(2H,m),10.21(1H,brs).
LC/MS retention time: 3.9 minutes; m/z (ESI, POS): 671 [M+H]⁺

### Synthesis Example 3

### Synthesis of (((4-((S)-4-amino-4-carboxybutanamido)benzyl)oxy)carbonyl)camptothecin

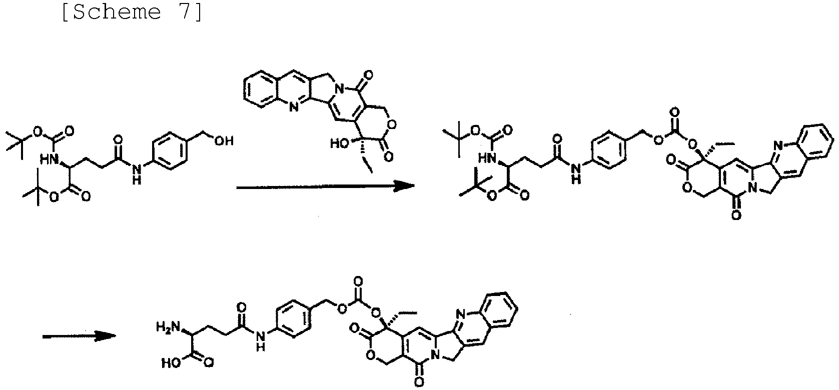

### Synthesis Example 3-1

### Synthesis of (((4-((S)-5-(t-butoxy)-4-((t-butoxycarbonyl)amino)-5-oxopentanamido)benzyl)oxy)carbonyl)camptothecin

To a dry dichloromethane (6 mL) suspension of camptothecin (0.050 g) and triphosgene (0.0158 g), a dichloromethane (2 mL) solution of dimethylaminopyridine (0.0561 g) was slowly added dropwise. After the mixture was stirred for 30 minutes, t-butyl (S)-2-((t-butoxycarbonyl)amino)-5-((4-(hydroxymethyl)phenyl)amino)-5-oxopentanoate (0.059 g) was added to the mixture, and the mixture was stirred for 18 hours at room temperature. 1 N hydrochloric acid (50 mL) was added thereto, and the mixture was extracted two times with dichloromethane. The organic layer was washed with a saturated saline solution and then was dried over anhydrous magnesium sulfate. The solvent was distilled off under reduced pressure, and (((4-((S)-5-(t-butoxy)-4-((t-butoxycarbonyl)amino)-5-oxopentanamido)benzyl)oxy)carbonyl)camptothecin (0.108 g) was obtained as a crude product.
LC/MS retention time: 6.8 minutes, m/z (ESI, POS): 805 [M+Na]⁺

### Synthesis 3-2

### Synthesis of (((4-((S)-4-amino-4-carboxybutanamido)benzyl)oxy)carbonyl)camptothecin

Crude (((4-((S)-5-(t-butoxy)-4-((t-butoxycarbonyl)amino)-5-oxopentanamido)benzyl)oxy)carbonyl)camptothecin (0.028 g) was dissolved in a 4 N hydrochloric acid-ethyl acetate solution (3.0 mL) at 0°C, and the solution was stirred for 3 hours. The solvent was distilled off, the residue thus obtained was purified by preparative HPLC, and (((4-((S)-4-amino-4-carboxybutanamido)benzyl)oxy)carbonyl)camptothecin (Synthesis Example 3, 0.0017 g) was obtained. NMR[400MHz,DMSO-d₆,TMS]ppm:0.90(3H,t),1.90-1.97(3H,m),2.13-2.21(3H,m),3.16-3.28(1H,m),5.09(2H,q),5.33(2H,s),5.52(2H,s),7.02(1H,s),7.27(2 H,d),7.53(2H,d),7.74(1H,t),7.89(1H,t),8.15-8.21(2H,m),8.73(1H,s),10.29(1H,brs).
LC/MS retention time: 3.9 minutes; m/z (ESI, POS): 627 [M+H]⁺

### Synthesis Example 4

### Synthesis of (((4-((S)-4-amino-4-carboxybutanamido)benzyl)oxy) carbonyl)epirubicin

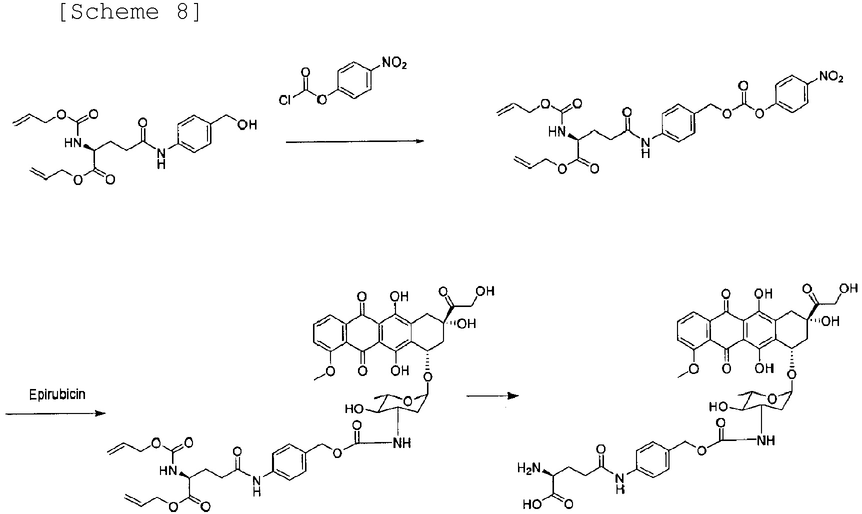

### Synthesis Example 4-1

### Synthesis of allyl (S)-2-(allyloxycarbonylamino)-5-((4-((((4-nitrophenoxy)carbonyl)oxy)methyl)phenyl)amino)-5-oxopentanoate

To a dry tetrahydrofuran (20 mL) solution of allyl (S)-2-((allyloxycarbonyl)amino)-5-((4-(hydroxymethyl)phenyl)amino)-5-oxopentanoate (0.400 g) and pyridine (0.214 mL), a dry tetrahydrofuran (1 mL) solution of 4-nitrophenyl chloroformate (0.428 g) was added dropwise at 0°C, and the mixture was stirred for 18 hours at room temperature. 1 N hydrochloric acid (10 mL) was added thereto, and then the mixture was extracted with ethyl acetate. The organic layer was washed with water and then was dried over anhydrous sodium sulfate. The solvent was distilled off under reduced pressure, the residue was purified by silica gel column chromatography, and allyl (S)-2-((allyloxycarbonyl)amino)-5-((4-((((4-nitrophenoxy)carbonyl)oxy)methyl)phenyl)amino)-5-oxopentanoate (0.633 g) was obtained.
NMR[400MHz,CDCl₃,TMS]ppm:1.96-2.08(1H,m)2.34-2.44(1H,m),2.48-2.53(2H,m),4.42-4.50(1H,m),4.62(2H,d),4.70(2H,d),5.24-5.39(6H,m),5.63(1H,brd),5.86-5.97(2H,m),7.39(2H,d),7.43(2H,d),7.65(2H,d),8.30(2H,d),8.41(1 H,brs).
LC/MS retention time: 6.6 minutes; m/z (ESI, POS): 564 [M+Na]⁺

### Synthesis Example 4-2

### Synthesis of (((4-((S)-5-allyl-4-(allyloxycarbonylamino)-5-oxopentanamido)benzyl)oxy)carbonyl)epirubicin

Allyl (S)-2-(allyloxycarbonylamino)-5-((4-((((4-nitrophenoxy)carbonyl)oxy)methyl)phenyl)amino)-5-oxopentanoate (0.130 g) and epirubicin hydrochloride (0.127 g) were dissolved in N,N-dimethylformamide (10 mL), and diisopropylethylamine (0.0928 mL) was added to the solution. After the mixture was stirred for 6 hours at room temperature, the reaction liquid was added dropwise to diisopropyl ether (200 mL). A solid thus produced was dissolved in chloroform, and the solvent was distilled off under reduced pressure. The residue was purified by silica gel column chromatography (chloroform/methanol = 20/1), and (((4-((S)-5-allyl-4-(allyloxycarbonylamino)-5-oxopentanamido)benzyl)oxy)carbonyl)epirubicin (0.222 g) was obtained. NMR[400MHz,DMSO-d₆,TMS]ppm:1.18(3H,d),1.53-1.64(1H,m),1.79-1.91(2H,m),2.02-2.24(3H,m),2.38-2.46(2H,m),2.90-3.02(3H,m),3.49-3.59(1H,m),3.84-3.93(1H,m),3.98(3H,s),4.06-4.13(1H,m),4.45-4.49(2H,m),4.53-4.61(4H,m),4.83-4.88(3H,m),4.93-4.98(2H,m),5.16-5.23(3H,m),5.26-5.34(2H,m),5.49(1H,s),5.83-5.96(2H,m),7.02(1H,d),7.23(2H,d),7.53(2H,d),7.65(1H,t),7.75(1 H,d),7.91(2H,d),9.93(1H,s),13.27(1H,brs),14.03(1H,brs).
LC/MS retention time: 6.2 minutes; m/z (ESI, POS): 968 [M+Na]⁺

### Synthesis Example 4-3

### Synthesis of (((4-((S)-4-amino-4-carboxybutanamido)benzyl)oxy)carbonyl)epirubicin

(((4-((S)-5-allyl-4-(allyloxycarbonylamino)-5-oxopentanamido)benzyl)oxy)carbonyl)epirubicin (0.200 g) was dissolved in dichloromethane (4.5 mL) and N,N-dimethylformamide (1.0 mL), and tetrakis(triphenylphosphine)palladium (0.012 g) and phenylsilane (0.026 mL) were added to the solution. The mixture was stirred for 30 minutes in an argon atmosphere. The reaction liquid was added dropwise to 10% methanol-containing diisopropyl ether (450 mL). A solid thus produced was purified by silica gel column chromatography (chloroform/methanol/water = 13/6/1), and (((4-((S)-4-amino-4-carboxybutanamido)benzyl)oxy)carbonyl)epirubicin (Synthesis Example 4, 0.080 g) was obtained.
NMR[400MHz, DMSO-d₆,TMS]ppm:
1.18(3H,d),1.52-1.67(1H,m),1.80-1.97(3H,m),2.13-2.23(2H,m),2.90-3.05(3H,m),3.15-3.23(2H,m),3.49-3.61(1H,m),3.84-3.93(1H,m),3.99(3H,s),4.56(2H,s),4.87(2H,s),4.92-5.01(2H,m),5.21(1H,s),5.51(1H,s),7.03(1H,d),7.23(2H,d),7.54(2 H,d),7.66(1H,t),7.91(2H,d),10.36(1H,s),14.04(1H,brs).
LC/MS retention time: 4.4 minutes; m/z (ESI, POS): 822 [M+H]⁺

### Synthesis Example 5

### Synthesis of (((4-((S)-4-amino-4-carboxybutanamido)benzyl)oxy)carbonyl)cytarabine

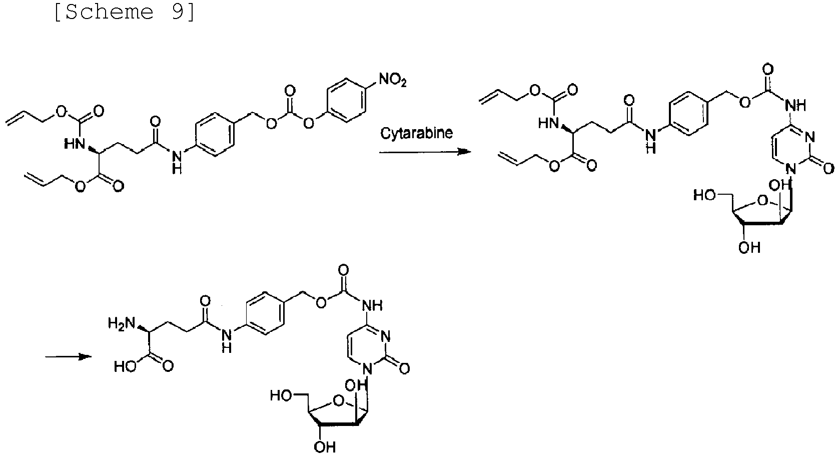

### Synthesis Example 5-1

### Synthesis of (((4-((S)-5-allyl-4-(allyloxycarbonylamino)-5-oxopentanamido)benzyl)oxy)carbonyl)cytarabine

Allyl (S)-2-(allyloxycarbonylamino)-5-((4-((((4-nitrophenoxy)carbonyl)oxy)methyl)phenyl)amino)-5-oxopentanoate (0.030 g) was dissolved in tetrahydrofuran (1.85 mL), cytarabine (0.014 g) was added to the solution, and then a 1 N aqueous solution of sodium hydroxide (0.15 mL) was added thereto. The mixture was stirred for 3 hours at room temperature, subsequently ethyl acetate was added thereto, and an organic layer was obtained. The organic layer thus obtained was dried over anhydrous sodium sulfate, subsequently the solvent was distilled off under reduced pressure, and the residue was purified by silica gel column chromatography (chloroform/methanol = 20/1 to 5/1). Thus, (((4-((S)-5-allyl-4-(allyloxycarbonylamino)-5-oxopentanamido)benzyl)oxy)carbonyl)cytarabine (0.016 g) was obtained.
NMR[400MHz,DMSO-d₆,TMS]ppm:1.79-1.93 (1H,m) 2.02-2.34(1H,m),3.49-3.63(2H,m),4.05-4.16(1H,m),4.47(2H,d),4.60(2H,d),4.92-5.09(3H,m),5.16-5.34(4H,m),5.68(1H,d),5.83(1H,d),5.84-5.97(2H,m),6.17(1H,d),7.24(2H,d),7.56(2H,d),7.63(1H,d),7.76(1 H,d),10.00(1H,brs).
LC/MS retention time: 3.6 minutes; m/z (ESI, POS): 646 [M+H]⁺

### Synthesis Example 5-2

### Synthesis of (((4-((S)-4-amino-4-carboxybutanamido)benzyl)oxy)carbonyl)cytarabine

(((4-((S)-5-allyl-4-(allyloxycarbonylamino)-5-oxopentanamido)benzyl)oxy)carbonyl)cytarabine (0.016 g) was dissolved in dichloromethane (2.0 mL) and N,N-dimethylformamide (0.40 mL), and in an argon atmosphere, tetrakis(triphenylphosphine)palladium(0) (0.0028 g) and phenylsilane (0.0030 mL) were added to the solution. The mixture was stirred for 45 minutes at room temperature and then was left to stand as received for 18 hours. The solvent was distilled off, and the residue was purified by preparative HPLC. Thus, (((4-((S)-4-amino-4-carboxybutanamido)benzyl)oxy)carbonyl)cytarabine (Synthesis Example 5, 0.0015 g) was obtained.
NMR[400MHz,DMSO-d₆,TMS]ppm:2.02-2.14(2H,m),3.53-3.65(2H,m),3.73-3.81(1H,m),3.90-3.99(1H,m),4.06-4.12(1H,m),4.94-5.11(4H,m),5.71-5.5.75(1H,m),5.82-5.85(1H,m), 6.16 (1H,d), 7.25 (2H,d), 7.57 (2H,d), 7.95 (1H,s), 8.05-8.30 (2H,m), 10.08 (1H,brs).
LC/MS retention time: 0.8 minutes; m/z (ESI, POS): 522 [M+H]⁺

### Synthesis Example 6

### Synthesis of 10-(4-((S)-4-amino-4-carboxybutanamido)benzyloxy)-7-ethylcamptothecin ditrifluoroacetate

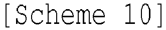

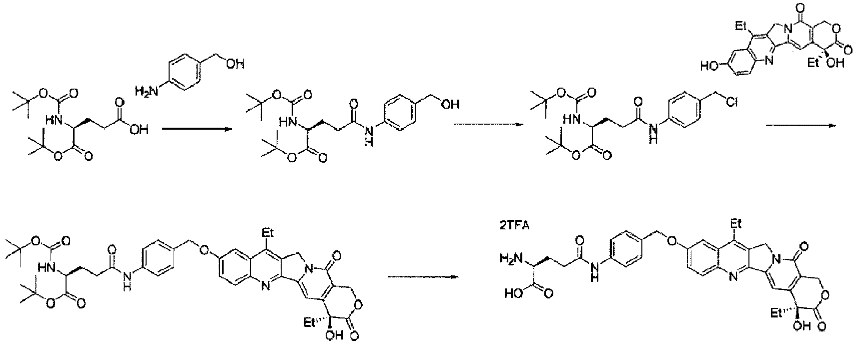

### Synthesis Example 6-1

### Synthesis of 5-((4-hydroxymethyl)benzylamino)-1-(t-butyl) N-(t-butoxycarbonyl)-L-glutamate

To a dry dichloromethane (15 mL) solution of 1-t-butyl N-(t-butoxycarbonyl)-L-glutamate (1.00 g) and 4-aminobenzyl alcohol (0.487 g), N-ethoxycarbonyl-2-ethoxy-1,2-dihydroquinoline (EEDQ) (1.019 g) was added, and the mixture was stirred for 18 hours at room temperature. 1 N hydrochloric acid was added thereto, and the mixture was extracted with dichloromethane. The organic layer was washed with water and then was dried over anhydrous sodium sulfate. The solvent was distilled off under reduced pressure, the residue was washed with diethyl ether, and 5-((4-hydroxymethyl)benzylamino)-1-(t-butyl) N-(t-butoxycarbonyl)-L-glutamate (0.890 g) was obtained. NMR[400MHz,CDCl₃,TMS]ppm:1.46(9H,s), 1.47 (9H,s), 1.82-1.89 (1H,m), 2.27-2.30 (2H,m), 2.44 (2H,t), 4.21-4.24 (1H,m), 4.66 (2H,s), 5.35-5.37 (1H,m), 7 .33 (2H,d), 7.62 (2H,d), 8.87 (1H,brs).
LC/MS retention time: 5.6 minutes; m/z (ESI, POS): 431 [M+Na]⁺

### Synthesis Example 6-2

### Synthesis of 5-((4-chloromethyl)benzylamino)-1-(t-butyl) N-(t-butoxycarbonyl)-L-glutamate

In an argon atmosphere, while the system was stirred under ice cooling, diisopropylethylamine (0.073 mL, 0.43 mmol) and methanesulfonyl chloride (0.025 mL, 0.32 mmol) were added in this order to a dry dichloromethane (4.3 mL) solution of 5-((4-hydroxymethyl)benzylamino)-1-(t-butyl) N-(t-butoxycarbonyl)-L-glutamate (88 mg, 0.22 mmol), and the mixture was stirred for 2 hours and 5 minutes under ice cooling. Ethyl acetate (40 mL) was added to the reaction liquid to dilute the reaction liquid, the reaction liquid was washed with an aqueous solution of 0.3 mol of sodium hydrogen carbonate (3 mL)-water (10 mL), water (10 mL), and saline (10 mL), and then the reaction liquid was dried over anhydrous sodium sulfate. Sodium sulfate was removed by filtration, the solvent was distilled off under reduced pressure, and 5-((4-chloromethyl)benzylamino)-1-(t-butyl) N-(t-butoxycarbonyl)-L-glutamate (83.7 mg) was obtained. This was used in the subsequent condensation reaction without purification.

### Synthesis Example 6-3

### Synthesis of 10-(((4-((S)-5-(t-butoxy)-4-((t-butoxycarbonyl)amino)-5-oxopentanamido)benzyloxy)-7-ethylcamptothecin

In an argon atmosphere, while the system was stirred at room temperature, cesium carbonate (24 mg, 0.074 mmol) was added to a dry dimethylformamide (1.5 mL) suspension of 7-ethyl-10-hydroxycamptothecin (EHC 29 mg, 0.074 mmol), and a pale yellow suspension thus obtained was stirred for 9 minutes at room temperature to obtain an orange-colored uniform solution. Subsequently, a dry dimethylformamide (1.5 mL) solution of crude 5-((4-chloromethyl)benzylamino)-1-(t-butyl) N-(t-butoxycarbonyl)-L-glutamate (36 mg, 0.084 mmol) was added to the solution while being stirred at room temperature, and an orange-colored solution thus obtained was stirred for 2 hours and 40 minutes at room temperature and then for 1.5 hours under ice cooling. While the system was stirred under ice cooling, ethyl acetate (20 mL) was added to the reaction to dilute the reaction liquid, and an aqueous solution of ammonium chloride (6 mL) was added thereto to terminate the reaction. An organic layer was separated, and the organic layer was washed with water (8 mL, two times) and saline (6 mL) and then was dried over anhydrous sodium sulfate. Sodium sulfate was removed by filtration, the solvent was distilled off under reduced pressure, and a pale yellow solid (61 mg) was obtained. This was purified by preparative thin layer chromatography (silica gel, ethyl acetate) . Thus, 10-(((4-((S)-5-(t-butoxy)-4-((t-butoxycarbonyl)amino)-5-oxopentanamido)benzyloxy)-7-ethylcamptothecin (19 mg) was obtained. NMR[400MHz,CDCl3,TMS]ppm:1.033(3H,t,J=7.3Hz),1.353(3H,t,J=7.7 Hz),1.459(9H,s),1.473(9H,s),1.80-1.95(3H,m),2.289(lH,m),2.453(2H,t,J=6.5Hz),3.108
(2H,q, J=7.7Hz), 3.828(1H,s), 4.229(1H,m), 5.205(2H,s), 5.220 (2H,s), 5.298(1H,d,J=16.2Hz), 5.389(1H,d,J=8.1Hz), 5.745(1H,d,J= 16.2Hz, 7.368(1H, d, J=2.6Hz), 7.463(2H, d, J=8.6Hz), 7.510(1H, dd, J= 9.2Hz, 2.6Hz), 7.600(1H,s), 7.693(2H, d, J=8.2Hz), 8.125(1H, d, J=9.2 Hz), 9.062 (1H, s) .
LC/MS retention time: 6.9 minutes; m/z (ESI, NEG): 781 [M-H]⁻

### Synthesis Example 6-4

### Synthesis of 10-(4-((S)-4-amino-4-carboxybutanamido)benzyloxy)-7-ethylcamptothecin ditrifluoroacetate

In an argon atmosphere, while the system was stirred under ice cooling, trifluoroacetic acid (1 mL) was added to a 1,2-dichloroethane (3 mL) solution of 10-(((4-((S)-5-(t-butoxy)-4-((t-butoxycarbonyl)amino)-5-oxopentanamido)benzyloxy)-7-ethylcamptothecin (33.7 mg, 0.043 mmol), and a yellow solution thus obtained was stirred for 5 minutes under ice cooling and then for 2 hours at room temperature (21°C). The reaction liquid was exsiccated under reduced pressure, 1,2-dichloroethane (8 mL) was added to the residue, and the mixture was exsiccated again under reduced pressure. Thus, a yellow solid (41 mg) was obtained. Dimethylformamide (1 mL), acetonitrile (MeCN, 7 mL), and water (4 mL) were added to this solid to dissolve the solid, and a solution (12 mL) thus obtained was separated and purified by preparative liquid chromatography (4 mL, three times). Fractions that did not contain impurities were combined, and the solvent was distilled off under reduced pressure. Thus, 10-(4-((S)-4-amino-4-carboxybutanamido)benzyloxy)-7-ethylcamptothecin ditrifluoroacetate (Synthesis Example 6, 21.8 mg) was obtained.
NMR[400MHz,DMSO-d6, TMS]ppm:0.874(3H, t, J=7.3Hz), 1.272(3H, t, J=7.6Hz), 1.864(2H, m ),2.082(2H,m),2.566(overlapped),3.185(2H,q,J=7.3Hz),3.980(ove rlapped),5.299,5.309,5.429(each 2H, s), 6.50(1H, b), 7.269(1H, s), 7.47-7.65(6H, m),8.09(1H, d, J=9.0Hz), 8.262(2H, d, J=4.1Hz), 10.105(1H, s ),13.9(1H,b) .
LC/MS retention time: 4.1 minutes; m/z (ESI, POS): 628 [M+2H]⁺

### Comparative Example 1

### Synthesis of (S)-(4-amino-4-carboxybutanamido)doxorubicin

### Comparative Example 1-1

(S)-5-((9H-fluoren-9-yl)methoxy)-4-((((9H-fluoren-9-yl)methoxy)carbonyl)amino)-5-oxopentanoic acid (0.100 g), doxorubicin hydrochloride (0.095 g), hydroxybenzotriazole (3 mg), and triethylamine (0.024 mL) were dissolved in N,N-dimethylformamide (2 mL), and 1-ethyl-3-(3-dimethylaminopropyl)carbodiimide hydrochloride (0.100 g) was added to the solution. The mixture was stirred for 22 hours at room temperature. A precipitate produced by adding water (40 mL) to the mixture was filtered. The precipitate was purified by silica gel column chromatography, and (4-((S)-5-((9H-fluoren-9-yl)methoxy)-4-(((9H-fluoren-9-yl)methoxy)carbonyl)amino)-5-oxopentanamido)doxorubicin (0.090 g) was obtained.
NMR[400MHz, DMSO-d₆, TMS]ppm:1.13(3H, d), 1.42-1.54(1H, m),1.64-1.94(3H,m),2.06-2.25(4H,m),2.90-3.04(2H,m),3.90-4.38(10H, m), 4.59(2H, d), 4.77(1H, d), 4.88-4.99(2H, m), 5.20-5.26(1H, m), 5.50(1H, s), 7.20-7.89(19H,m),13.28(1H,s),14.07(1H,s).
LC/MS retention time: 7.6 minutes; m/z (ESI, POS): 1096 [M+Na]⁺

### Comparative Example 1-2

(4-((S)-5-((9H-fluoren-9-yl)methoxy)-4-(((9H-fluoren-9-yl)methoxy)carbonyl)amino)-5-oxopentanamido)doxorubicin (0.020 g) was dissolved in dichloromethane (0.1365 mL), a dichloromethane solution of piperidine (10% v/v, 0.635 mL) was added to the solution at 0°C, and the mixture was stirred for 3.5 hours. Dichloromethane (1 mL) was added thereto, and then a dichloromethane solution of piperidine (10% v/v, 0.0158 mL) was added to the mixture at 0°C. Stirring was continued for 2 hours. A precipitate was filtered and washed with ethyl acetate. The precipitate thus obtained was purified by preparative HPLC, and (S)-(4-amino-4-carboxybutanamido)doxorubicin (Comparative Example 1, 0.0021 g) was obtained.
NMR[400MHz, D₂O, TMS]ppm:1.17(3H, d), 1.61-1.74(1H, m), 1.76-1.93(2H, m), 1.94-2.04(2H, m), 2.11-2.20(1H, m), 2.24-2.50(3H, m), 2.66-2.78(1H, m)3.59(2H, m),3.75(3H, s), 3.99-4.16(2H,m),5.25(1H,m),7.02-7.15(2H,m),7.35-7.42(1H,m).
LC/MS retention time: 3.8 minutes; m/z (ESI, POS): 673 [M+H]⁺

### Comparative Example 2

### Synthesis of 9-((S)-4-amino-4-carboxybutanamido)camptothecin

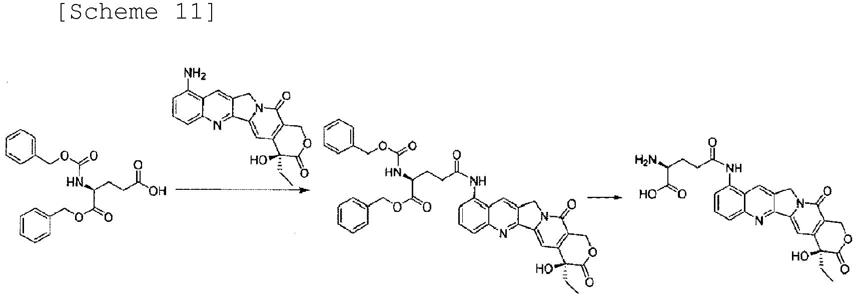

### Comparative Example 2-1

(S)-5-(benzyloxy)-4-(benzyloxycarbonyl)amino-5-oxopentanoic acid (0.173 g) was added to a N,N-dimethylformamide (5 mL) solution of 9-aminocamptothecin (0.090 g), and then hydroxybenzotriazole (0.005 g) and 1-(3-dimethylaminopropyl)-3-ethylcarbodiimide (0.297 g) were added to the mixture. The mixture was stirred for 2 hours at room temperature and then was left to stand for 2 days. Water (10 mL) was added to the mixture, and the mixture was extracted with ethyl acetate. The organic layer was dried over anhydrous sodium sulfate, and then the solvent was distilled off under reduced pressure. The residue was purified by silica gel column chromatography (hexane/ethyl acetate = 1/5 to 0/1), and 9-((4-((S)-5-(benzyloxy)-4-(benzyloxycarbonyl)amino-5-oxo)pentanamido) camptothecin (0.024 g) was obtained.
NMR[400MHz,DMSO-d₆, TMS]ppm:0.89(3H, t), 1.81-2.02(4H, m), 2.13-2.29(1H, m), 2.58-2.70(2H, m), 4.22-4.30(1H, m), 5.08(2H, d), 5.17(2H, s), 5.28(2H, s), 5.44(2H, s), 6.56(1 H,s),7.23-7.40(11H,m),7.76-7.87(2H,m),7.92(1H,d),8.03(1H,d),8.75(1H,s),10.20(1H,s).
LC/MS retention time: 5.8 minutes; m/z (ESI, POS): 717 [M+H]⁺

### Comparative Example 2-2

9-((4-((S)-5-(benzyloxy)-4-(benzyloxycarbonyl)amino-5-oxo)pentanamido)camptothecin (0.020 g) was dissolved in N,N-dimethylformamide (1 mL), 10% palladium-carbon (0.005 g) was added to the solution, and the mixture was stirred for 2 hours at room temperature in a hydrogen atmosphere. The reaction liquid was filtered through Celite, and the filtrate was washed with a mixed solution of N,N-dimethylformamide (1 mL) and water (8 mL). The filtrate was purified by preparative HPLC, and 9-((S)-4-amino-4-carboxybutanamido)camptothecin (0.012 g) was obtained. NMR[400MHz,D₂O,TMS]ppm:0.86(3H, t),1.85(2H, q), 2.26(2H, q), 2.71-2.82(2H, m), 3.97(1H, m),5.24-5.35(2H, m),7.21(1H, s), 7.39-7.44(1H, m), 7.49(2H, d), 8.30(1H, s).
LC/MS retention time: 1.2 minutes; m/z (ESI, POS): 493 [M+H]⁺

### Test Example 1: γ-Glutamyl transpeptidase (GGT) enzyme recognition test

The doxorubicin-bonded glutamic acid derivative (0.49 mg) of Synthesis Example 1 was dissolved in a phosphate buffered physiological saline (PBS) (0.298 mL), and a 2 mM doxorubicin-bonded glutamic acid derivative PBS solution was prepared.

To 0.150 mL of this doxorubicin-bonded glutamic acid derivative (prodrug) PBS solution, 0.150 mL of a 2 U/mL GGT solution obtained by dissolving GGT (0.22 mg, 8 U/mg) (Sigma-Aldrich Company) in PBS (0.880 mL) was added, and the mixture was allowed to react at 37°C.

The reaction liquid was analyzed by HPLC, and thus the residual ratio of the doxorubicin-bonded glutamic acid derivative (prodrug) and the production ratio of doxorubicin were determined. The results are summarized in Table 1.

**[Table 1]**

| Reaction time (hr.) | 0 | 1 | 6.5 |
|---|---|---|---|
| Residual ratio of unchanged drug (Synthesis Example 1) | 100% | 39% | 1% |
| Doxorubicin production rate | 0% | 49% | 98% |

Comparative Test Example 1: GGT enzyme recognition test The doxorubicin-bonded glutamic acid derivative (0.17 mg) obtained in Comparative Example 1 was dissolved in PBS (0.1265 mL), and a 2 mM doxorubicin-bonded glutamic acid derivative PBS solution was prepared.

To 0.100 mL of the doxorubicin-bonded glutamic acid derivative PBS solution, 0.100 mL of a 2 U/mL GGT solution obtained by dissolving GGT (0.38 mg, 8 U/mg) (Sigma-Aldrich Company) in PBS (1.520 mL) was added, and the mixture was allowed to react at 37°C.

After 6 hours, the reaction liquid was analyzed by HPLC, and 93% of the doxorubicin-bonded glutamic acid derivative remained. From this, it was found that the doxorubicin-bonded glutamic acid derivative according to Comparative Example 1 was not recognized by GGT and could not release doxorubicin, which is a physiologically active substance.

From the results of Test Example 1, it was found that the doxorubicin-bonded glutamic acid derivative according to Synthesis Example 1 could release doxorubicin having pharmacological activity rapidly in the presence of GGT. Meanwhile, in Comparative Test Example 1, results suggested that the doxorubicin-bonded glutamic acid derivative of Comparative Example 1 obtained by directly bonding γ-glutamic acid to doxorubicin almost did not undergo a dissociation reaction of doxorubicin caused by GGT. Therefore, it became clear that for a rapid dissociation reaction induced by recognition of GGT, it was necessary to mediate an appropriate linker segment between the γ-glutamic acid bond site and the drug, and the aromatic amide type linker segment of the present invention could exhibit superior GGT recognition ability.

### Test Example 2: Phosphate buffer physiological saline (PBS) solution stability test

The doxorubicin-bonded glutamic acid derivative (0.49 mg) of Synthesis Example 1 was dissolved in phosphate buffered physiological saline (PBS) (0.298 mL), and a doxorubicin-bonded glutamic acid derivative PBS solution was prepared.

0.148 mL of the doxorubicin-bonded glutamic acid derivative PBS solution was dissolved in 0.148 mL of a PBS solution, and the solution was allowed to react at 37°C. After 6.5 hours, the reaction liquid was analyzed by HPLC, and reduction of the doxorubicin-bonded glutamic acid derivative was not confirmed.

Therefore, it was found that the doxorubicin-bonded glutamic acid derivative of Synthesis Example 1 according to the present invention did not release doxorubicin in a PBS solution and was chemically stable.

### Test Example 3: Cell proliferation inhibitory activity test

The cell proliferation inhibitory activity of the doxorubicin-bonded glutamic acid derivative according to Synthesis Example 1 was evaluated using OS-RC-2 cells having high GGT activity (RIKEN BioResource Center) and SK-OV-3 cells having low GGT activity (American Type Culture Collection).

On a 96-well plate, OS-RC-2 cells having high GGT activity and SK-OV-3 cells having low GGT activity were each inoculated in an amount of 4,000 cells/well and were incubated for one day at 37°C and 5% CO₂, and then the doxorubicin-bonded glutamic acid derivative according to Synthesis Example 1 was added to the cells at a final concentration of 0.039 to 10 µM. After the cells were incubated for 6 hours, the interior of the wells was washed, and after the cells were further incubated for 3 days, the cells were immobilized with methanol and stained using Methylene Blue dye. After staining, the dye was extracted with a 0.3% aqueous solution of hydrochloric acid was extracted, and the light absorbance at 660 nm was measured. For the light absorbance thus obtained, the cell proliferation suppressive activity was evaluated based on the reduction ratio for the light absorbance measured from cells to which the compound was not added.

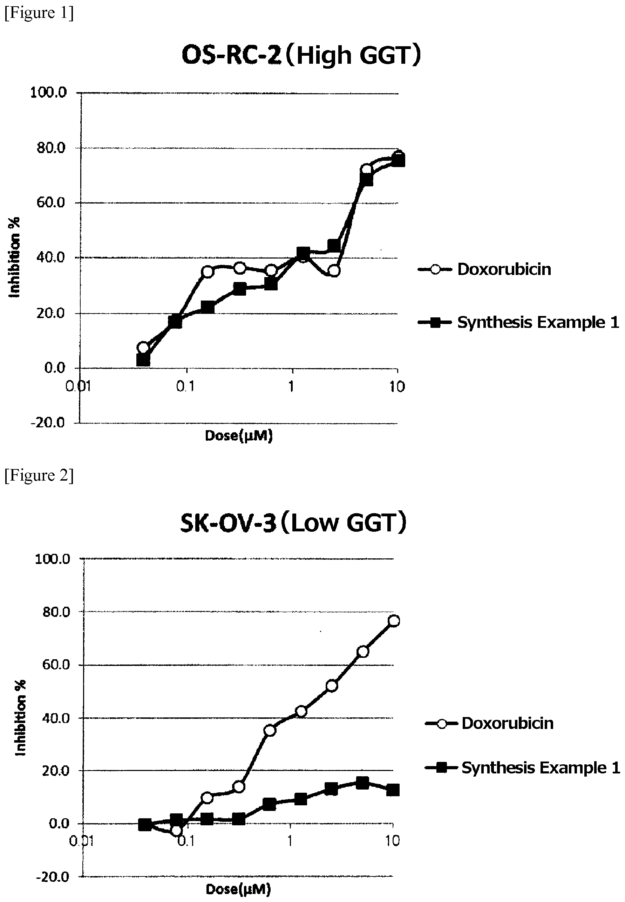

By a similar operation, the cell proliferation suppressive activity was evaluated from the light absorbance, using doxorubicin that was not converted to a glutamic acid derivative. The results are shown in Fig. 1 (OS-RC-2 cells having high GGT activity) and Fig. 2 (SK-OV-3 cells having low GGT activity).

### Test Example 4: Test for confirming GGT dependency of glutamic acid-derivatized prodrug

The GGT activity dependency of the cell proliferation inhibitory activity of the doxorubicin-bonded glutamic acid derivative (prodrug) according to Synthesis Example 1 was evaluated using OS-RC-2 cells having high GGT activity.

OS-RC-2 cells were inoculated by a method similar to that of Test Example 3. After the cells were incubated for one day, GGsTop (Wako Pure Chemical Industries, Ltd.), which is a GGT inhibitor, was added to the cells at a final concentration of 10 µM, and after the cells were incubated for one hour, the doxorubicin-bonded glutamic acid derivative according to Synthesis Example 1 was added to the cells at a final concentration of 0.039 to 10 µM. After the cells were incubated for 6 hours, the interior of the wells was washed, and after the cells were further incubated for 3 days, the cell proliferation suppressive activity was evaluated by a method similar to that of Test Example 3.

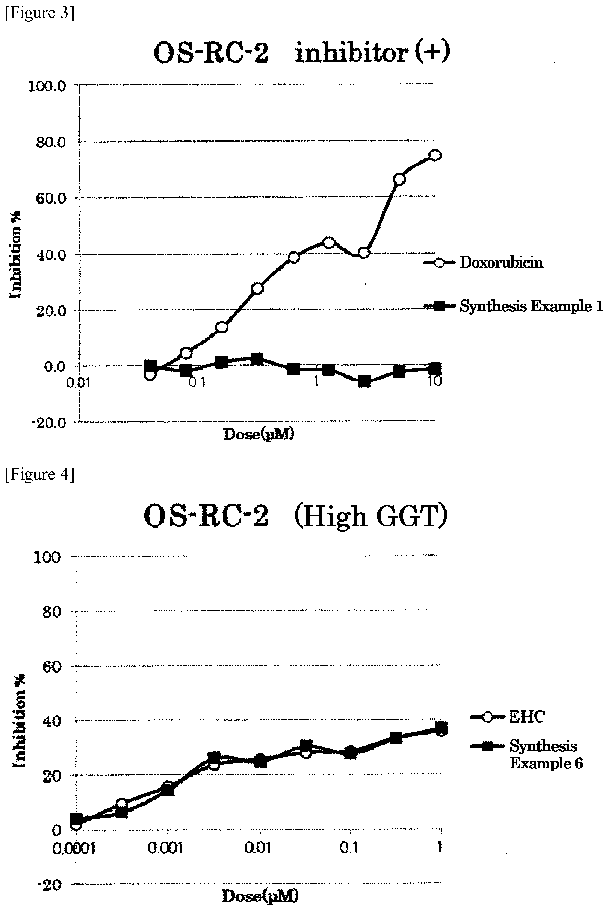

By a similar operation, the cell proliferation suppressive activity was evaluated using doxorubicin that was not glutamic acid-derivatized, from the light absorbance of the compound. The results are presented in Fig. 3.

As a result of Test Example 3, when 10 µM doxorubicin was added to OS-RC-2 cells having high GGT activity, the cell proliferation inhibitory activity was 77%. In contrast, when the doxorubicin-bonded glutamic acid derivative according to Synthesis Example 1 was added at 10 µM, the glutamic acid derivative exhibited a cell proliferation inhibitory activity of 76%.

Meanwhile, when 10 µM doxorubicin was added to SK-OV-3 cells having low GGT activity, the cell proliferation inhibitory activity was 77%. In contrast, when the doxorubicin-bonded glutamic acid derivative according to Synthesis Example 1 was added at 10 µM, the glutamic acid derivative exhibited a cell proliferation inhibitory activity of 13%.

When a comparison was made between the results for the cell proliferation inhibitory activity in cells having high GGT activity and cells having low GGT activity, it became clear that the doxorubicin-bonded glutamic acid derivative of Synthesis Example 1 according to the present invention released doxorubicin by means of GGT and exhibited cell proliferation inhibitory activity, and in the case of absence of GGT, the doxorubicin-bonded glutamic acid derivative had low cell proliferation inhibitory activity and almost did not exhibit cytocidal properties. From this, it was found that the doxorubicin-bonded glutamic acid derivative according to Synthesis Example 1 had properties of exhibiting cell proliferation suppressive activity that was dependent on GGT activity.

Furthermore, as a result of Test Example 4, a GGT inhibitor did not affect the cell proliferation suppressive activity of doxorubicin in OS-RC-2 cells having high GGT activity; however, a GGT inhibitor completely inhibited the activity of the doxorubicin-bonded glutamic acid derivative. From this, it was found that the doxorubicin-bonded glutamic acid derivative according to the present invention had properties of exhibiting cell proliferation suppressive activity that was dependent on GGT activity.

### Test Example 5: GGT enzyme recognition test

Similarly to Test Example 1, a 2 mM PBS solution of the epirubicin-bonded glutamic acid derivative of Synthesis Example 4 and a 2 U/mL GGT solution were used, and the solutions were allowed to react at 37°C.

The reaction liquid was analyzed by HPLC over time, and thereby it was confirmed that the epirubicin-bonded glutamic acid derivative was decomposed by an enzyme reaction. The residual ratio of the epirubicin-bonded glutamic acid derivative after 6 hours was 5%.

From this, it was found that the epirubicin-bonded glutamic acid derivative according to Synthesis Example 4 could release epirubicin having pharmacological activity rapidly in the presence of GGT.

### Test Example 6: GGT enzyme recognition test

Similarly to Test Example 1, a 1 mM PBS solution of the cytarabine-bonded glutamic acid derivative of Synthesis Example 5 and a 2 U/mL GGT solution were used, and the solutions were allowed to react at 37°C.

The reaction liquid was analyzed by HPLC over time, and thereby it was confirmed that the cytarabine-bonded glutamic acid derivative was decomposed by an enzyme reaction. The residual ratio of the cytarabine-bonded glutamic acid derivative after 6.5 hours was 34%. From this, it was found that the cytarabine-bonded glutamic acid derivative according to Synthesis Example 5 could rapidly release cytarabine having pharmacological activity in the presence of GGT.

### Comparative Test Example 2: GGT enzyme recognition test

Similarly to Test Example 1, a 2 mM PBS solution of the 9-aminocamptothecin-bonded glutamic acid derivative of Comparative Example 2 and a 2 U/mL GGT solution were used, and the solutions were allowed to react at 37°C.

After 6 hours, the reaction liquid was analyzed by HPLC, and 99% of the 9-aminocamptothecin-bonded glutamic acid derivative remained. From this, it was found that the 9-aminocamptothecin-bonded glutamic acid derivative according to Comparative Example 2 was not recognized by GGT and could not release 9-aminocamptothecin, which was a physiologically active substance.

Since the 9-aminocmptothecin-bonded glutamic acid derivative of Comparative Example 2 produced results in which a dissociation reaction of 9-aminocamptothecin caused by GGT almost did not occur, it became clear that a rapid dissociation reaction caused by recognition by GGT needed mediation of an appropriate linker segment between the γ-glutamic acid bonding site and the drug, and the aromatic amide type linker segment of the present invention could exhibit a superior GGT recognition ability.

### Test Example 7: Test on stability in PBS

PBS (0.220 mL) was added to a 60 mg/mL DMSO solution of the EHC-bonded glutamic acid derivative of Synthesis Example 2 (0.005 mL), and thus an EHC-bonded glutamic acid derivative solution was prepared. PBS (0.100 mL) was added to the EHC-bonded glutamic acid derivative solution (0.100 mL), and the mixture was allowed to react at 37°C. The reaction liquid was analyzed by HPLC, and thereby the residual ratio of the EHC-bonded glutamic acid derivative was determined. The residual ratio of the glutamic acid derivative prodrug after 3.5 hours was 52%.

PBS (0.294 mL) was added to a 6.3 mg/mL DMSO solution of the camptothecin-bonded glutamic acid derivative of Synthesis Example 3 (0.073 mL), and a camptothecin-bonded glutamic acid derivative solution was prepared. PBS (0.117 mL) was added to the camptothecin-bonded glutamic acid derivative solution (0.117 mL), and the mixture was allowed to react at 37°C. The reaction liquid was analyzed by HPLC, and thereby the residual ratio of the glutamic acid derivative was determined. The residual ratio of the glutamic acid derivative after 3 hours was 74%.

PBS (0.450 mL) was added to a 0.864 mg/mL DMSO solution of the EHC-bonded glutamic acid derivative of Synthesis Example 6 (0.050 mL), and the mixture was allowed to react at 37°C. The reaction liquid was analyzed by HPLC, and thereby the residual ratio of the EHC-bonded glutamic acid derivative was determined. 90% of the EHC-bonded glutamic acid derivative remained after 24 hours.

Test Example 8: Test on stability in mouse blood plasma Mouse blood plasma (0.180 mL) was added to a 0.856 mg/mL DMSO solution of the EHC-bonded glutamic acid derivative of Synthesis Example 6 (0.020 mL), and the mixture was allowed to react at 37°C.

The reaction liquid was analyzed by HPLC, and thereby the residual ratio of the EHC-bonded glutamic acid derivative was determined. 94% of the EHC-bonded glutamic acid derivative prodrug remained after 24 hours.

Therefore, it was found that the EHC-bonded glutamic acid derivative of Synthesis Example 6 according to the present invention was chemical stable even in the mouse blood plasma.

### Test Example 9: GGT enzyme recognition test

The EHC-bonded glutamic acid derivative of Synthesis Example 6 was dissolved in DMSO, and a 0.34 mg/mL solution was prepared. PBS (0.300 mL) was added to the solution (0.300 mL), and an EHC-bonded glutamic acid derivative solution (2) was prepared.

γ-Glutamyl transpeptidase (GGT, Sigma-Aldrich Company) was dissolved in PBS, and a 0.227 mg/mL solution was prepared.

The GGT solution (0.250 mL) was added to the EHC-bonded glutamic acid derivative solution (2) (0.250 mL), and the mixture was allowed to react at 37°C.

The reaction liquid was analyzed by HPLC, and thereby the residual ratio of the EHC-bonded glutamic acid derivative and the production ratio of EHC were determined.

As a result of Test Example 9, the EHC-bonded glutamic acid derivative of Synthesis Example 6 rapidly disappeared in one hour of the reaction and produced EHC. This result is a result showing that the compound of Synthesis Example 6 can produce EHC, which is a physiologically active substance, by being recognized by GGT and rapidly cleaving the γ-glutamyl aromatic amide linker including an ether bond.

From the results of Test Examples 7 and 8, it was found that the EHC prodrug according to Synthesis Example 6 had stable physical properties in a PBS solution and a solution in the presence of mouse blood plasma. Furthermore, it was found that the EHC prodrug according to Synthesis Example 6 could rapidly release EHC having pharmacological activity in the presence of GGT.

### Test Example 10: Cell proliferation inhibitory activity test

The cell proliferation inhibitory activity of the EHC-bonded glutamic acid derivative according to Synthesis Example 6 was evaluated using OS-RC-2 cells (RIKEN BioResource Center), which are known to have high GGT activity, and SK-OV-3 cells (American Type Culture Collection), which are known to have low GGT activity.

On a 96-well plate, OS-RC-2 cells having high GGT activity and SK-OV-3 cells having low GGT activity were each inoculated in an amount of 4,000 cells/well and were incubated for one day at 37°C and 5% CO₂, and then the EHC prodrug according to Synthesis Example 6 was added to the cells at a final concentration of 0.0001 to 1 µM. After the cells were incubated for 6 hours, the interior of the wells was washed, and the cells were further incubated for 3 days. After the incubation, the cells were immobilized with methanol and were stained using Methylene Blue dye. After staining, the dye was extracted with a 0.3% aqueous solution of hydrochloric acid, and the light absorbance at 660 nm was measured. The cell proliferation suppressive activity was evaluated based on the reduction ratio for the light absorbance thus obtained, with respect to the light absorbance measured from cells to which the compound was not added.

By a similar operation, EHC, which was an active ingredient of Synthesis Example 6, was used without modification, and the cell proliferation suppressive activity was evaluated from the light absorbance.

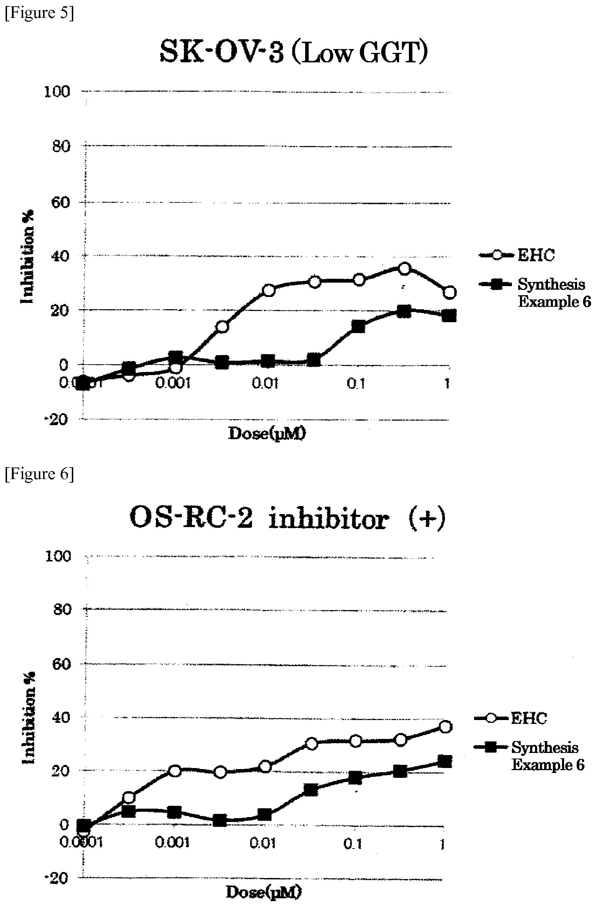

The results are shown in Fig. 4 (OS-RC-2 cells having high GGT activity) and Fig. 5 (SK-OV-3 cells having low GGT activity).

### Test Example 11: Test for confirming GGT dependency of EHC-bonded glutamic acid derivative

The GGT activity dependency of the cell proliferation inhibitory activity of the EHC-bonded glutamic acid derivative according to Synthesis Example 6 was evaluated using OS-RC-2 cells having high GGT activity.

OS-RC-2 cells were inoculated by a method similar to that of Test Example 10. After the cells were incubated for one day, GGsTop (Wako Pure Chemical Industries, Ltd.), which is a GGT inhibitor, was added to the cells at a final concentration of 10 µM, and the cells were incubated for one hour. Subsequently, the EHC-bonded glutamic acid derivative according to Synthesis Example 6 was added to the cells at a final concentration of 0.0001 to 1 µM. After the cells were incubated for 6 hours, the interior of the wells was washed, and after the cells were incubated for 3 days, the cell proliferation suppressive activity was evaluated by a method similar to that of Test Example 10.

By a similar operation, the cell proliferation suppressive activity was evaluated using EHC, which is the active ingredient of Synthesis Example 6, from the light absorbance of the compound.

The results are presented in Fig. 6.

As a result of Test Example 10, when 0.01 µM EHC was added to OS-RC-2 cells, which are known to have high GGT activity, the cell proliferation inhibitory activity was 26%. Furthermore, when the EHC-bonded glutamic acid derivative according to Synthesis Example 6 was added at 0.01 µM, the glutamic acid derivative exhibited a cell proliferation inhibitory activity of 25%.

Meanwhile, when 0.01 µM EHC was added to SK-OV-3 cells, which are known to have low GGT activity, the cell proliferation inhibitory activity was 27.7%. On the other hand, when the EHC-bonded glutamic acid derivative according to Synthesis Example 6 was added at 0.01 µM, the glutamic acid derivative exhibited a cell proliferation inhibitory activity of 1.3%.

When a comparison was made between the results for the cell proliferation inhibitory activity obtained in cells having high GGT activity and cells having low GGT activity, it became clear that the EHC-bonded glutamic acid derivative of Synthesis Example 6 according to the present invention released EHC in the presence of GGT and exhibited cell proliferation inhibitory activity, and in a case in which GGT was not present, the glutamic acid derivative had low cell proliferation inhibitory activity and almost did not show cytocidal properties. From this, it was found that the EHC-bonded glutamic acid derivative of Synthesis Example 6 could exhibit a cell proliferation suppressive activity that is dependent on GGT activity.

Furthermore, as a result of Test Example 11, the GGT inhibitor did not affect the cell proliferation suppressive activity of EHC in OS-RC-2 cells having high GGT activity; however, the GGT inhibitor inhibited the activity of the EHC-bonded glutamic acid derivative. From this, it was found that the EHC-bonded glutamic acid derivative of Synthesis Example 6 had a property of exhibiting a cell proliferation suppressive activity that was dependent on the GGT activity.

### Test Example 12: Test on pharmacokinetics of Synthesis Example 1

Human renal cell cancer OS-RC-2 tumors that had been subcutaneously subcultured in SCID mice were produced into blocks that measured about 2 mm on each side, and the blocks were subcutaneously transplanted into SCID mice using a trocar. After tumor engraftment was confirmed, the doxorubicin-bonded glutamic acid derivative of Synthesis Example 1 was suspended in a solution of DMSO/5% aqueous solution of glucose = 1 : 19, and the suspension was intravenously administered through the caudal vein at a dose of 5 mg/kg in terms of doxorubicin. Furthermore, doxorubicin hydrochloride (DXR•HCl) as a control drug was dissolved in physiological saline, and the solution was intravenously administered through the caudal vein at a dose of 5 mg/kg in terms of doxorubicin.

At time points of 5 minutes, 3 hours, and 24 hours after the administration, various tissues such as blood plasma, heart, liver, bone marrow, and tumor were collected under ether anesthesia. The drug concentrations of doxorubicin, which was an active drug, and the doxorubicin-bonded glutamic acid derivative of Synthesis Example 1, which was an unchanged drug, in the various tissues were quantitatively analyzed by a LC-MS/MS method under the following measuring instrument and conditions.
Machine model: ABSciex API4000
   Shimadzu LC-20AD
Column: CAPCELL PAKC18 MGIII of Shiseido Co., Ltd.,
   2.0 mm × 75 mm
Mobile phase A: Formic acid/acetonitrile/water (1/200/800)
Mobile phase B: Formic acid/acetonitrile (1/1000)
Mobile phase C: Formic acid/acetonitrile/water (1/500/500)
Ionizing method: Electrospray ionization (positive ions)
Detection method: Multiple reaction monitoring

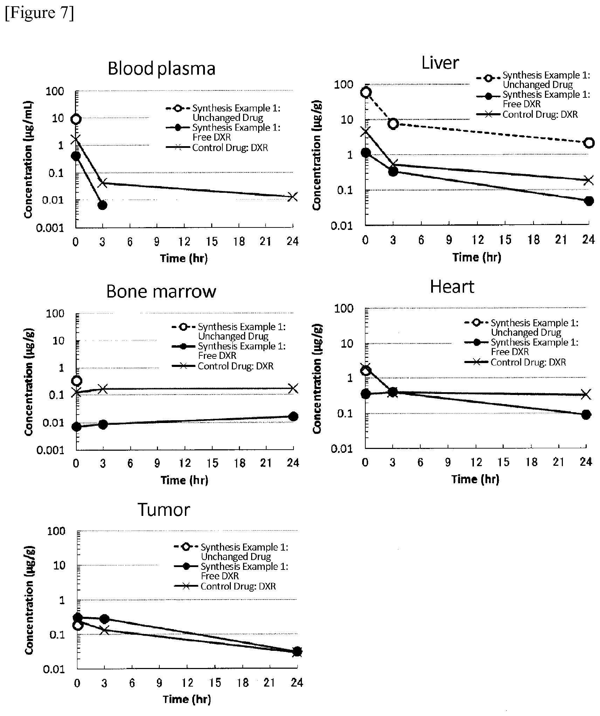

The time-dependent concentration profiles of doxorubicin (DXR), which was an active drug, and the doxorubicin-bonded glutamic acid derivative according to Synthesis Example 1, which was an unchanged drug, in tissues at various time points are shown in Fig. 7. Furthermore, as the activation rates of the doxorubicin-bonded glutamic acid derivative of Synthesis Example 1 in the various tissues, the ratios of the active drug (DXR) with respect to the unchanged drug (Synthesis Example 1 compound) as the values obtained 5 minutes after administration, were determined and presented in Table 2.

**[Table 2]**

| | Synthesis Example 1 (value after 5 minutes) (µg/mL or g) | | Activation rate DXR/unchanged drug |
|---|---|---|---|
| | DXR (active drug) | Unchanged drug | |
| Blood plasma | 0.428 | 9.36 | 0.0460 |
| Liver | 1.15 | 60.6 | 0.019 |
| Bone marrow | 0.00702 | 0.336 | 0.0210 |
| Heart | 0.354 | 1.63 | 0.217 |
| Tumor | 0.316 | 0.192 | 1.65 |

As a result of Test Example 12, the doxorubicin-bonded glutamic acid derivative of Synthesis Example 1 gave results in which the concentration of doxorubicin produced from the glutamic acid derivative was low in blood plasma, liver, bone marrow, and heart, in which GGT was expressed at a low level, and the concentration of doxorubicin was high in the tumor in which GGT was expressed at a high level, compared to doxorubicin hydrochloride. Furthermore, the activation rates of Synthesis Example 1 into doxorubicin (comparison of values after 5 minutes) were activation rates as low as about 0.02 to 0.2 times in blood plasma, liver, bone marrow, and heart, in which GGT was expressed at a low level. In contrast, in the tumors in which GGT was expressed at a high level, the activation rate showed a markedly high value such as 1.65 times, and thus, it was found that Synthesis Example 1 was selectively activated in the tumors.

From these results, it was found that in an in vivo test, the doxorubicin-bonded glutamic acid derivative of Synthesis Example 1 had a property of being capable of selectively releasing doxorubicin, which was a physiologically active substance, in tumors in which GGT was expressed at a high level in vivo. Meanwhile, it was found that in tissues in which GGT was expressed at a low level, such as bone marrow and heart, the doxorubicin concentrations had low values compared to the doxorubicin hydrochloride-administered group, and in these tissues, the glutamic acid derivative of Synthesis Example 1 was not activated. The tissue selectivity concerning the above-described activation implies a possibility by which the doxorubicin-bonded glutamic acid derivative of Synthesis Example 1 may reduce myelosuppression and cardiac toxicity, which are side effects of doxorubicin, and may selectively exhibit an antitumor effect.

### Test Example 13: Test on antitumor effect of Synthesis Example 1

By an operation similar to that of Test Example 12, human renal cell cancer OS-RC-2 tumors were subcutaneously transplanted into SCID mice, and tumor engraftment was confirmed. Subsequently, the doxorubicin-bonded glutamic acid derivative of Synthesis Example 1 was intravenously administered through the caudal vein at a dose of 5 or 20 mg/kg two times in total at an interval of 14 days. As a control drug, doxorubicin hydrochloride (DXR·HCl) was intravenously administered through the caudal vein at a dose of 5 mg/kg under the same schedule.

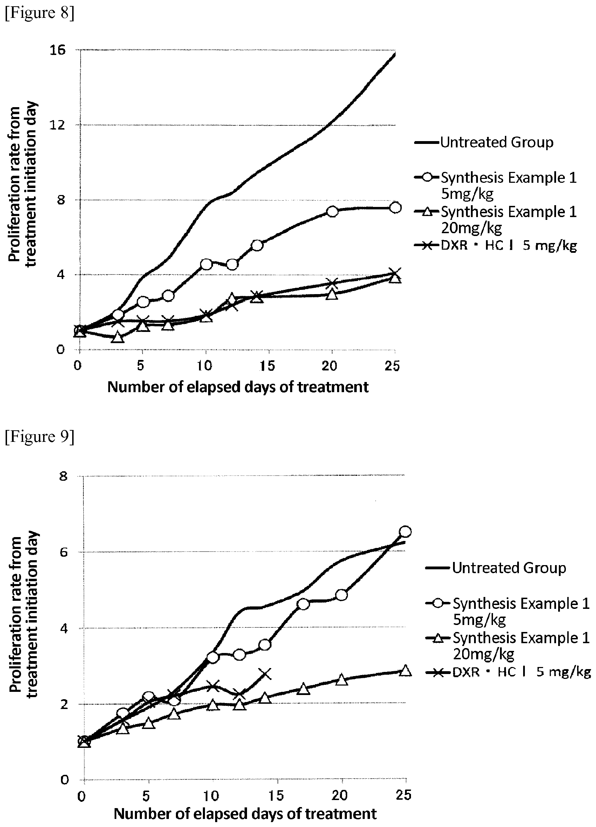

After the administration, the major axis (L) and the minor axis (W) of a tumor were measured using calipers at an interval of 2 to 3 days, the tumor volume was calculated by the formula: (L × W²)/2, and the tumor volume change rate from the administration initiation day was determined. The changes over time of relative tumor volumes in the various drug-administered groups are shown in Fig. 8.

As a result of Test Example 13, the doxorubicin-bonded glutamic acid derivative of Synthesis Example 1 exhibited a dose-dependent antitumor effect. The 20 mg/kg-administered group exhibited an antitumor effect that was equal to the effect of the doxorubicin hydrochloride-administered group (5 mg/kg), which was a control group. Therefore, it was found that the doxorubicin-bonded glutamic acid derivative according to Synthesis Example 1 had a sufficient antitumor effect as a medicine.

### Test Example 14: Test on antitumor effect of Synthesis Example 1

By an operation similar to that of Test Example 12, human ovarian cancer SHIN-3 tumors were subcutaneously transplanted into SCID mice, and tumor engraftment was confirmed. Subsequently, the doxorubicin-bonded glutamic acid derivative of Synthesis Example 1 was intravenously administered through the caudal vein at a dose of 5 or 20 mg/kg three times in total at an interval of 7 days. As a control drug, doxorubicin hydrochloride (DXR·HCl) was intravenously administered through the caudal vein at a dose of 5 mg/kg under the same schedule.

After the administration, the major axis (L) and the minor axis (W) of a tumor were measured using calipers at an interval of 2 to 3 days, the tumor volume was calculated by the formula: (L × W²)/2, and the tumor volume change rate from the administration initiation day was determined. The changes over time of relative tumor volumes in the various drug-administered groups are shown in Fig. 9.

As a result of Test Example 14, the doxorubicin-bonded glutamic acid derivative of Synthesis Example 1 exhibited a dose-dependent antitumor effect. In regard to doxorubicin hydrochloride (5 mg/kg) used as a control, the mice died after the second administration due to toxicity. Synthesis Example 1 exhibited an antitumor effect that was equivalent to that of the control drug in the 20 mg/kg-administered group and accomplished the dosage regimen. Thus, sufficient tolerance was confirmed. Therefore, it was found that the doxorubicin-bonded glutamic acid derivative according to Synthesis Example 1 had a sufficient antitumor effect as a medicine. Furthermore, it was suggested that the doxorubicin-bonded glutamic acid derivative was safer than the control drug.

### Test Example 15: Test on pharmacokinetics of Synthesis Example 6

Human renal cell cancer OS-RC-2 tumors that had been subcutaneously subcultured in SCID mice were produced into blocks that measured about 2 mm on each side, and the blocks were subcutaneously transplanted into SCID mice using a trocar. After tumor engraftment was confirmed, the EHC-bonded glutamic acid derivative of Synthesis Example 6 was suspended in a solution of DMSO/5% aqueous solution of glucose = 1 : 9, and the suspension was intravenously administered through the caudal vein at a dose of 20 mg/kg in terms of EHC. Furthermore, irinotecan hydrochloride (CPT-11), which is a prodrug of EHC, as a control drug was dissolved in a 5% aqueous solution of glucose, and the solution was intravenously administered through the caudal vein at a dose of 20 mg/kg in terms of EHC.

At time points of 5 minutes, 1 hour, 3 hours, and 6 hours after the administration, various tissues such as blood plasma, liver, bone marrow, and tumor were collected under ether anesthesia. The drug concentrations of EHC as an active drug of Synthesis Example 6, the unchanged drug of Synthesis Example 6, and the unchanged drug of CPT-11 in the various tissues were quantitatively analyzed by a LC-MS/MS method under the following measuring instrument and conditions.
Machine model: ABSciex API4000
   Shimadzu LC-20AD
Column: WATERS XBRIDGE, C18, 2.1 mm × 50 mm
Mobile phase A: Formic acid/water (1/1000)
Mobile phase B: Formic acid/acetonitrile (1/1000)
Ionizing method: Electrospray ionization (positive ions)
Detection method: Multiple reaction monitoring

From the concentrations of EHC, the EHC-bonded glutamic acid derivative according to Synthesis Example 6, and CPT-11 in the tissues at various time points, the AUC₀₋₆ₕᵣ (µg·hr/mL or g) values of the various components up to 6 hours after the initiation of administration were calculated. As the prodrug activation rates of Synthesis Example 6 and CPT-11 in the various tissues, the AUC₀₋₆ₕᵣ ratios of the active drug with respect to the unchanged drug (AUC_{EHC}/AUC_{Synthesis} Example 6 unchanged drug, and AUC_{EHC}/AUC_{CPT-11}) were determined. The results are presented in Table 3.

**[Table 3]**

| | Prodrug activation rate: AUC_{0-6hr EHC/prodrug} | | |
|---|---|---|---|
| Tissue | Synthesis Example 6 | CPT-11 | Ratio of activation rates of Synthesis Example 6/CPT-11 |
| Blood plasma | 0.163 | 0.154 | 1.058 |
| Liver | 0.125 | 0.0327 | 3.823 |
| Bone marrow | 0.0323 | 0.00345 | 9.362 |
| Tumor | 1.02 | 0.0165 | 61.82 |

As a result of Test Example 15, the EHC-bonded glutamic acid derivative of Synthesis Example 6 gave results in which the AUC_{EHC}/AUC_{Synthesis Example 6 unchanged drug} representing the activation rate as a EHC prodrug was low, such as 0.03 to 0.16 times, in blood plasma, liver, and bone marrow, in which GGT was expressed at a low level. In contrast to this, it was found that the ratio was high, such as 1.0 time, in the tumors in which GGT was expressed at a high level, and the glutamic acid derivative was tumor-selectively activated. Meanwhile, the AUC_{EHC}/AUC_{CPT-11} of CPT-11 as a control drug was all low such as 0.1 or less, and CPT-11 gave results with insufficient tissue selectivity.

The EHC-bonded glutamic acid derivative of Synthesis Example 6 exhibited activation rates of 1.1 times to 9.4 times, compared to CPT-11, in blood plasma, liver, and bone marrow with low GGT activity. In contrast to this, Synthesis Example 6 exhibited a markedly high value, such as 61.8 times, compared to the CPT-11-administered group in tumors in which GGT was expressed at a high level. From this, it was found that the EHC-bonded glutamic acid derivative of Synthesis Example 6 according to the present invention had a property of being activated GGT-dependently in tissues and being capable of selectively releasing EHC in tissues where GGT was expressed at a high level. Such selectivity suggests a possibility in which the EHC-bonded glutamic acid derivative of Synthesis Example 6 according to the present invention may avoid myelosuppression, which is a side effect of CPT-11, and thereby exhibit an antitumor effect.

### Test Example 16: Test on antitumor effect of Synthesis Example 6

By an operation similar to that of Test Example 15, to those mice having human renal cell cancer OS-RC-2 transplanted thereinto, the EHC-bonded glutamic acid derivative of Synthesis Example 6 was intravenously administered through the caudal vein, on the 17^{th} day after tumor transplantation, at a dose of 20, 40, or 80 mg/kg three times in total at an interval of 4 days.

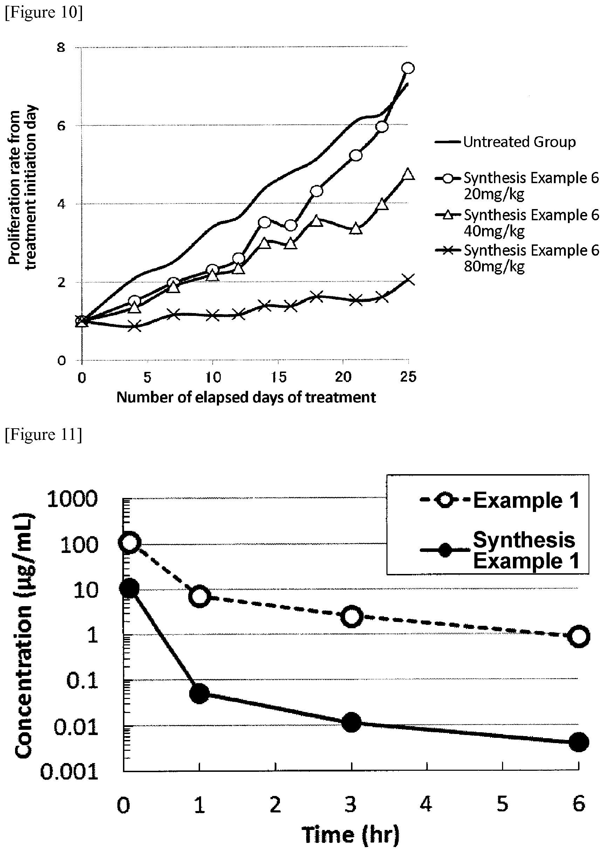

After the administration, the major axis (L) and the minor axis (W) of a tumor were measured using calipers at an interval of 2 to 3 days, the tumor volume was calculated by the formula: (L × W²)/2, and the tumor volume change rate from the administration initiation day was determined. The changes over time of relative tumor volumes in the various drug-administered groups are shown in Fig. 10.

As a result of Test Example 16, it was found that the EHC-bonded glutamic acid derivative of Synthesis Example 6 exhibited a dose-dependent antitumor effect. In the 80 mg/kg-administered group, the glutamic acid derivative exhibited a noticeable tumor regression effect, and it was found that the glutamic acid derivative has a property of exhibiting excellent antitumor action.

### Synthesis Example 7: Synthesis of block copolymer having cholesterol as hydrophobic functional group

According to the method described in JP H06-206815 A (Patent Literature 6), a block copolymer in which a polyethylene glycol segment (average molecular weight: 12,000) was linked to a polyaspartic acid segment (polyaspartic acid; average polymerization number: 43) was produced.

This block copolymer (10.00 g) and cholesterol (4.88 g) were dissolved in N,N-dimethylformamide (DMF, 145 mL), N,N-dimethylaminopyridine (DMAP, 0.31 g) was added thereto, and then diisopropylcarbodiimide (DIPCI, 8.91 mL) was added dropwise thereto at 25°C. After the mixture was stirred for 23 hours, DIPCI (4.46 mL) was further added to the mixture, and the mixture was stirred for 4 hours. The reaction solution was added dropwise to diisopropyl ether (3 L) under stirring, and a precipitate produced therefrom was collected by filtration. The precipitate was dissolved in a 50% aqueous solution of acetonitrile (800 mL), 200 mL Of a cation exchange resin, DOWEX 50W8 (manufactured by Dow Chemical Company), was added to the solution at 0°C, and the mixture was stirred for 5 hours. The cation exchange resin was eliminated by filtration, and the filtrate was concentrated under reduced pressure and then was freeze-dried. Thus, 12.52 g of a block copolymer having cholesterol as a hydrophobic functional group was obtained.

The bonding ratio of cholesterol in the block copolymer was 28% of the polyaspartic acid segment polymerization number from the reaction ratio.

### Synthesis Example 8

### Synthesis Example of block copolymer having tryptophan benzyl ester as hydrophobic functional group

A block copolymer in which a polyethylene glycol segment (average molecular weight: 12,000) was linked to a polyaspartic acid segment (polyaspartic acid; average polymerization number: 43) was produced according to the method described in JP H06-206815 A (Patent Literature 6).

This block copolymer (0.50 g) and tryptophan benzyl hydrochloride (0.21 g) were dissolved in DMF (7 mL), and DMAP (16 mg) and diisopropylethylamine (DIPEA, 0.11 mL) were added to the solution. Subsequently, DIPCI (0.45 mL) was added dropwise to the mixture at 15°C. After the mixture was stirred for 3 hours, DIPCI (0.23 mL) was further added thereto, and the resulting mixture was stirred for 3 hours at 25°C. The reaction solution was added dropwise to diisopropyl ether (210 mL) under stirring, and a precipitate thus produced was collected by filtration. The precipitate was dissolved in a 50% aqueous solution of acetonitrile (40 mL), and then 10 mL of a cation exchange resin, DOWEX 50W8 (manufactured by Dow Chemical Company), was added to the solution at 0°C. The mixture was stirred for 4 hours. The cation exchange resin was eliminated by filtration, and the filtrate was concentrated under reduced pressure and then freeze-dried. Thereby, 0.73 g of a block copolymer having tryptophan benzyl ester as a hydrophobic functional group was obtained.

The bonding ratio of tryptophan in the block copolymer was 50% of the polyaspartic acid segment polymerization number of the block copolymer from the reaction ratio.

### Synthesis Example 9

### Synthesis of block copolymer having cholesterol and tryptophan benzyl ester as hydrophobic functional groups

A block copolymer in which a polyethylene glycol segment (average molecular weight: 12,000) was linked to a polyaspartic acid segment (polyaspartic acid; average polymerization number: 43) was produced according to the method described in JP H06-206815 A (Patent Literature 6).

This block copolymer (2.00 g), cholesterol (0.98 g), and tryptophan benzyl hydrochloride (0.42 g) were dissolved in DMF (60 mL), and DIPEA (0.21 mL) and DMAP (62 mg) were added to the solution. DIPCI (1.78 mL) was added dropwise to the mixture at 25°C, and after the mixture was stirred for 21 hours, DIPCI (0.89 mL) was further added thereto. The mixture was stirred for 4 hours. The reaction solution was added dropwise to diisopropyl ether (1,500 mL) under stirring, and a precipitate thus produced was collected by filtration. The precipitate was dissolved in a 50% aqueous solution of acetonitrile (160 mL), and then 40 mL of a cation exchange resin, DOWEX 50W8 (manufactured by Dow Chemical Company), was added to the solution at 0°C. The mixture was stirred for 2.5 hours. The cation exchange resin was eliminated by filtration, and the filtrate was concentrated under reduced pressure and then freeze-dried. 2.50 g of the block copolymer was obtained.

The bonding ratios of tryptophan and cholesterol in the block copolymer were 25% and 13%, respectively, of the polyaspartic acid segment polymerization number of the block copolymer from the reaction ratios.

### Synthesis Example 10

### Synthesis of block copolymer having doxorubicin as hydrophobic functional group

A block copolymer having doxorubicin as a hydrophobic functional group was obtained from a block copolymer in which a polyethylene glycol segment (average molecular weight: 5,000) was linked to a polyaspartic acid segment (polyaspartic acid unit content: 41%), according to the method described in JP H07-69900 A (Patent Literature 5).

The bonding ratio of doxorubicin in the block copolymer was 45% of the polyaspartic acid segment polymerization number of the block copolymer.

### Synthesis Example 11

### Synthesis of block copolymer having phenylbutanol as hydrophobic functional group

A block copolymer having phenylbutanol as a hydrophobic functional group was obtained from a block copolymer in which a polyethylene glycol segment (average molecular weight: 12,000) was linked to a polyaspartic acid segment (polyaspartic acid unit content: 28%), according to the method described in WO 2006/033296 A (Patent Literature 4).

The bonding ratio of phenylbutanol in the block copolymer was 15% of the polyaspartic acid segment polymerization number of the block copolymer.

### Synthesis Example 12

### Synthesis of block copolymer having benzyl alcohol as hydrophobic functional group

A block copolymer in which a polyethylene glycol segment (average molecular weight: 12,000) was linked to a polyaspartic acid benzyl ester segment (polyaspartic acid benzyl ester; average polymerization number: 43) was produced according to the method described in JP H06-206815 A (Patent Literature 6).

### [Example 1]

### Composition of glutamic acid derivative (I) of Synthesis Example 1 and block copolymer (II) of Synthesis Example 10

The doxorubicin-bonded glutamic acid derivative of Synthesis Example 1 (1.0 mg) and the block copolymer of Synthesis Example 10 (10 mg) were dissolved in DMF (80 µL), and then water (320 µL) was added to the solution. This mixture was subjected to dialysis against water overnight using a dialysis kit (GE Healthcare Biosciences Corp., Mini Dialysis Kit 1kDa cut-off, molecular cut-off = 1,000). The dialysis solution thus obtained was introduced into a 1-mL graduated cylinder, water was added thereto to make up 1 mL, and thereby a composition according to Example 1 was obtained as a 1 mg/mL aqueous solution.

### [Example 2]

### Composition of glutamic acid derivative (I) of Synthesis Example 1 and block copolymer (II) of Synthesis Example 11

The doxorubicin-bonded glutamic acid derivative of Synthesis Example 1 (1.0 mg) and the block copolymer of Synthesis Example 11 (30 mg) were dissolved in DMF (120 µL) and DMSO (120 µL), and then water (360 µL) was added thereto. This mixture was subjected to dialysis against water overnight using a dialyzing membrane (Spectrum Laboratories, Inc., SPECTRA/POR (registered trademark), molecular cut-off = 6,000 to 8,000). The dialysate thus obtained was introduced into a 5-mL graduated flask, and water was added thereto to make up 5 mL. Thus, a composition according to Example 2 was obtained as a 0.6 mg/mL aqueous solution.

### [Example 3]

### Composition of glutamic acid derivative (I) of Synthesis Example 1 and block copolymer (II) of Synthesis Example 7

The doxorubicin-bonded glutamic acid derivative of Synthesis Example 1 (1.0 mg) and the block copolymer of Synthesis Example 7 (10 mg) were dissolved in hexafluoroisopropanol (HFIP, 400 µL), and then the solvent was distilled off under reduced pressure. A 40 mg/mL aqueous solution of maltose (1 mL) was added to the residue, and the mixture was stirred overnight. Maltose (20 mg) was added to the solution thus obtained, and the mixture was freeze-dried. Thereby, a composition according to Example 3 was obtained.

The content of the doxorubicin-bonded glutamic acid derivative according to Synthesis Example 1 in the composition of Example 3 was 1.4%.

### [Example 4]

### Composition of glutamic acid derivative (I) of Synthesis Example 1 and block copolymer (II) of Synthesis Example 8

The doxorubicin-bonded glutamic acid derivative (1.6 mg) of Synthesis Example 1 was dissolved in DMF (160 µL) and water (160 µL), and the solution was mixed with a DMF (150 µL) solution of the block copolymer of Synthesis Example 8 (7.5 mg). The mixture was subjected to dialysis against water one day and night using a dialysis kit (GE Healthcare Biosciences Corp., Mini Dialysis Kit 1kDa cut-off, molecular cut-off = 1,000). Macrogol (10 mg) was added to the dialysate thus obtained, and an aqueous solution thus obtained was freeze-dried. Thereby, a composition according to Example 4 was obtained.

The content of the doxorubicin-bonded glutamic acid derivative according to Synthesis Example 1 in the composition of Example 4 was 8.4%.

### [Example 5]

### Composition of glutamic acid derivative (I) of Synthesis Example 1 and block copolymer (II) of Synthesis Example 9

The doxorubicin-bonded glutamic acid derivative of Synthesis Example 1 (1.2 mg) was dissolved in DMF (150 µL) and water (120 µL), and the solution was mixed with a DMF (150 µL) solution of the block copolymer of Synthesis Example 9 (7.5 mg). The mixture was subjected to dialysis against water one day and night using a dialysis kit (GE Healthcare Biosciences Corp., Mini Dialysis Kit 1kDa cut-off, molecular cut-off = 1,000). Macrogol (10 mg) was added to the dialysate thus obtained, and an aqueous solution thus obtained was freeze-dried. Thereby, a composition according to Example 5 was obtained.

The content of the doxorubicin-bonded glutamic acid derivative according to Synthesis Example 1 in the composition of Example 5 was 6.4%.

### [Example 6]

### Composition of glutamic acid derivative (I) of Synthesis Example 4 and block copolymer (II) of Synthesis Example 10

The epirubicin-bonded glutamic acid derivative of Synthesis Example 4 (42.2 mg) was dissolved in DMF (2.11 mL) and water (2.11 mL), and the solution was mixed with a DMF (8.44 mL) solution of the block copolymer of Synthesis Example 10 (422 mg). The mixture was subjected to dialysis against water one day and night using a dialyzing membrane (Spectrum Laboratories, Inc., SPECTRA/POR (registered trademark), molecular cut-off = 6,000 to 8,000). Macrogol (840 mg) was added to the dialysate thus obtained, and an aqueous solution thus obtained was freeze-dried. Thereby, a composition according to Example 6 (1.27 g) was obtained. The content of the epirubicin-bonded glutamic acid derivative according to Synthesis Example 4 as determined by a content analysis by HPLC was 1.8%.

### [Example 7]

### Composition of glutamic acid derivative (I) of Synthesis Example 6 and block copolymer (II) of Synthesis Example 11

The camptothecin-bonded glutamic acid derivative of Synthesis Example 6 (5 mg) was dissolved in HFIP (250 µL), and the solution was added dropwise to a 40 mg/mL aqueous solution of maltose (5 mL) of the block copolymer of Synthesis Example 11 (100 mg). After the mixture was stirred two days and nights, Macrogol (100 mg) was added to the mixture, and a solution thus obtained was freeze-dried. Thereby, a composition according to Example 7 was obtained.

The content of the camptothecin-bonded glutamic acid derivative according to Synthesis Example 6 as determined by a content analysis by HPLC was 1.1%.

### [Example 8]

### Composition of glutamic acid derivative (I) of Synthesis Example 6 and block copolymer (II) of Synthesis Example 7

The camptothecin-bonded glutamic acid derivative of Synthesis Example 6 (5 mg) and the block copolymer of Synthesis Example 7 (21 mg) were dissolved in HFIP, and then the solvent was distilled off under reduced pressure. The residue was dissolved in a 40 mg/mL aqueous solution of maltose (2 mL), and the solution was stirred overnight. Macrogol (20 mg) was added thereto, and a solution thus obtained was freeze-dried. Thereby, a composition according to Example 8 was obtained.

The content of the camptothecin-bonded glutamic acid derivative according to Synthesis Example 6 in the composition of Example 8 was 0.82%.

### [Example 9]

### Composition of glutamic acid derivative (I) of Synthesis Example 6 and block copolymer (II) of Synthesis Example 9

The camptothecin-bonded glutamic acid derivative of Synthesis Example 6 (5 mg) was dissolved in HFIP (250 µL), and the solution was added dropwise to a 40 mg/mL aqueous solution of maltose (5 mL) of the block copolymer of Synthesis Example 9 (100 mg). After the mixture was stirred two days and nights, Macrogol (100 mg) was added thereto. A solution thus obtained was freeze-dried, and thereby a composition according to Example 9 was obtained.

The content of the camptothecin-bonded glutamic acid derivative according to Synthesis Example 6 as determined by a content analysis of HPLC was 1.1%.

### [Example 10]

### Composition of glutamic acid derivative (I) of Synthesis Example 6 and block copolymer (II) of Synthesis Example 12

The camptothecin-bonded glutamic acid derivative of Synthesis Example 6 (1 mg) and the block copolymer of Synthesis Example 12 (21 mg) were dissolved in HFIP, and then the solvent was distilled off under reduced pressure. A 40 mg/mL aqueous solution of maltose (2 mL) was added to the residue, and the mixture was stirred for 24 hours. Macrogol (20 mg) was added to the mixture, and a solution thus obtained was freeze-dried. Thereby, a composition according to Example 10 was obtained.

The content of the camptothecin-bonded glutamic acid derivative according to Synthesis Example 6 in the composition of Example 10 was 0.82%.

### Test Example 17: Test on pharmacokinetics of Example 1

The composition of Example 1 was dissolved in distilled water, and the solution was intravenously administered through the caudal vein of mice at a dose of 10 mg/kg in terms of doxorubicin (DXR). Furthermore, as a control drug, the doxorubicin-bonded glutamic acid derivative (DXR prodrug) of Synthesis Example 1 was suspended in a DMSO/5% aqueous solution of glucose (1 : 19 (v/v)) solution, and the suspension was intravenously administered through the caudal vein at a dose of 10 mg/kg in terms of DXR.

At time points of 5 minutes, 1 hour, 3 hours, and 6 hours after administration, blood plasma was collected under ether anesthesia. The drug concentration of the DXR prodrug as an unchanged drug and the concentration of doxorubicin (DXR) as an active drug were quantitatively analyzed by the same method as that of Test Example 12.

The results of the concentration profiles of the doxorubicin-bonded glutamic acid derivative (DXR prodrug) in blood plasma after the administration of Example 1 and Synthesis Example 1 are shown in Fig. 11. Furthermore, the AUC₀₋₆ₕᵣ of the active drug (DXR) and the unchanged drug (DXR prodrug) in blood plasma were calculated, and the values are shown in Table 4.

**[Table 4]**

| | AUC₀₋₆ₕᵣ. (µg ·hr/mL) | | |
|---|---|---|---|
| | DXR (active drug) | Unchanged drug | Ratio of unchanged drug |
| Example 1 | 3.05 | 82.2 | 13.2 |
| Synthesis Example 1 | 0.370 | 6.24 | - |

As a result of Test Example 17, it was found that the composition of Example 1 disappeared slowly from the blood plasma compared to the DXR prodrug of Synthesis Example 1, and the blood retentivity was markedly increased. The AUC₀₋₆ₕᵣ (µg·hr/mL) of the unchanged drug (DXR prodrug) of Example 1 increased very obviously to 13 times, compared to Synthesis Example 1.

Since the pharmacokinetics of Example 1 showed the unique pharmacokinetic characteristics shown in a pharmaceutical preparation by a polymerized carrier, it is contemplated that a complex based on an interaction between the doxorubicin-bonded glutamic acid (I) according to Synthesis Example 1 and the block copolymer (II) according to Synthesis Example 10 has been formed. It is known that the pharmaceutical composition based on the formation of a complex with a polymer carrier is retained in blood for a long time and then accumulates at a tumor affected area. Therefore, it was suggested that the composition of Example 1 has enhanced tumor accumulation properties, efficiently produces an active drug as a result of the GGT enzyme activity in a tumor, and could exhibit an antitumor effect.

### Test Example 18: Test on pharmacokinetics of Examples 7 and 9

The compositions of Examples 7 and 9 were dissolved in 5% glucose, and the solutions were intravenously administered through the caudal vein of nude mice at a dose of 10 mg/kg in terms of EHC. Furthermore, as a control drug, the EHC-bonded glutamic acid derivative (EHC prodrug) of Synthesis Example 6 was suspended in a DMSO/5% aqueous solution of glucose (1 : 9 (v/v)) solution, and the suspension was intravenously administered through the caudal vein at a dose of 10 mg/kg in terms of EHC.

At time points of 5 minutes, 6 hours, and 24 hours after administration, blood plasma was collected under isoflurane anesthesia. The drug concentration of the EHC prodrug as an unchanged drug was quantitatively analyzed by the same method as that of Test Example 15.

The AUC₀₋₂₄ₕᵣ of the EHC prodrug in blood plasma of the compositions of Examples 7 and 9 and Synthesis Example 6 were calculated. A comparison was made between the blood retentivity of the EHC prodrug of Examples 7 and 9 and the blood retentivity of Synthesis Example 6 based on the AUC baseline, and the results are shown in Table 5.

**[Table 5]**

| | AUC₀₋₂₄ₕᵣ (µg ·hr/mL) | Ratio of unchanged drug |
|---|---|---|
| Example 7 | 375 | 10.4 |
| Example 9 | 234 | 6.5 |
| Synthesis Example 6 | 36 | - |

As a result of Test Example 18, it was found that the compositions of Examples 7 and 9 had markedly enhanced blood retentivity, compared to the EHC prodrug of Synthesis Example 6. The AUC₀₋₂₄ₕᵣ (µg·hr/mL) of the unchanged drugs of Examples 7 and 9 (EHC prodrugs) increased to 10.4 times and 6.5 times, respectively, compared to Synthesis Example 6.

Since the pharmacokinetics of Examples 7 and 9 showed the unique pharmacokinetic characteristics shown in a pharmaceutical preparation by a polymerized carrier, it was suggested that the corresponding glutamic acid derivative and the block copolymer were integrated by forming a complex, and such pharmacokinetics were exhibited. Therefore, it was suggested that the compositions of Examples 7 and 9 passively exhibited tumor accumulation properties, efficiently produced an active drug as a result of GGT enzyme activity in the tumor, and could exhibit an antitumor effect.

### Test Example 19: Test on antitumor effect of Example 6

Human renal cell cancer OS-RC-2 was transplanted into nude mice similarly to Test Example 15, and after tumor engraftment was confirmed, the composition of Example 6 according to the present invention was dissolved in 5% glucose. The solution was intravenously administered through the caudal vein once at a dose of 10 or 20 mg/kg in terms of epirubicin. Furthermore, as a control drug, epirubicin hydrochloride was dissolved in a physiological solution, and the solution was intravenously administered through the caudal vein once at a dose of 10 or 20 mg/kg in terms of epirubicin.

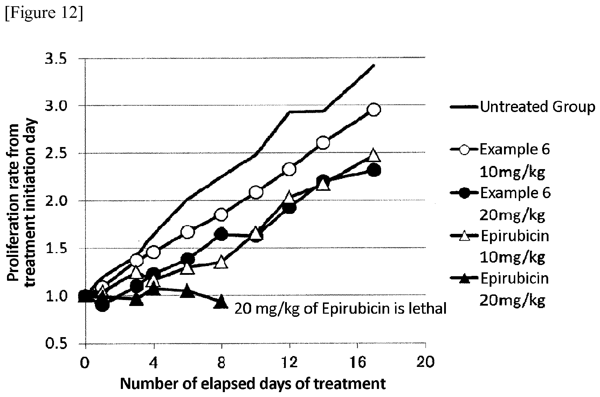

After the administration, the major axis (L) and the minor axis (W) of a tumor were measured using calipers at an interval of 2 to 3 days, the tumor volume was calculated by the formula: (L × W²)/2, and the tumor volume change rate from the administration initiation day was determined. The changes over time of relative tumor volumes in the various drug-administered groups are shown in Fig. 12.

As a result of Test Example 19, the composition of Example 6 exhibited a dose-dependent antitumor effect. In regard to epirubicin hydrochloride (20 mg/kg) used as a control, the mice died 8 days after the administration due to toxicity. Example 6 exhibited an antitumor effect that was equivalent to that of the control drug in the 20 mg/kg-administered group and accomplished the dosage regimen. Thus, sufficient tolerance was confirmed. Therefore, it was found that the composition of Example 6 had a sufficient antitumor effect as a medicine. Furthermore, it was suggested that the composition of Example 6 was safer than the control drug.

## Claims

1. A composition comprising:
(I) a glutamic acid derivative represented by General Formula (1): 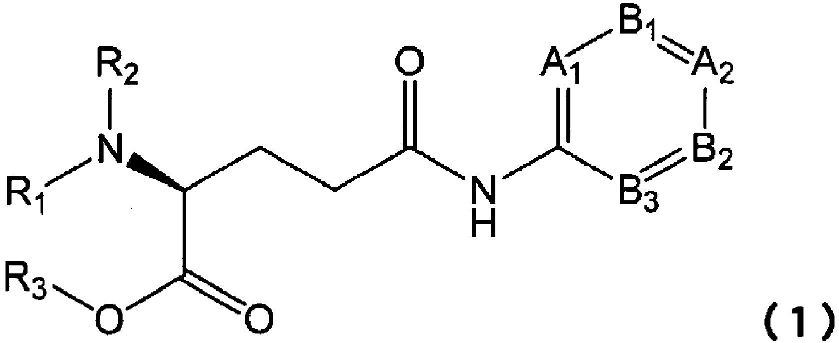 wherein **R**₁ and **R**₂ each independently represent a group selected from the group consisting of a hydrogen atom, an alkyl group which may have a substituent, and an alkoxycarbonyl group which may have a substituent; **R**₃ represents a hydrogen atom or an alkyl group which may have a substituent; **A**₁ and **A**₂ each represent a group selected from the group consisting of C-**R**₆, C-**R**₇, and a nitrogen atom; **R**₆ represents one or more groups selected from the group consisting of a hydrogen atom, a halogen atom, a nitro group, a hydroxy group, an alkyl group which may have a substituent, and an alkoxy group which may have a substituent; **R**₇ is represented by the following General Formula (2): 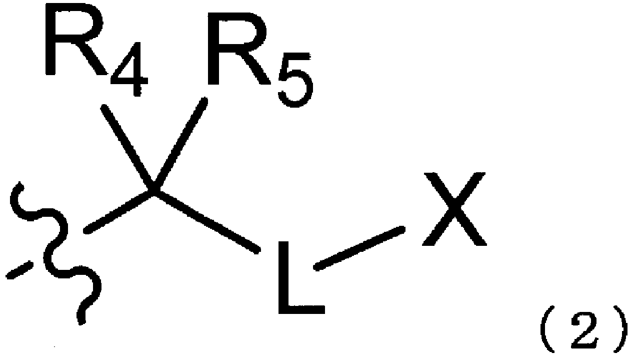 wherein **R**₄ and **R**₅ each independently represent a hydrogen atom or an alkyl group which may have a substituent; **L** represents a linking group selected from the group consisting of an oxygen atom, an oxycarbonyl group, and a bond; **X** represents a residue of a physiologically active substance having one or more functional groups selected from the group consisting of an aliphatic hydroxy group, an aromatic hydroxy group, an amino group, and a carboxy group,
(a) when **X** is a residue of a physiologically active substance having one or more functional groups selected from the group consisting of an aliphatic hydroxy group and an amino group, **L** represents an oxycarbonyl group;
(b) when **X** represents a residue of a physiologically active substance having a carboxy group, **L** represents an oxygen atom; and
(c) when **X** represents a residue of a physiologically active substance having an aromatic hydroxy group, **L** represents a bond or an oxycarbonyl group,
wherein any one of **A**₁ and **A**₂ represents C-**R**₇; the other represents C-**R**₆ or a nitrogen atom; and **B**₁, **B**₂, and **B**₃ each independently represent C-**R**₆ or a nitrogen atom;
or a pharmacologically acceptable salt thereof; and
(II) a block copolymer having a polyethylene glycol segment linked to a polyamino acid segment with a hydrophobic functional group.

2. The composition according to claim 1, wherein the polyamino acid in the block copolymer (II) is selected from the group consisting of a polyaspartic acid, a polyglutamic acid, and a poly(aspartic acid-glutamic acid) copolymer.

3. The composition according to claim 1 or 2, wherein the hydrophobic functional group in the block copolymer (II) is one or more groups selected from the group consisting of a linear, branched or cyclic (C1-C30) alkyl group which may have a substituent; a linear, branched or cyclic (C2-C30) alkenyl group which may have a substituent; a linear or branched (C7-C30) aralkyl group which may have a substituent; an aryl group which may have a substituent; a heterocyclic aryl group which may have a substituent; and a residue of a physiologically active substance.

4. The composition according to any one of claims 1 to 3,
wherein the weight average molecular weight of the polyethylene glycol segment in the block copolymer (II) is 1 kilodalton to 500 kilodaltons, and the polymerization number of the polyamino acid is 2 to 200.

5. The composition according to any one of claims 1 to 4, wherein the block copolymer (II) is a block copolymer represented by General Formula (3): 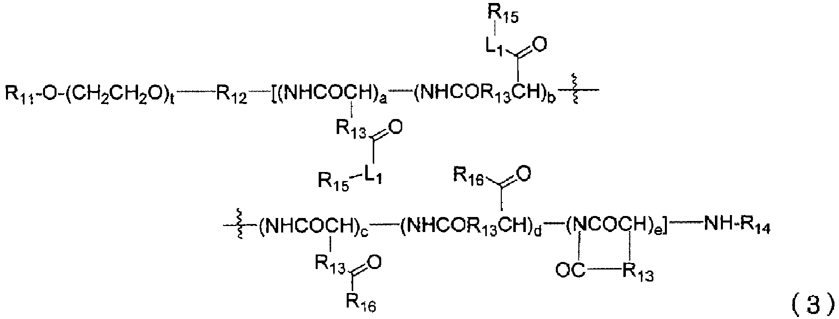
wherein **R**₁₁ represents a hydrogen atom or a linear or branched (C1-C10) alkyl group; **R**₁₂ represents a (C1-C6) alkylene group; **R**₁₃ represents a methylene group and/or an ethylene group; **R**₁₄ represents one selected from the group consisting of a hydrogen atom, a (C1-C6) acyl group, and a (C1-C6) alkyloxycarbonyl group;
**R**₁₅ represents one or more groups selected from the group consisting of a linear, branched or cyclic (C1-C30) alkyl group which may have a substituent, a linear, branched or cyclic (C2-C30) alkenyl group which may have a substituent, a linear, branched or cyclic (C7-C30) aralkyl group which may have a substituent, an aryl group which may have a substituent, a heterocyclic aryl group which may have a substituent, and a residue of a physiologically active substance;
**R**₁₆ represents a hydroxy group and/or -N(**R**₁₇)CONH(**R**₁₈) ; wherein **R**₁₇ and **R**₁₈, which may be identical or different, each represent a linear, branched or cyclic (C3-C8) alkyl group, or a (C1-C6) alkyl group which may be substituted with a tertiary amino group;
**L**₁ represents a linking group or a bond; t represents an integer from 20 to 11,500; a, b, c, d, and e each independently represent an integer from 0 to 100; (a + b + c + d + e), which is the total polymerization number of the polyamino acid segment, represents an integer from 10 to 100; (a + b) represents an integer from 3 to 100; the respective constituent units to which **R**₁₅ and **R**₁₆ are bonded, and the constituent unit in which a side-chain carboxy group is intramolecularly cyclized, each independently have a randomly arranged segment structure.

6. The composition according to any one of claims 1 to 5, wherein the hydrophobic functional group in the block copolymer (II) is one or more groups selected from the group consisting of a residue of an amino acid derivative modified with a hydrophobic functional group, a residue of a sterol derivative, a (C7-C20) aralkyl group which may have a substituent, an anthracycline-based antibiotic substance, a camptothecin derivative, and a nucleic acid antimetabolite.

7. The composition according to any one of claims 1 to 6, wherein in regard to the glutamic acid derivative represented by General Formula (1) or a pharmacologically acceptable salt thereof (I), **R**₇ is represented by the following General Formula (4): 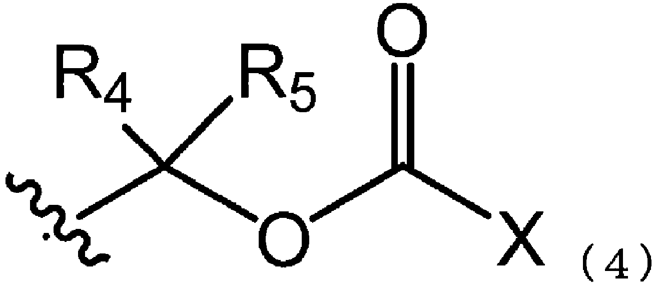 wherein **R**₄ and **R**₅ are as defined above; and **X** represents a residue of a physiologically active substance having one or more functional groups selected from the group consisting of an aliphatic hydroxy group, an aromatic hydroxy group, and an amino group.

8. The composition according to claim 7, wherein the physiologically active substance in the residue of a physiologically active substance represented by **X** is camptothecin or a derivative thereof.

9. The composition according to claim 7, wherein the physiologically active substance in the residue of a physiologically active substance represented by **X** is a physiologically active substance selected from the group consisting of doxorubicin, daunorubicin, epirubicin, pirarubicin, and amrubicin.

10. The composition according to claim 7, wherein the physiologically active substance in the residue of a physiologically active substance represented by **X** is a physiologically active substance selected from the group consisting of gemcitabine, ethynyl cytidine, cytarabine, and CNDAC (2'-cyano-2'-deoxy-1-β-D-arabinofuranosylcytosine).

11. The composition according to any one of claims 1 to 6, wherein **R**₇ is represented by the following General Formula (5) : 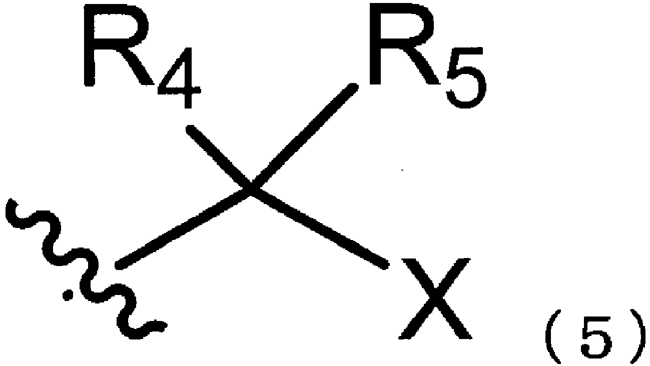 wherein **R**₄ and **R**₅ are as defined above; and **X** represents a residue of a physiologically active substance having an aromatic hydroxy group.

12. The composition according to claim 11, wherein the physiologically active substance in the residue of a physiologically active substance represented by **X** is selected from the group consisting of 7-ethyl-10-hydroxycamptothecin, nogitecan, and derivatives thereof.

13. The composition according to any one of claims 1 to 12, wherein the mass ratio of the glutamic acid derivative or a pharmacologically acceptable salt thereof (I) to the block copolymer (II) is (I) : (II) = 1 : 0.5 to 50.

14. The composition according to any one of claims 1 to 13, wherein the glutamic acid derivative or a pharmacologically acceptable salt thereof (I) is associated with the block copolymer (II).

15. A medicine comprising the composition according to any one of claims 1 to 14.

## Patentansprüche

1. Zusammensetzung, umfassend:
(I) ein Glutaminsäurederivat, dargestellt durch die allgemeine Formel (1): 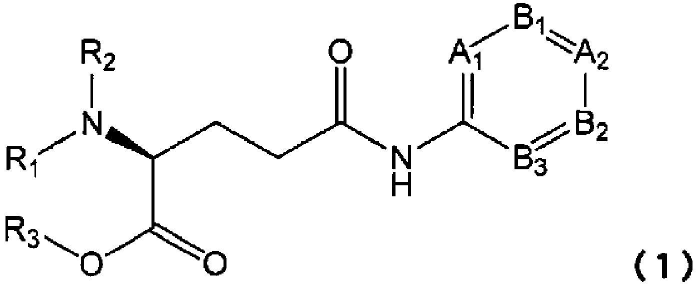
wobei **R**₁ und **R**₂ jeweils unabhängig voneinander eine Gruppe darstellen, ausgewählt aus der Gruppe bestehend aus einem Wasserstoffatom, einer Alkylgruppe, die einen Substituenten aufweisen kann, und einer Alkoxycarbonylgruppe, die einen Substituenten aufweisen kann;
**R**₃ ein Wasserstoffatom oder eine Alkylgruppe darstellt, die einen Substituenten aufweisen kann;
**A**₁ und **A**₂ jeweils eine Gruppe darstellen, ausgewählt aus der Gruppe bestehend aus C-**R**₆, C-**R**₇ und einem Stickstoffatom;
**R**₆ eine oder mehrere Gruppen darstellt, ausgewählt aus der Gruppe bestehend aus einem Wasserstoffatom, einem Halogenatom, einer Nitrogruppe, einer Hydroxygruppe, einer Alkylgruppe, die einen Substituenten aufweisen kann, und einer Alkoxygruppe, die einen Substituenten aufweisen kann;
**R**₇ durch die folgende allgemeine Formel (2) dargestellt ist: 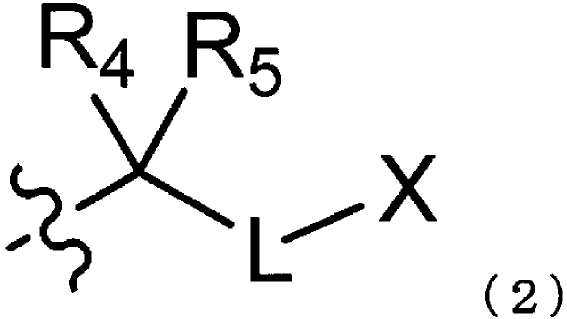
wobei **R**₄ und **R**₅ jeweils unabhängig voneinander ein Wasserstoffatom oder eine Alkylgruppe darstellen, die einen Substituenten aufweisen kann;
**L** eine Verknüpfungsgruppe darstellt, ausgewählt aus der Gruppe bestehend aus einem Sauerstoffatom, einer Oxycarbonylgruppe und einer Bindung;
**X** einen Rest einer physiologisch aktiven Substanz darstellt, die eine oder mehrere funktionelle Gruppen aufweist, ausgewählt aus der Gruppe bestehend aus einer aliphatischen Hydroxygruppe, einer aromatischen Hydroxygruppe, einer Aminogruppe und einer Carboxygruppe,
(a) wenn **X** ein Rest einer physiologisch aktiven Substanz ist, die eine oder mehrere funktionelle Gruppen aufweist, ausgewählt aus der Gruppe bestehend aus einer aliphatischen Hydroxygruppe und einer Aminogruppe, stellt **L** eine Oxycarbonylgruppe dar;
(b) wenn **X** einen Rest einer physiologisch aktiven Substanz darstellt, die eine Carboxygruppe aufweist, stellt **L** ein Sauerstoffatom dar; und
(c) wenn **X** einen Rest einer physiologisch aktiven Substanz darstellt, die eine aromatische Hydroxygruppe aufweist, stellt **L** eine Bindung oder eine Oxycarbonylgruppe dar, wobei eines von **A**₁ und **A**₂ C-**R**₇ darstellt; der andere C-**R**₆ oder ein Stickstoffatom darstellt; und **B**₁, **B**₂ und **B**₃ jeweils unabhängig C-**R**₆ oder ein Stickstoffatom darstellen; oder ein pharmakologisch unbedenkliches Salz davon; und
(II) ein Blockcopolymer, das ein Polyethylenglykolsegment aufweist, das an ein Polyaminosäuresegment mit einer hydrophoben funktionellen Gruppe gebunden ist.

2. Zusammensetzung nach Anspruch 1, wobei die Polyaminosäure in dem Blockcopolymer (II) ausgewählt ist aus der Gruppe bestehend aus einer Polyasparaginsäure, einer Polyglutaminsäure und einem Poly (asparaginsäure-Glutaminsäure-)Copolymer.

3. Zusammensetzung nach Anspruch 1 oder 2, wobei die hydrophobe funktionelle Gruppe in dem Blockcopolymer (II) eine oder mehrere Gruppen ist, ausgewählt aus der Gruppe bestehend aus einer linearen, verzweigten odercyclischen (C1-C30-) Alkylgruppe, die einen Substituenten aufweisen kann;
einer linearen, verzweigten oder cyclischen (C2-C30-)Alkenylgruppe, die einen Substituenten aufweisen kann;
einer linearen oder verzweigten (C7-C30-)Aralkylgruppe, die einen Substituenten aufweisen kann;
einer Arylgruppe, die einen Substituenten aufweisen kann;
einer heterocyclischen Arylgruppe, die einen Substituenten aufweisen kann; und einem Rest einer physiologisch aktiven Substanz.

4. Zusammensetzung nach einem der Ansprüche 1 bis 3, wobei das Gewichtsmittel der Molekularmasse des Polyethylenglykolsegments in dem Blockcopolymer (II) 1 Kilodalton bis 500 Kilodalton und die Polymerisationszahl der Polyaminosäure 2 bis 200 beträgt.

5. Zusammensetzung nach einem der Ansprüche 1 bis 4, wobei das Blockcopolymer (II) ein Blockcopolymer ist, dargestellt durch die allgemeine Formel (3): 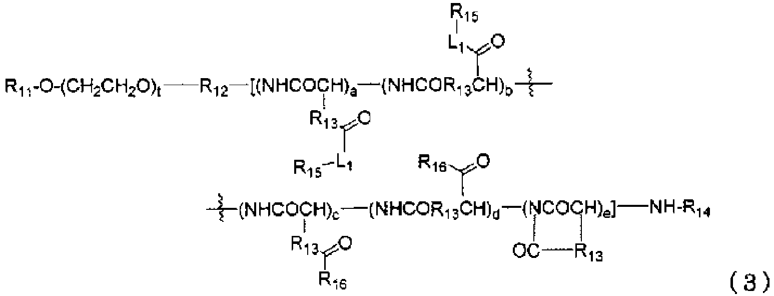
wobei **R**₁₁ ein Wasserstoffatom oder eine lineare oder verzweigte (C1-C10-) Alkylgruppe darstellt;
**R**₁₂ eine (C1-C6-)Alkylengruppe darstellt;
**R**₁₃ eine Methylengruppe und/oder eine Ethylengruppe darstellt;
**R**₁₄ eines ausgewählt aus der Gruppe bestehend aus einem Wasserstoffatom, einer (C1-C6-)Acylgruppe und einer (C1-C6-)Alkyloxycarbonylgruppe darstellt;
**R**₁₅ eine oder mehrere Gruppen darstellt, ausgewählt aus der Gruppe bestehend aus einer linearen, verzweigten oder cyclischen (C1-C30-)Alkylgruppe, die einen Substituenten aufweisen kann, einer linearen, verzweigten oder cyclischen (C2-C30-)Alkenylgruppe, die einen Substituenten aufweisen kann, einer linearen, verzweigten oder cyclischen (C7-C30-)Aralkylgruppe, die einen Substituenten aufweisen kann, einer Arylgruppe, die einen Substituenten aufweisen kann, einer heterocyclische Arylgruppe, die einen Substituenten aufweisen kann, und einen Rest einer physiologisch aktiven Substanz;
**R**₁₆ eine Hydroxygruppe und/oder -N(**R**₁₇)CONH(**R**₁₈) darstellt;
wobei **R**₁₇ und **R**₁₈, die identisch oder verschieden sein können, jeweils eine lineare, verzweigte oder cyclische (C3-C8-)Alkylgruppe oder eine (C1-C6-) Alkylgruppe darstellen, die mit einer tertiären Aminogruppe substituiert sein kann;
**L**₁ eine Verknüpfungsgruppe oder eine Bindung darstellt;
t eine ganze Zahl von 20 bis 11.500 darstellt;
a, b, c, d und e jeweils unabhängig voneinander eine ganze Zahl von 0 bis 100 darstellen;
(a + b + c + d + e), die die Gesamtpolymerisationszahl des Polyaminosäuresegments ist, eine ganze Zahl von 10 bis 100 darstellen;
(a + b) eine ganze Zahl von 3 bis 100 darstellt;
die jeweiligen Bestandteileinheiten, an die **R**₁₅ und **R**₁₆ gebunden sind, und die Bestandteileinheiten, in denen eine Seitenkettencarboxygruppe intramolekular cyclisiert ist, jeweils unabhängig voneinander eine zufällig angeordnete Segmentstruktur aufweisen.

6. Zusammensetzung nach einem der Ansprüche 1 bis 5, wobei die hydrophobe funktionelle Gruppe in dem Blockcopolymer (II) eine oder mehrere Gruppen ist, ausgewählt aus der Gruppe bestehend aus einem Rest eines Aminosäurederivats, modifiziert mit einer hydrophoben funktionellen Gruppe, einem Rest eines Sterolderivats, einer (C7-C20-)Aralkylgruppe, die einen Substituenten, eine Antibiotikasubstanz auf Anthracyclinbasis, ein Camptothecinderivat und einen Nukleinsäureantimetaboliten aufweisen kann.

7. Zusammensetzung nach einem der Ansprüche 1 bis 6, wobei in Bezug auf das durch die allgemeine Formel (1) dargestellte Glutaminsäurederivat oder ein pharmakologisch unbedenkliches Salz davon (I), **R**₇ durch die folgende allgemeine Formel (4) ist: 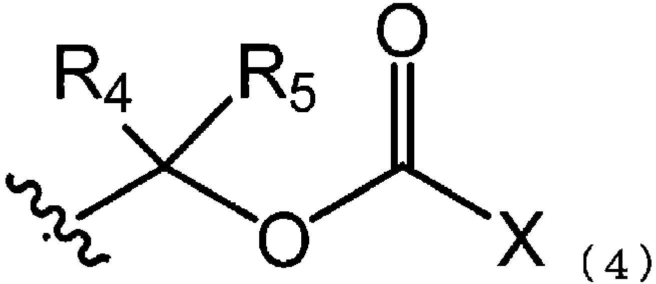
wobei **R**₄ und **R**₅ wie oben definiert sind; und
**X** einen Rest einer physiologisch aktiven Substanz darstellt, die eine oder mehrere funktionelle Gruppen aufweist, ausgewählt aus der Gruppe bestehend aus einer aliphatischen Hydroxygruppe, einer aromatischen Hydroxygruppe und einer Aminogruppe.

8. Zusammensetzung nach Anspruch 7, wobei die physiologisch aktive Substanz in dem Rest einer durch **X** dargestellten physiologisch aktiven Substanz Camptothecin oder ein Derivat davon ist.

9. Zusammensetzung nach Anspruch 7, wobei die physiologisch aktive Substanz in dem Rest einer durch **X** dargestellten physiologisch aktiven Substanz eine physiologisch aktive Substanz ist, ausgewählt aus der Gruppe bestehend aus Doxorubicin, Daunorubicin, Epirubicin, Pirarubicin und Amrubicin.

10. Zusammensetzung nach Anspruch 7, wobei die physiologisch aktive Substanz in dem Rest einer durch **X** dargestellten physiologisch aktiven Substanz eine physiologisch aktive Substanz ist, ausgewählt aus der Gruppe bestehend aus Gemcitabin, Ethinylcytidin, Cytarabin und CNDAC (2'-Cyano-2,-Desoxy-1-β-D-Arabinofuranosylcytosin).

11. Zusammensetzung nach einem der Ansprüche 1 bis 6, wobei **R**₇ durch die folgende allgemeine Formel (5) dargestellt ist: 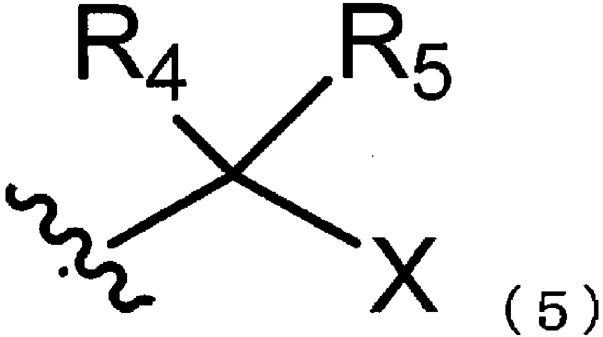
wobei **R**₄ und **R**₅ wie oben definiert sind; und
**X** einen Rest einer physiologisch aktiven Substanz darstellt, der eine aromatische Hydroxygruppe aufweist.

12. Zusammensetzung nach Anspruch 11, wobei die physiologisch aktive Substanz in dem Rest einer durch **X** dargestellten physiologisch aktiven Substanz ausgewählt ist aus der Gruppe bestehend aus 7-Ethyl-10-hydroxycamptothecin, Nogitecan und Derivaten davon.

13. Zusammensetzung nach einem der Ansprüche 1 bis 12, wobei das Massenverhältnis des Glutaminsäurederivats oder eines pharmakologisch unbedenklichen Salzes davon (I) zum Blockcopolymer (II) (I) : (II) = 1 : 0,5 bis 50 ist.

14. Zusammensetzung nach einem der Ansprüche 1 bis 13, wobei das Glutaminsäurederivat oder ein pharmakologisch unbedenkliches Salz davon (I) mit dem Blockcopolymer (II) verknüpft ist.

15. Arzneimittel, umfassend die Zusammensetzung nach einem der Ansprüche 1 bis 14.

## Revendications

1. Composition comprenant:
(I) un dérivé d'acide glutamique représenté par la Formule générale (1): 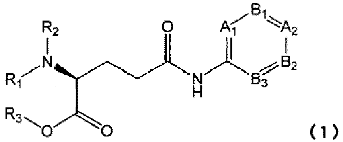 dans laquelle **R**₁ et **R**₂ représentent chacun indépendamment un groupe sélectionné dans le groupe constitué d'un atome d'hydrogène, d'un groupe alkyle qui peut avoir un substituant, et d'un groupe alcoxycarbonyle qui peut avoir un substituant; **R**₃ représente un atome d'hydrogène ou un groupe alkyle qui peut avoir un substituant; **A**₁ et **A**₂ représentent chacun un groupe sélectionné dans le groupe constitué de C-**R**₆, C-**R**₇, et d'un atome d'azote; **R**₆ représente un ou plusieurs groupes sélectionnés dans le groupe constitué d'un atome d'hydrogène, d'un atome d'halogène, d'un groupe nitro, d'un groupe hydroxyle, d'un groupe alkyle qui peut avoir un substituant, et d'un groupe alcoxy qui peut avoir un substituant; **R**₇ est représenté par la Formule générale suivante (2): 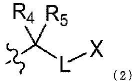 dans laquelle **R**₄ et **R**₅ représentent chacun indépendamment un atome d'hydrogène ou un groupe alkyle qui peut avoir un substituant; **L** représente un groupe de liaison sélectionné dans le groupe constitué d'un atome d'oxygène, d'un groupe oxycarbonyle, et d'une liaison; **X** représente un résidu d'une substance physiologiquement active ayant un ou plusieurs groupes fonctionnels sélectionnés dans le groupe constitué d'un groupe hydroxyle aliphatique, d'un groupe hydroxyle aromatique, d'un groupe amino, et d'un groupe carboxyle,
(a) lorsque **X** est un résidu d'une substance physiologiquement active ayant un ou plusieurs groupes fonctionnels sélectionnés dans le groupe constitué d'un groupe hydroxyle aliphatique et d'un groupe amino, **L** représente un groupe oxycarbonyle;
(b) lorsque **X** représente un résidu d'une substance physiologiquement active ayant un groupe carboxyle, **L** représente un atome d'oxygène; et
(c) lorsque **X** représente un résidu d'une substance physiologiquement active ayant un groupe hydroxyle aromatique, **L** représente une liaison ou un groupe oxycarbonyle,
où l'un quelconque de **A**₁ et **A**₂ représente C-**R**₇; l'autre représente C-**R**₆ ou un atome d'azote; et **B**₁, **B**₂, et **B**₃ représentent chacun indépendamment C-**R**₆ ou un atome d'azote;
ou son sel pharmacologiquement acceptable; et
(II) un copolymère séquencé ayant un segment polyéthylène glycol lié à un segment poly(acide aminé) avec un groupe fonctionnel hydrophobe.

2. Composition selon la revendication 1, dans laquelle le poly(acide aminé) dans le copolymère séquencé (II) est sélectionné dans le groupe constitué d'un poly(acide aspartique), d'un poly(acide glutamique), et d'un copolymère de poly(acide aspartique-acide glutamique).

3. Composition selon la revendication 1 ou 2, le groupe fonctionnel hydrophobe dans le copolymère séquencé (II) étant un ou plusieurs groupes sélectionnés dans le groupe constitué d'un groupe alkyle linéaire, ramifié ou cyclique en C₁ à C₃₀ qui peut avoir un substituant; d'un groupe alcényle linéaire, ramifié ou cyclique en C₂ à C₃₀ qui peut avoir un substituant; ou d'un groupe aralkyle linéaire ou ramifié en C₇ à C₃₀ qui peut avoir un substituant; d'un groupe aryle qui peut avoir un substituant; d'un groupe aryle hétérocyclique qui peut avoir un substituant; et d'un résidu d'une substance physiologiquement active.

4. Composition selon l'une quelconque des revendications 1 à 3,
dans laquelle le poids moléculaire moyen en poids du segment polyéthylène glycol dans le copolymère séquencé (II) est de 1 kilodalton à 500 kilodaltons, et l'indice de polymérisation du poly(acide aminé) est de 2 à 200.

5. Composition selon l'une quelconque des revendications 1 à 4, dans laquelle le copolymère séquencé (II) est un copolymère séquencé représenté par la Formule générale (3): 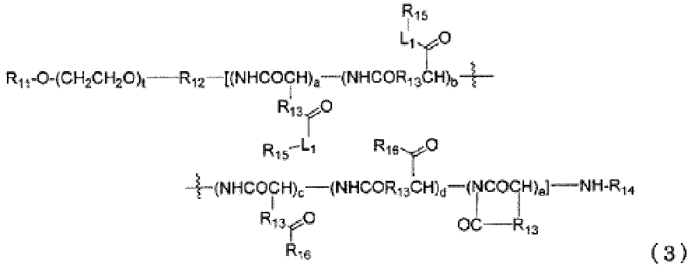
dans laquelle **R**₁₁ représente un atome d'hydrogène ou un groupe alkyle linéaire ou ramifié en C₁ à C₁₀; **R**₁₂ représente un groupe alcylène en C₁ à C₆; **R**₁₃ représente un groupe méthylène et/ou un groupe éthylène; **R**₁₄ représente l'un sélectionné dans le groupe constitué d'un atome d'hydrogène, d'un groupe acyle en C₁ à C₆, et d'un groupe alkyloxycarbonyle en C₁ à C₆;
**R**₁₅ représente un ou plusieurs groupes sélectionnés dans le groupe constitué d'un groupe alkyle linéaire, ramifié ou cyclique en C₁ à C₃₀ qui peut avoir un substituant, d'un groupe alcényle en C₂ à C₃₀ linéaire, ramifié ou cyclique qui peut avoir un substituant, d'un groupe aralkyle en C₇ à C₃₀ linéaire, ramifié ou cyclique qui peut avoir un substituant, d'un groupe aryle qui peut avoir un substituant, d'un groupe aryle hétérocyclique qui peut avoir un substituant, et d'un résidu d'une substance physiologiquement active;
**R**₁₆ représente un groupe hydroxyle et/ou -N(**R**₁₇)CONH(**R**₁₈);
où **R**₁₇ et **R**₁₈, qui peuvent être identiques ou différents, représentent chacun un groupe alkyle en C₃ à C₈ linéaire, ramifié ou cyclique, ou un groupe alkyle en C₁ à C₆ qui peut être substitué par un groupe amino tertiaire;
**L**₁ représente un groupe de liaison ou une liaison; t représente un nombre entier d'une valeur de 20 à 11 500; a, b, c, d, et e représentent chacun indépendamment un nombre entier d'une valeur de 0 à 100; (a + b + c + d + e), qui est l'indice de polymérisation total du segment poly(acide aminé), représente un nombre entier d'une valeur de 10 à 100; (a + b) représente un nombre entier d'une valeur de 3 à 100; les motifs constitutifs respectifs auxquels **R**₁₅ et **R**₁₆ sont liés, et le motif constitutif dans lequel un groupe carboxyle à chaîne latérale est cyclisé de manière intramoléculaire, ayant chacun indépendamment une structure de segment disposée de manière aléatoire.

6. Composition selon l'une quelconque des revendications 1 à 5, le groupe fonctionnel hydrophobe dans le copolymère séquencé (II) étant un ou plusieurs groupes sélectionnés dans le groupe constitué d'un résidu d'un dérivé acide aminé modifié par un groupe fonctionnel hydrophobe, d'un résidu d'un dérivé stérol, d'un groupe aralkyle en C₇ à C₂₀ qui peut avoir un substituant, d'une substance antibiotique à base d'anthracycline, d'un dérivé camptothécine, et d'un antimétabolite acide nucléique.

7. Composition selon l'une quelconque des revendications 1 à 6, dans laquelle concernant le dérivé d'acide glutamique représenté par la Formule générale (1) ou son sel pharmacologiquement acceptable (I), **R**₇ est représenté par la Formule générale suivante (4): 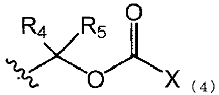 dans laquelle **R**₄ et **R**₅ sont tels que définis ci-dessus; et **X** représente un résidu d'une substance physiologiquement active ayant un ou plusieurs groupes fonctionnels sélectionnés dans le groupe constitué d'un groupe hydroxyle aliphatique, d'un groupe hydroxyle aromatique, et d'un groupe amino.

8. Composition selon la revendication 7, dans laquelle la substance physiologiquement active dans le résidu d'une substance physiologiquement active représenté par **X** est la camptothécine ou son dérivé.

9. Composition selon la revendication 7, dans laquelle la substance physiologiquement active dans le résidu d'une substance physiologiquement active représenté par **X** est une substance physiologiquement active sélectionnée dans le groupe constitué de la doxorubicine, de la daunorubicine, de l'épirubicine, de la pirarubicine, et de l'amrubicine.

10. Composition selon la revendication 7, dans laquelle la substance physiologiquement active dans le résidu d'une substance physiologiquement active représenté par **X** est une substance physiologiquement active sélectionnée dans le groupe constitué de la gemcitabine, de l'éthynyl cytidine, de la cytarabine, et de la CNDAC (2'-cyano-2'-désoxy-1-β-D-arabinofuranosylcytosine).

11. Composition selon l'une quelconque des revendications 1 à 6, dans laquelle **R**₇ est représenté par la Formule générale suivante (5): 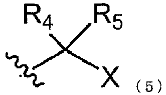 dans laquelle **R**₄ et **R**₅ sont tels que définis ci-dessus; et **X** représente un résidu d'une substance physiologiquement active ayant un groupe hydroxyle aromatique.

12. Composition selon la revendication 11, dans laquelle la substance physiologiquement active dans le résidu d'une substance physiologiquement active représenté par **X** est sélectionnée dans le groupe constitué de la 7-éthyl-10-hydroxycamptothécine, du nogitécane, et leurs dérivés.

13. Composition selon l'une quelconque des revendications 1 à 12, dans laquelle le rapport en masse du dérivé d'acide glutamique ou son sel pharmacologiquement acceptable (I) au copolymère séquencé (II) est (I):(II) = 1:0,5 à 50.

14. Composition selon l'une quelconque des revendications 1 à 13, dans laquelle le dérivé d'acide glutamique ou son sel pharmacologiquement acceptable (I) est associé au copolymère séquencé (II).

15. Médicament comprenant la composition selon l'une quelconque des revendications 1 à 14.
